(19) Europäisches Patentamt
European Patent Office
Office européen des brevets

(11) Veröffentlichungsnummer: **0 233 140 B1**

(12) # EUROPÄISCHE PATENTSCHRIFT

(45) Veröffentlichungstag der Patentschrift: **08.11.89**

(51) Int. Cl.⁴: **C 10 M 135/20,** C 07 C 149/24, C 07 C 149/42, C 07 D 249/08, C 07 D 295/08

(21) Anmeldenummer: **87810008.0**

(22) Anmeldetag: **07.01.87**

(54) **Schwefel-und stickstoffhaltige Schmiermittelzusätze.**

(30) Priorität: **10.01.86 CH 87/86**

(43) Veröffentlichungstag der Anmeldung: **19.08.87 Patentblatt 87/34**

(45) Bekanntmachung des Hinweises auf die Patenterteilung: **08.11.89 Patentblatt 89/45**

(84) Benannte Vertragsstaaten: **BE DE FR GB IT**

(56) Entgegenhaltungen:
**EP-A- 0 154 721**
**GB-A- 2 172 284**
**US-A- 4 393 026**

**THE JOURNAL OF ORGANIC CHEMISTRY, Band 33, Nr. 2, 12. Februar 1968, Seiten 523-530, American Chemical Society; V.R. GAERTNER: "Ring-Opening Alkylations of 1,1-Dialkyl-3-Substituted Azetidinium Cations; Substituent Entropy-Controlled Strained Ring-Chain Equilibria"**

(73) Patentinhaber: **CIBA-GEIGY AG, Klybeckstrasse 141, CH-4002 Basel (CH)**

(72) Erfinder: **Wirth, Hermann Otto, Dr., Lessingstrasse 24, D-6140 Bensheim 3 (DE)**
Erfinder: **Friedrich, Hans-Helmut, Am Rauhenstein 8, D-6147 Lautertal 2 (DE)**

EP 0 233 140 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Schmiermittel, Hydraulikflüssigkeiten und Metallbearbeitungsflüssigkeiten, enthaltend stickstoffhaltige Schwefelverbindungen, die Verwendung dieser Verbindungen als Additive, sowie neue stickstoffhaltige Schwefelverbindungen.

Schmiermittel, Hydraulikflüssigkeiten sowie Metallbearbeitungsflüssigkeiten werden im allgemeinen Additive zur Verbesserung der Gebrauchseigenschaften zugesetzt. Besondere Anforderungen in bezug auf das Lasttragevermögen werden an Schmiermittel zur Übertragung grösserer Kräfte gestellt. Durch Zusatz von Hochdruck- und verschleissmindernden Additiven werden die ansonsten auftretenden negativen Erscheinungen stark reduziert.

In der US-PS 4 450 138 sind schwefelhaltige Ammoniumverbindungen als Korrosionsschutzmittel beschrieben.

Gegenstand der vorliegenden Erfindung ist eine Zusammensetzung enthaltend ein Schmiermittel, eine Hydraulikflüssigkeit oder eine Metallbearbeitungsflüssigkeit und wenigstens eine Verbindung der Formel I

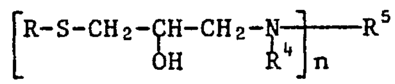 (I)

worin n gleich 1 bis 6 ist und R ein Radikal der Formel

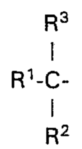

sein kann, wobei $R^1$, $R^2$ und $R^3$ unabhängig voneinander $C_1$-$C_{21}$-Alkyl sind und zusammen nicht mehr als 22 C-Atome besitzen und $R^2$ und $R^3$ ausserdem Wasserstoff sein können, oder worin R $C_5$,$C_{12}$-Cycloalkyl, $C_7$-$C_9$-Aralkyl, unsubstituiertes oder durch $C_1$-$C_4$-Alkyl substituiertes Phenyl oder Naphthyl, Furyl, Furfuryl, Thienyl, Morpholinyl, Imidazolyl, Thiazolyl, Oxazolyl, Imidazolinyl, Thiazolinyl, Oxazolinyl, Benzimidazolinyl, Benzthiazolinyl oder Benzoxazolinyl ist, und worin $R^4$ Wasserstoff, unsubstituiertes oder durch -OH, $-OCH_3$, -CN oder $-N(R^6)_2$ substituiertes $C_1$-$C_{20}$-Alkyl ist, und $R^6$ $C_1$-$C_4$-Alkyl bedeutet, das gegebenenfalls durch -OH substituiert ist, und $R^4$ als $C_1$-$C_{20}$-Alkyl gegebenenfalls durch -O-, -S- oder -N< unterbrochen ist, oder $R^4$ $C_4$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl, unsubstituiertes oder durch ein oder zwei $C_1$-$C_4$-Alkyl oder $-CF_3$, eine oder zwei OH-Gruppen oder durch ein oder zwei $-N(R^7)(R^8)$ substituiertes $C_6$-, $C_{10}$- oder $C_{14}$-Aryl ist, und $R^7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_{10}$-Hetaryl, ein nichtaromatischer $C_2$-$C_5$-Heterocyclus, unsubstituiertes oder durch -OH, $C_1$-$C_4$-Alkoxy oder $-N(R^6)_2$ substituiertes $C_7$-$C_{14}$-Aralkyl ist oder $R^4$ $-CH_2$-CH(OH)-$CH_2$-S-R ist, und $R^5$ unsubstituiertes oder durch $-OCH_3$, -CN oder $-N(R^6)_2$ substituiertes $C_4$-$C_{20}$-Alkyl mit $R^6$ in der vorstehenden Bedeutung ist, das gegebenenfalls durch -O-, -S- oder -N< unterbrochen sein kann, oder $R^5$ durch $N(R^6)$(Tolyl) oder $C_1$-$C_{10}$-Hetaryl substituiertes $C_1$-$C_{20}$-Alkyl ist oder $R^5$ unsubstituiertes $C_4$-$C_{20}$-Alkenyl oder durch ein oder mehrere -CN substituiertes $C_3$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl oder unsubstituiertes oder durch eine oder mehrere $C_1$-$C_4$-Alkylgruppen, die gegebenenfalls durch -NH- oder $-N(R^6)$- unterbrochen sind, eine oder mehrere $C_1$-$C_4$-Alkoxy-, $C_1$-$C_4$-Alkylthio- und/oder OH-Gruppen, ein oder mehrere $-NO_2$, $-CF_3$ und/oder -CN, Hydroxyethoxy, Phenoxy, Ureido, Carbamoyl, Sulfamoyl, Benzolazo, Toluolazo, Anilinocabonyl, Anilinosulfonyl, $-S-CH_2$-CH(OH)-$CH_2$-S-R und/oder durch ein oder zwei $-N(R^7)(R^8)$ substituiertes $C_6$-, $C_{10}$- oder $C_{14}$-Aryl ist, mit $R^7$ und $R^8$ in der vorstehenden Bedeutung wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl oder $C_1$-$C_4$-Alkyl substituiertes $C_1$-$C_{10}$-Hetaryl, unsubstituiertes oder durch -OH, eine oder mehrere $C_1$-$C_4$-Alkoxygruppen oder durch $-N(R^6)_2$ substituiertes $C_7$-$C_{14}$-Aralkyl ist, mit $R^6$ in der vorstehenden Bedeutung, oder $R^5$ $-CH_2$-$CH(OH)CH_2$-S-R ist oder $R_5$ einen 2wertigen $C_2$-$C_{12}$-aliphatischen Rest bedeutet, der sich von einem 2fach durch $-NH_2$ substituierten $C_2$-$C_{12}$-Alkan herleitet und unsubstituiert oder durch -OH, $-OCH_3$ oder $-N(R^6)_2$ substituiert sein kann und gegebenenfalls durch -O-, -S- oder $-N(R^9)$- unterbrochen ist, wobei $R^9$ unsubstituiertes oder durch -OH substituiertes $C_1$-$C_4$-Alkyl oder $-CH_2$-CH(OH)-$CH_2$-S-R ist, oder $R^5$ einen 2- bis 4wertigen $C_6$-$C_{12}$-cycloaliphatischen Rest bedeutet, der sich von einem 2fach bis 4fach durch $-NH_2$ substituierten $C_6$-$C_{12}$-Cycloalkan herleitet und unsubstituiert oder durch $C_1$-$C_4$-Alkyl substituiert sein kann, oder $R^5$ einen Rest der Formel

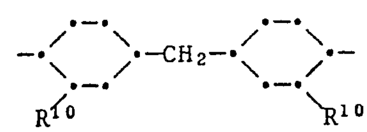

darstellt, worin $R^{10}$ Wasserstoff oder $C_1$-$C_4$-Alkyl ist, oder $R^5$ einen Rest der Formel

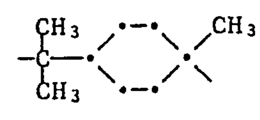

oder einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $-NO_2$ oder $C_1$-$C_4$-Alkyl substituiert sein kann, oder $R^5$ Anthrachinonylen, 2,3-Dihydroanthrachinonylen oder einen Rest der Formel

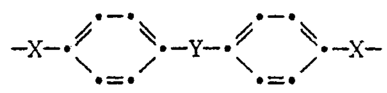

darstellt, worin X $-CH_2-$ oder eine direkte Bindung von Y $-CH_2-$, $-C(C_6H_5)H-$, -S-S-, -NH- oder eine direkte Bindung ist, oder $R^5$ einen 2- oder 3wertigen $C_2$-$C_{10}$-heteroaromatischen Rest bedeutet, der sich von einem 2fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^5$ einen 2wertigen $C_7$-$C_{14}$-araliphatischen Rest bedeutet, der sich von einem 2fach durch $-NH_2$ substituierten $C_7$-$C_{14}$-Aralkan herleitet, oder $R^4$ und $R^5$ zusammen mit dem N-Atom, an das sie gebunden sind, einen $C_1$-$C_7$-azacyclischen Ring bilden, der aromatisch oder nicht aromatisch sein kann und gegebenenfalls ein oder mehrere N-, O- oder S-Atome enthalten kann, wobei das N-Atom gegebenenfalls durch $C_1$-$C_4$-Alkyl, welches seinerseits durch -OH substituiert sein kann, substituiert ist und der $C_1$-$C_7$-azacyclische Ring an einem C-Atom gegebenenfalls durch $C_1$-$C_4$-Alkyl, =O oder =S substituiert sein kann, oder $R^4$ und $R^5$ zusammen mit dem N-Atom, an das sie gebunden sind, 2,2,4-Trimethyl-1,2-dihydrochinolyl, einen Teil eines $C_1$-$C_7$-azacyclischen 2- bis 6wertigen Rings oder ein Radikal der Formeln

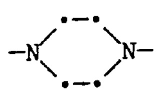 ,

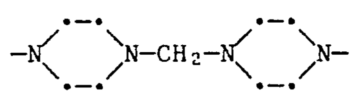

oder

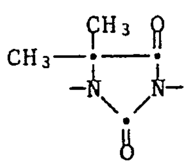

bedeuten.

Stellt R ein Radikal der Formel

$$R^1\text{-}\underset{R^2}{\overset{R^3}{C}}\text{-}$$

dar, so kann es sich um z.B. $R^1$-$CH_2$-, $\genfrac{}{}{0pt}{}{R^1}{R^2}{>}CH$- oder $R^1\text{-}\underset{R^2}{\overset{R^3}{C}}\text{-}$ handeln, wobei $R^1$, $R^2$ und $R^3$ jeweils $C_1$-$C_{21}$-Alkyl sind. Bei $C_1$-$C_{21}$-Alkyl handelt es sich um geradkettige oder verzweigte Substituenten, wie beispielsweise Methyl, Ethyl, n-Propyl, Isopropyl, n-Butyl, Isobutyl, sec.-Butyl, tert.-Butyl, geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl oder Heneicosyl.

Bevorzugt ist

$$R^1\text{-}\underset{R^2}{\overset{R^3}{C}}\text{-} ,$$

worin $R^1$, $R^2$ und $R^3$ zusammen mit dem C-Atom, an das sie gebunden sind, $C_4$-$C_{22}$-Alkyl bilden, wobei keiner dieser Substituenten $R^1$, $R^2$ und $R^3$ Wasserstoff sein darf; besonders bevorzugt ist hier nun $C_4$-$C_{16}$-Alkyl, insbesondere ist tert.-Butyl, tert.-Pentyl, tert.-Nonyl oder tert.-Dodecyl (ex Phillips Petroleum) und ganz speziell tert.-Butyl oder tert.-Nonyl bevorzugt, wobei z.B. unter tert.-Dodecyl solch ein Rest verstanden werden soll, wie er für tertiäres Dodecylmercaptan in «Ullmanns Enzyklopädie der technischen Chemie, 4. Auflage, Band 23, Seite 181 und 182, Verlag Chemie, Weinheim» beschrieben ist.

Stellt R $C_5$-$C_{12}$-Cycloalkyl dar, so handelt es sich beispielsweise um Cyclopentyl, Cyclohexyl, Cycloheptyl, Cyclooctyl, Cyclononyl, Cyclodecyl, Cycloundecyl oder Cyclododecyl, vorzugsweise Cyclohexyl.

Stellt R $C_7$-$C_9$-Aralkyl dar, so handelt es sich beispielsweise um Benzyl, 1- oder 2-Phenylethyl, 3-Phenylpropyl oder 2-Phenylisopropyl, vorzugsweise um Benzyl.

Stellt R durch $C_1$-$C_4$-Alkyl substituiertes Phenyl oder Naphthyl dar, so können Phenyl oder Naphthyl ein- bis dreifach, bevorzugt jedoch einfach substituiert sein; bei $C_1$-$C_4$-Alkyl handelt es sich um Methyl, Ethyl, n-Propyl, Isopropyl, n-Butyl, Isobutyl, sec.-Butyl oder tert.-Butyl.

Stellt $R^4$ $C_1$-$C_{20}$-Alkyl dar, so handelt es sich um geradkettige oder verzweigte Alkylradikale, wie beispielsweise Methyl, Ethyl, n-Propyl, Isopropyl, n-Butyl, Isobutyl, sec.-Butyl, tert.-Butyl, oder geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Eicosyl; bevorzugt sind Isopropyl, tert.-Butyl, Isooctyl, 2-Ethylhexyl, tert.-Nonyl, tert.-Dodecyl oder tert.-Tridecyl. Dabei wird unter Isooctyl ein Rest verstanden, der sich vom Isooctylalkohol ableitet, und eine Mischung verschieden verzweigter Octylreste ist; für tert.-Nonyl sowie für tert.-Dodecyl sind die oben bereits gegebenen Definitionen anzuwenden.

Stellt $R^4$ durch -OH, $-OCH_3$, -CN oder $N(R^6)_2$ substituiertes $C_1$-$C_{20}$-Alkyl dar, so kann $C_1$-$C_{20}$-Alkyl ein- oder mehrfach, bei Substitution durch -OH jedoch höchstens zweifach, substituiert sein, wobei die Substitution in jeder Position möglich ist, jedoch bei einfacher Substitution vorzugsweise terminal ist.

Stellt $R^4$ $C_1$-$C_{20}$-Alkyl dar, das durch -O-, -S- oder -N$<$ unterbrochen ist, so können die Heteroatome sich in jeder möglichen Position befinden, und das $C_1$-$C_{20}$-Alkylradikal kann einfach oder mehrfach unterbrochen sein, wobei die Heteroatome gleich oder verschieden sein können.

Stellen $R^4$ oder $R^5$ $C_4$-$C_{20}$-Alkenyl dar, so handelt es sich um geradkettige oder verzweigte Alkenylradikale, die eine oder mehrere, bevorzugt jedoch eine Doppelbindung enthalten wie beispielsweise n-Butenyl, i-Pentenyl, Pentenyl, Hexenyl, Heptenyl, Octenyl, Nonenyl, Decenyl, Undecenyl, Dodecenyl, Tridecenyl, Tetradecenyl, Pentadecenyl, Hexadecenyl, Heptadecenyl, Octadecenyl, Nonadecenyl oder Eicosenyl.

Stellen $R^4$ oder $R^5$ $C_4$-$C_{20}$-Alkinyl dar, so handelt es sich um geradkettige oder verzweigte Alkinylradikale, die eine oder mehrere, bevorzugt jedoch eine Dreifachbindung enthalten wie beispielsweise n-Butinyl, n-Pentinyl, 1,1-Dimethyl-prop-2-in-1-yl, Hexinyl, Heptinyl, Octinyl, Noninyl, Decinyl, Undecinyl, Dodecinyl, Tridecinyl, Tetradecinyl, Pentadecinyl, Hexadecinyl, Heptadecinyl, Octadecinyl, Nonadecinyl oder Eicosinyl.

Stellen $R^4$ oder $R^5$ $C_6$-$C_{12}$-Cycloalkyl dar, so handelt es sich beispielsweise um Cyclohexyl, Cycloheptyl, Cyclooctyl, Cyclononyl, Cyclodecyl, Cycloundecyl oder Cyclododecyl, vorzugsweise um Cyclohexyl.

Stellen $R^4$ oder $R^5$ durch $C_1$-$C_4$-Alkyl substituiertes $C_6$-$C_{12}$-Cycloalkyl dar, so handelt es sich um ein- oder mehrfach, bevorzugt jedoch einfach, substituiertes $C_6$-$C_{12}$-Cycloalkyl, wobei $C_1$-$C_4$-Alkyl beispielsweise Methyl, Ethyl, n-Propyl, Isopropyl, n-Butyl, Isobutyl, sec.-Butyl oder tert.-Butyl darstellt.

Stellen $R^4$ oder $R^5$ $C_6$-, $C_{10}$- oder $C_{14}$-Aryl dar, so handelt es sich beispielsweise um Phenyl, Naphthyl, Anthryl oder Phenanthryl.

Stellen $R^4$ oder $R^5$ durch $C_1$-$C_4$-Alkyl, $C_1$-$C_4$-Alkoxy und/oder $C_1$-$C_4$-Alkylthio substituiertes $C_6$-, $C_{10}$- oder $C_{14}$-Aryl dar, so kann das Arylradikal ein- oder mehrfach, bevorzugt jedoch ein- bis zweifach, substituiert sein; bei $C_1$-$C_4$-Alkyl handelt es sich um Methyl, Ethyl, n-Propyl, Isopropyl, n-Butyl, Isobutyl, sec.-Butyl oder tert.-Butyl.

Stellen $R^4$ oder $R^5$ durch $-CF_3$, -OH und/oder $-N(R^7)(R^8)$ substituiertes $C_6$-, $C_{10}$- oder $C_{14}$-Aryl dar, so kann das Arylradikal ein- oder zweifach, bevorzugt jedoch einfach, substituiert sein.

Stellen $R^4$ oder $R^5$ $C_1$-$C_{10}$-Hetaryl dar, so kann das Hetarylradikal ein oder mehrere Heteroatome enthalten und gegebenenfalls benzannelliert sein, wobei es sich bei den Heteroatomen beispielsweise um Stickstoff-, Sauerstoff- oder Schwefelatome handelt. Bei $C_1$-$C_{10}$-Hetaryl handelt es sich beispielsweise um Tetrazolyl, Triazolyl, Imidazolyl, Pyrazolyl, Pyrryl, Thiazolyl, Thiadiazolyl, Thiatriazolyl, Thienyl, Furyl, Oxazolyl, Oxadiazolyl, Oxatriazolyl, Pyridyl, Pyridazinyl, Pyrimidyl, Purazinyl, Triazinyl, Tetrazinyl, Benztriazolyl, Benzthiazolyl, Purinyl, Indolyl, Carbazolyl oder Pteridinyl, bevorzugt aber um Tetrazolyl, Triazolyl, Imidazolyl, Pyrazolyl, Thiazolyl, Thiadiazolyl oder Pyridyl.

Stellen $R^4$ oder $R^5$ durch -OH, Phenyl und/oder $C_1$-$C_4$-Alkyl substituiertes $C_1$-$C_{10}$-Hetaryl dar, so kann die Substitution einfach oder mehrfach, bevorzugt jedoch einfach sein; bei $C_1$-$C_4$-Alkyl handelt es sich beispielsweise um Methyl, Ethyl, n-Propyl, Isopropyl, n-Butyl, Isobutyl, sec.-Butyl oder tert.-Butyl.

Stellt $R^4$ einen nicht aromatischen $C_2$-$C_5$-Heterocyclus dar, so handelt es sich beispielsweise um Dihydrooxazolyl, Dihydrothiazolyl, Pyranyl, Thietanyl, Oxetanyl, Morpholinyl, Piperidinyl oder Piperazinyl.

Stellen $R^4$ oder $R^5$ $C_7$-$C_{14}$-Aralkyl dar, so handelt es sich beispielsweise um Benzyl, 1- oder 2-Phenylethyl, 3-Phenylpropyl, 2-Phenylisopropyl, 2-Phenylhexyl, Naphthylmethyl oder Naphthylbutyl, vorzugsweise jedoch um Benzyl oder 2-Phenylethyl.

Stellen $R^4$ oder $R^5$ durch -OH, $C_1$-$C_4$-Alkoxy oder $-N(R^6)_2$ substituiertes $C_7$-$C_{14}$-Aralkyl dar, so kann die Substitution z.B. einfach bis dreifach sein; bei $C_1$-$C_4$-Alkoxy handelt es sich beispielsweise um Methoxy, Ethoxy, n-Propyloxy, Isopropyloxy, n-Butyloxy, Isobutyloxy, sec.-Butyloxy oder tert.-Butyloxy.

Stellt $R^5$ $C_4$-$C_{20}$-Alkyl dar, so handelt es sich um geradkettige oder verzweigte Alkylradikale, wie beispielsweise n-Butyl, Isobutyl, sec.-Butyl, tert.-Butyl, geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Eicosyl.

Stellt $R^5$ durch $-OCH_3$, -CN oder $-N(R^6)_2$ substituiertes $C_4$-$C_{20}$-Alkyl dar, so kann $C_4$-$C_{20}$-Alkyl ein- oder mehrfach substituiert sein, wobei die Substitution in jeder Position möglich ist, jedoch bei einfacher Substitution vorzugsweise terminal ist.

Stellt $R^5$ $C_4$-$C_{20}$-Alkyl dar, das durch -O-, -S- oder -N$\langle$ unterbrochen ist, so können die Heteroatome sich in jeder Position befinden, und das $C_4$-$C_{20}$-Alkylradikal kann einfach oder mehrfach unterbrochen sein, wobei die Heteroatome gleich oder verschieden sein können.

Stellt $R^5$ durch $N(R^6)$(Tolyl) oder durch $C_1$-$C_{10}$-Hetaryl substituiertes $C_1$-$C_{20}$-Alkyl dar, so handelt es sich um ein- oder zweifach, bevorzugt einfach, substituiertes geradkettiges oder verzweigtes $C_1$-$C_{20}$-Alkyl, wobei die Substitution in jeder Position möglich ist, jedoch bei einfacher Substitution bevorzugt terminal ist.

Stellt $R^5$ durch -CN substituiertes $C_3$-$C_{20}$-Alkenyl dar, so kann die Substitution ein- oder mehrfach, bevorzugt mehrfach, sein. Bei $C_3$-$C_{20}$-Alkenyl handelt es sich um geradkettige oder verzweigte Alkenylreste, die eine oder mehrere, bevorzugt eine Doppelbindung besitzen. Insbesondere handelt es sich um den 1,1,3-Tri-cyano-prop-1-en-2-yl-Rest.

Stellt $R^5$ durch durch -NH- oder $-N(R^6)$- unterbrochene $C_1$-$C_4$-Alkylgruppen substituiertes $C_6$-, $C_{10}$- oder $C_{14}$-Aryl dar, so handelt es sich bevorzugt um einfach durch -NH- oder $-N(R^6)$- unterbrochenes $C_1$-$C_4$-Alkyl wie beispielsweise um N-Methyl-aminomethylphenyl oder N-Dimethyl-aminomethylphenyl, welche auch bevorzugt sind.

Stellt $R^5$ durch $-NO_2$, -CN, Hydroxyethoxy, Phenoxy, Ureido, Carbamoyl, Sulfamoyl, Benzolazo, Toluolazo, Anilinocarbonyl, Anilinosulfonyl und/oder $-S-CH_2-CH(OH)-CH_2-S-R$ substituiertes $C_6$-, $C_{10}$- oder $C_{14}$-Aryl dar, so kann die Substitution ein- oder mehrfach, bevorzugt ein- bis dreifach sein. Bevorzugt handelt es sich bei den Arylresten um Phenyl oder Naphthyl, insbesondere um Phenyl.

Stellt $R^5$ einen 2wertigen $C_2$-$C_{12}$-aliphatischen

Rest dar, der sich von einem 2fach durch $-NH_2$ substituierten $C_2$-$C_{12}$-Alkan herleitet, das z.B. durch Anlagerung von $NH_3$ an $C_2$-$C_{12}$-Olefine hergestellt werden kann, kann das $C_2$-$C_{12}$-Alkan geradkettig oder verzweigt sein, so dass es sich beispielsweise um Ethylen, Propylen, Trimethylen, Tetramethylen, Pentamethylen, 2,2-Dimethyl-1,3-Trimethylen, Hexamethylen, Heptamethylen, Octamethylen, Nonamethylen, Decamethylen, Undecymethylen, Dodecamethylen oder 2,5-Hexandiyl handelt.

Stellt $R^5$ einen durch -OH, $-OCH_3$ oder $-N(R^6)_2$ substituierten 2wertigen $C_2$-$C_{12}$-aliphatischen Rest dar, kann die Substitution in jeder Position möglich sein.

Stellt $R^5$ einen durch -O-, -S- oder $-N(R^9)$- unterbrochenen 2wertigen $C_2$-$C_{12}$-aliphatischen Rest dar, so können die Heteroatome sich in jeder Position befinden, und das $C_2$-$C_{12}$-aliphatische Radikal kann einfach oder mehrfach unterbrochen sein, wobei die Heteroatome gleich oder verschieden sein können; z.B. handelt es sich um 3-Thiapentamethylen.

Stellt $R^5$ einen 2- bis 4wertigen $C_6$-$C_{12}$-cycloaliphatischen Rest dar, der sich von einem 2fach bis 4fach durch $-NH_2$ substituierten $C_6$-$C_{12}$-Cycloalkan herleitet, das z.B. durch Anlagerung von $NH_3$ an $C_6$-$C_{12}$-Cycloalkene hergestellt werden kann, so handelt es sich beispielsweise um 1,4-Cyclohexan-diyl, 1,2-Cyclohexan-diyl, 1,3-Cycloheptan-diyl, 1,5-Cyclooctan-diyl, 1,3,5,7-Cyclooctan-tetrayl, 1,3-Cyclononan-diyl, 1,6-Cyclodecan-diyl, 1,4,8-Cycloundecan-triyl oder 1,4,7,10-Cyclododecan-tetrayl. Bevorzugt werden 2wertige $C_6$-$C_{12}$-cycloaliphatische Reste.

Stellt $R^5$ einen 2- bis 4wertigen $C_6$-$C_{12}$-cycloaliphatischen Rest dar, der durch $C_1$-$C_4$-Alkyl substituiert ist, kann die Substitution einfach oder mehrfach, bevorzugt ein- oder zweifach, sein und sich in jeder Position befinden.

Stellt $R^5$ einen 2- oder 3wertigen $C_6$-, $C_{10}$- oder $C_{14}$-aromatischen Rest dar, der z.B. durch Reduktion der entsprechenden Nitroaromaten erhalten werden kann, so handelt es sich beispielsweise um Phenylen, Naphthylen, Anthrylen oder 1,3,5-Benzol-triyl, bevorzugt um Phenylen oder Naphthylen.

Stellt $R^5$ einen 2- oder 3wertigen $C_6$-, $C_{10}$- oder $C_{14}$-aromatischen Rest dar, der durch -OH oder $C_1$-$C_4$-Alkyl substituiert ist, kann die Substitution einfach oder mehrfach, bevorzugt einfach oder zweifach, sein und sich in jeder Position befinden.

Stellt $R^5$ einen 2- oder 3wertigen $C_2$-$C_{10}$-heteroaromatischen Rest dar, der sich von einem 2- oder 3fach durch $-NH_2$ substituierten $C_2$-$C_{10}$-Heteroaromaten herleitet, der z.B. durch Reduktion der entsprechenden Nitroaromaten erhalten werden kann, so kann das Hetarylpolyradikal ein oder mehrere Heteroatome enthalten, wobei es sich bei den Heteroatomen beispielsweise um Stickstoff-, Sauerstoff- oder Schwefelatome handelt. Beispiele für solche Reste sind 2,5-Pyrrol-diyl, 3,5-Triazol-diyl, 3,4-Pyrazol-diyl, 2,4-Thiazol-diyl, 2,6-Pyridin-diyl, 4,6-Pyrimidin-diyl oder 2,4,6-Triazin-triyl, wobei die genannten Reste, insbesondere 2,4,6-Triazin-triyl, bevorzugt sind.

Stellt $R^5$ einen 2- oder 3wertigen $C_2$-$C_{10}$-heteroaromatischen Rest dar, der durch -OH oder $C_6$- oder $C_{10}$-Aryl substituiert ist, kann die Substitution einfach oder mehrfach, bevorzugt einfach oder zweifach, sein und sich in jeder Position befinden.

Stellt $R^5$ einen 2wertigen $C_7$-$C_{14}$-araliphatischen Rest dar, der sich von einem 2fach durch $-NH_2$ substituierten $C_7$-$C_{14}$-Aralkan herleitet, das z.B. durch Anlagerung von $NH_3$ an ein $C_7$-$C_{14}$-Aralken hergestellt werden kann, so handelt es sich beispielsweise um

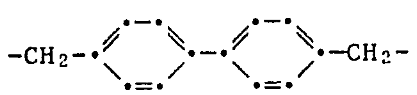 , 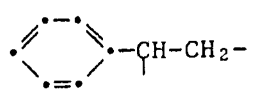 ,

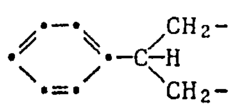 , 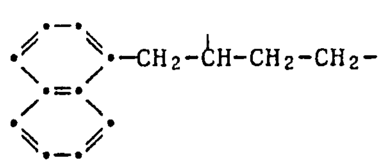 ,

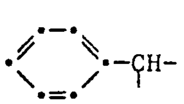 , wobei 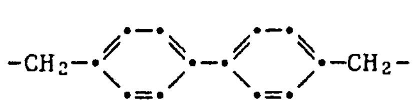

bevorzugt ist.

Bilden $R^4$ und $R^5$ zusammen mit dem N-Atom, an das sie gebunden sind, einen $C_1$-$C_7$-azacyclischen Ring, der aromatisch oder nicht aromatisch sein kann und gegebenenfalls ein oder mehrere N-, O- oder S-Atome enthalten kann, wobei das N-Atom gegebenenfalls durch $C_1$-$C_4$-Alkyl, welches seinerseits durch -OH substituiert sein kann, substituiert ist und der $C_1$-$C_7$-azacyclische Ring an einem C-Atom gegebenenfalls durch $C_1$-$C_4$-Alkyl, =O oder =S substituiert sein kann, so handelt es sich beispielsweise um

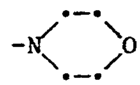 , 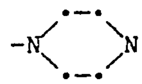 , 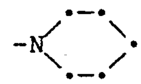 , 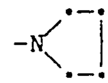 ,

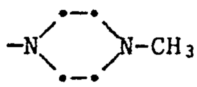 , 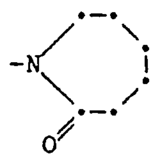 , 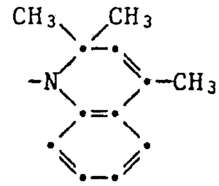 ,

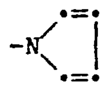 , 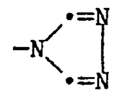 , 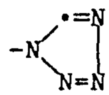 , 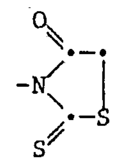 ,

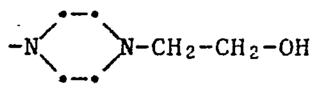 oder 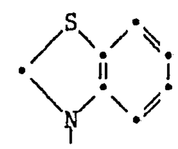 ,

wobei die Beispiele bevorzugt sind.

Bilden $R^4$ und $R_5$ zusammen einem Teil eines $C_1$-$C_7$-azacyclischen 2- bis 6wertigen Rings, so handelt es sich beispielsweise um

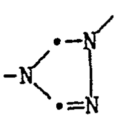 , 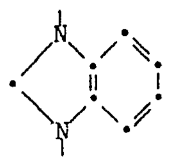

oder

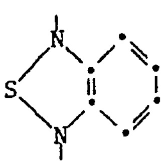 .

Stellen $R^6$, $R^7$, $R^8$, $R^9$ oder $R^{10}$ $C_1$-$C_4$-Alkyl dar, so handelt es sich um Methyl, Ethyl, n-Propyl, Isopropyl, n-Butyl, Isobutyl, sec-Butyl oder tert.-Butyl.

Stellen $R^6$ oder $R^9$ durch -OH substituiertes $C_1$-$C_4$-Alkyl dar, so kann die Substitution einfach oder mehrfach, bevorzugt jedoch einfach, sein. Es handelt sich dabei beispielsweise um 2-Hydroxyethyl oder 2,3-Dihydroxypropyl, bevorzugt um 2-Hydroxyethyl.

Stellt $R^8$ $C_6$- oder $C_{10}$-Aryl dar, so handelt es sich um Phenyl oder Naphthyl.

Eine spezielle Ausführungsform der erfindungsgemässen Zusammensetzung ist die, worin n in der Formel I gleich 1 ist.

Eine weitere Ausführungsform der erfindungsgemässen Zusammensetzung ist die, worin n in der Formel I gleich 2 bis 6, bevorzugt jedoch 2 ist.

Bevorzugt ist eine Zusammensetzung enthaltend ein Schmiermittel, eine Hydraulikflüssigkeit oder eine Metallbearbeitungsflüssigkeit und wenigstens eine Verbindung der Formel I, worin in Formel I R ein Radikal der Formel

$R^3$
|
$R^1$-C-
|
$R^2$

ist, wobei $R^1$, $R^2$ und $R^3$ unabhängig voneinander $C_1$-$C_{18}$-Alkyl sind und zusammen nicht mehr als 22 C-Atome besitzen und $R^2$ und $R^3$ ausserdem Wasserstoff sein können, oder worin R $C_5$-$C_6$-Cycloalkyl, Benzyl, unsubstituiertes oder durch $C_1$-$C_4$-Alkyl substituiertes Phenyl oder unsubstituiertes Naphthyl ist.

Besonders bevorzugt ist eine Zusammensetzung enthaltend ein Schmiermittel, eine Hydraulikflüssigkeit oder eine Metallbearbeitungsflüssigkeit und wenigstens eine Verbindung der Formel I, worin in Formel I R ein Radikal der Formel

$R^3$
|
$R^1$-C-
|
$R^2$

ist, wobei $R^1$, $R^2$ und $R^3$ zusammen mit dem C-Atom, an das sie gebunden sind, $C_4$-$C_{16}$-Alkyl bilden, wobei keiner dieser Substituenten $R^1$, $R^2$ und $R^3$ Wasserstoff sein darf, oder worin R Cyclohexyl, Benzyl, unsubstituiertes oder durch $C_1$-$C_4$-Alkyl substituiertes Phenyl oder unsubstituiertes Naphthyl ist.

Ebenfalls von Interesse ist eine Zusammensetzung enthaltend ein Schmiermittel, eine Hydraulikflüssigkeit oder eine Metallbearbeitungsflüssigkeit und wenigstens eine Verbindung der Formel I, worin in Formel I R ein Radikal der Formel

$R^3$
|
$R^1$-C-
|
$R^2$

ist, wobei $R^1$ $C_1$-$C_{12}$-Alkyl ist und $R^2$ und $R^3$ Wasserstoff sind.

Weiter von speziellem Interesse ist eine Zusammensetzung enthaltend ein Schmiermittel, eine Hydraulikflüssigkeit oder eine Metallbearbeitungsflüssigkeit und wenigstens eine Verbindung der Formel I, worin in Formel I R ein Radikal der Formel

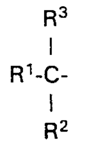

ist, wobei $R^1$ und $R^2$ unabhängig voneinander $C_1$-$C_{12}$-Alkyl sind und zusammen mit dem C-Atom, an das sie gebunden sind, $C_3$-$C_{14}$-Alkyl bilden und $R^3$ Wasserstoff ist.

Speziell bevorzugt ist eine Zusammensetzung enthaltend ein Schmiermittel, eine Hydraulikflüssigkeit oder eine Metallbearbeitungsflüssigkeit und wenigstens eine Verbindung der Formel I, worin in Formel I $R^4$ Wasserstoff, unsubstituiertes oder durch ein oder zwei $C_1$-$C_4$-Alkyl oder $-CF_3$, eine oder zwei OH-Gruppen oder durch ein oder zwei $-N(R^7)(R^8)$ substituiertes $C_6$-, $C_{10}$- oder $C_{14}$-Aryl ist und $R^7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_{10}$-Hetaryl bedeutet, und $R^5$ unsubstituiertes oder durch eine oder mehrere $C_1$-$C_4$-Alkylgruppen, die gegebenenfalls durch -NH- oder $-N(R^6)$- unterbrochen sind, eine oder mehrere $C_1$-$C_4$-Alkoxy-$C_1$-$C_4$-Alkylthio- und/oder OH-Gruppen, ein oder mehrere $-NO_2$, $-CF_3$ und/oder -CN, Hydroxyethoxy, Phenoxy, Ureido, Carbamoyl, Sulfamoyl, Benzolazo, Toluolazo, Anilinocarbonyl, Anilinosulfonyl, $-S-CH_2-CH(OH)-CH_2-S-R$ und/oder durch ein oder zwei $-N(R^7)(R^8)$ substituiertes $C_6$-, $C_{10}$- oder $C_{14}$-Aryl ist, mit $R^7$ und $R^8$ in der vorstehenden Bedeutung, wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl und/oder $C_1$-$C_4$-Alkyl substituiertes $C_1$-$C_{10}$-Hetaryl ist, oder $R^5$ einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $NO_2$ oder $C_1$-$C_4$-Alkyl substituiert sein kann, oder $R^5$ Anthrachinonylen, 2,3-Dihydroanthrachinonylen oder einen Rest der Formel

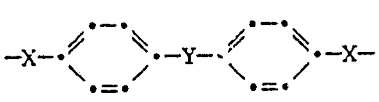

darstellt, worin X eine direkte Bindung und Y $-CH_2$-, $-C(C_6H_5)H$-, -S-S-, -NH- oder eine direkte Bindung ist, oder $R^5$ einen 2- oder 3wertigen $C_2$-$C_{10}$-heteroaromatischen Rest bedeutet, der sich von einem 2-fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^4$ und $R^5$ zusammen mit dem N-Atom, an das sie gebunden sind, einen $C_1$-$C_7$-azacyclischen Ring bilden, der aromatisch ist und gegebenenfalls ein oder mehrere N-, O- oder S-Atome enthalten kann, oder $R^4$ und $R^5$ zusammen mit dem N-Atom, an das sie gebunden sind, einen 2,2,4-Trimethyl-1,2-dihydrochinolylrest bedeuten.

Ein weiterer Gegenstand der Erfindung betrifft neue Verbindungen der Formel Ia

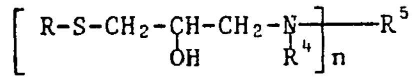

(Ia)

worin n gleich 1 bis 6 ist und R ein Radikal der Formel

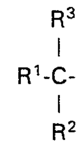

bedeutet, wobei $R^1$, $R^2$ und $R^3$ zusammen mit dem C-Atom, an das sie gebunden sind, $C_5$-$C_{20}$-Alkyl bilden und keiner dieser Substituenten $R^1$, $R^2$ und $R^3$ Wasserstoff sein darf und worin $R^4$ Wasserstoff, unsubstituiertes oder durch -OH, $-OCH_3$-, -CN oder $-N(R^6)_2$ substituiertes $C_1$-$C_{20}$-Alkyl ist, und $R^6$ $C_1$-$C_4$-Alkyl bedeutet, das gegebenenfalls durch -OH substituiert ist, und $R^4$ als $C_1$-$C_{20}$-Alkyl gegebenenfalls durch -O-, -S- oder -N$\langle$ unterbrochen ist, oder $R^4$ $C_4$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl, unsubstituiertes oder durch ein oder zwei $C_1$-$C_4$-Alkyl oder $-CF_3$, eine oder zwei OH-Gruppen oder durch ein oder zwei $-N(R^7)(R^8)$ substituiertes $C_6$-, $C_{10}$- oder $C_{14}$-Aryl ist, und $R_7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_{10}$-Hetaryl, ein nichtaromatischer $C_2$-$C_5$-Heterocyclus, unsubstituiertes oder durch -OH, $C_1$-$C_4$-Alkoxy oder $-N(R^6)_2$ substituiertes $C_7$-$C_{14}$-Aralkyl ist oder $R^4$ $-CH(OH)-CH_2-S-R$ ist, und $R^5$ unsubstituiertes oder durch $-OCH_3$, -CN oder $-N(R^6)_2$ substituiertes $C_5$-$C_{20}$- Alkyl mit $R^6$ in der vorstehenden Bedeutung ist, das gegebenenfalls durch -O-, -S- oder -N$\langle$ unterbrochen sein kann, oder $R^5$ durch $N(R^6)$(Tolyl) oder $C_1$-$C_{10}$-Hetaryl substituiertes $C_1$-$C_{20}$-Alkyl ist, oder $R^5$ unsubstituiertes $C_4$-$C_{20}$-Alkenyl, oder durch ein oder mehrere -CN substituiertes $C_3$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl oder unsubstituiertes oder durch eine oder mehrere $C_1$-$C_4$-Alkylgruppen, die gegebenenfalls durch -NH- oder $-N(R^6)$- unterbrochen sind, eine oder mehrere $C_1$-$C_4$-Alkoxy-$C_1$-$C_4$-Alkylthio- und/oder OH-Gruppen, ein oder mehrere $-NO_2$, $-CF_3$ und/oder -CN, Hydroxyethoxy, Phenoxy, Ureido, Carbamoyl, Sulfamoyl, Benzolazo, Toluolazo, Anilinocarbonyl, Anilinosulfonyl, $-S-CH_2-CH(OH)-CH_2$-$-S-R$ und/oder durch ein oder zwei $N(R^7)(R^8)$ substituiertes $C_6$-, $C_{10}$- oder $C_{14}$-Aryl ist, mit $R^7$ und $R^8$ in der vorstehenden Bedeutung, wobei $R^8$ zusätzlich Acetyl oder Methoxyphenyl darstellt, oder worin $R^5$

Anthrachinonyl, Hydroxyanthrachinonyl, unsubstituiertes oder durch -OH, Phenyl oder $C_1$-$C_4$-Alkyl substituiertes $C_1$-$C_{10}$-Hetaryl, unsubstituiertes oder durch -OH, eine oder mehrere $C_1$-$C_4$-Alkoxygruppen oder durch $-N(R^6)_2$ substituiertes $C_7$-$C_{14}$-Aralkyl ist, mit $R^6$ in der vorstehenden Bedeutung, oder $R^5$ $-CH_2-CH(OH)-CH_2-S-R$ ist oder $R^5$ einen 2wertigen $C_2$-$C_{12}$-aliphatischen Rest bedeutet, der sich von einem 2fach durch $-NH_2$ substituierten $C_2$-$C_{12}$-Alkan herleitet und unsubstituiert oder durch -OH, $-OCH_3$ oder $-N(R^6)_2$ mit $R^6$ in der vorstehenden Bedeutung substituiert sein kann und gegebenenfalls durch -O-, -S- oder $-N(R^9)$- unterbrochen ist, wobei $R^9$ unsubstituiertes oder durch -OH substituiertes $C_1$-$C_4$-Alkyl oder $-CH_2-CH(OH)-CH_2-S-R$ ist, oder $R^5$ einen 2- bis 4wertigen $C_6$-$C_{12}$-cycloaliphatischen Rest bedeutet, der sich von einem 2fach bis 4fach durch $-NH_2$ substituierten $C_6$-$C_{12}$-Cycloalkan herleitet und unsubstituiert oder durch $C_1$-$C_4$-Alkyl substituiert sein kann, oder $R^5$ einen Rest der Formel

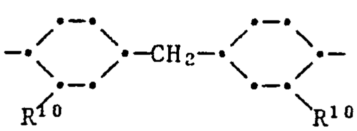

darstellt, worin $R^{10}$ Wasserstoff oder $C_1$-$C_4$-Alkyl ist, oder $R^5$ einen Rest der Formel

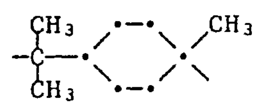

oder einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $-NO_2$ oder $C_1$-$C_4$-Alkyl substituiert sein kann, oder $R^5$ Anthrachinonylen, 2,3-Dihydroanthrachinonylen oder einen Rest der Formel

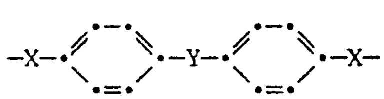

darstellt, worin X $-CH_2$- oder eine direkte Bindung und Y $-CH_2$-, $-C(C_6H_5)$-, -S-S-, -NH- oder eine direkte Bindung ist, oder $R^5$ einen 2- oder 3wertigen $C_2$-$C_{10}$-heteroaromatischen Rest bedeutet, der sich von einem 2fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^5$ einen 2wertigen $C_7$-$C_{14}$-araliphatischen Rest bedeutet, der sich von einem 2fach durch $-NH_2$ substituierten $C_7$-$C_{14}$-Aralkan herleitet, oder $R^4$ und $R^5$ zusammen mit dem N-Atom, an das sie gebunden sind, einen $C_1$-$C_7$-azacyclischen Ring bilden, der aromatisch oder nicht aromatisch sein kann und gegebenenfalls ein oder mehrere N-, O- oder S-Atome enthalten kann, wobei das N-Atom gegebenenfalls durch $C_1$-$C_4$-Alkyl, welches seinerseits durch -OH substituiert sein kann, substituiert ist und der $C_1$-$C_7$-azacyclische Ring an einem C-Atom gegebenenfalls durch $C_1$-$C_4$-Alkyl, =O oder =S substituiert sein kann, oder $R^4$ und $R^5$ zusammen mit dem N-Atom, an das sie gebunden sind, 2,2,4-Trimethyl-1,2-dihydrochinolyl, einen Teil eines $C_1$-$C_7$-azacyclischen 2- bis 6wertigen Rings oder ein Radikal der Formeln

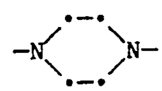 , 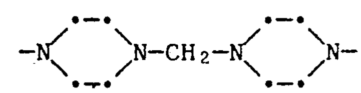

oder

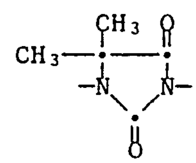

bedeuten.

Eine spezielle Ausführungsform besteht in Verbindungen der Formel Ia, worin n gleich 1 ist.

Eine weitere Ausführungsform besteht in Verbindungen der Formel Ia, worin n gleich 2 bis 6 ist, bevorzugt jedoch 2 ist.

Weiter bevorzugt sind Verbindungen der Formel Ia, worin in Formel Ia $R^4$ Wasserstoff, unsubstituiertes oder durch ein oder zwei $C_1$-$C_4$-Alkyl oder $-CF_3$, eine oder zwei OH-Gruppen oder durch ein oder zwei $-N(R^7)(R^8)$ substituiertes $C_6$-, $C_{10}$- oder $C_{14}$-Aryl ist und $R^7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_6$-Hetaryl bedeutet, und $R^5$ unsubstituiertes oder durch eine oder mehrere $C_1$-$C_4$-Alkylgruppen, die gegebenenfalls durch -NH- oder $-N(R^6)$- unterbrochen sind, eine oder mehrere $C_1$-$C_4$-Alkoxy-$C_1$-$C_4$-Alkylthio- und/oder OH-Gruppen, ein oder mehrere $-NO_2$, $-CF_3$ und oder -CN, Hydroxyethoxy, Phenoxy, Ureido, Carbamoyl, Sulfamoyl, Benzolazo, Toluolazo, Anilinocarbonyl, Anilinosulfonyl, $-S-CH_2-CH(OH)-CH_2-S-R$ und/oder durch ein oder zwei $-N(R^7)(R^8)$ substituiertes $C_6$-, $C_{10}$- oder $C_{14}$-Aryl ist, mit $R^7$ und $R^8$ in der vorstehenden Bedeutung, wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl und/oder $C_1$-$C_4$-Alkyl substituiertes $C_1$-$C_{10}$-Hetaryl ist, oder $R^5$ einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $-NO_2$ oder $C_1$-$C_4$-Alkyl substituiert sein kann, oder $R^5$ Anthrachinonylen, 2,3-Dihydroanthrachinonylen oder einen Rest der Formel

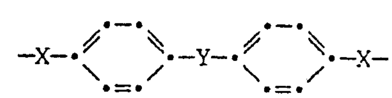

darstellt, worin X eine direkte Bindung und Y $-CH_2-$, $-C(C_6H_6)H-$, -S-S-, -NH- oder eine direkte Bindung ist, oder $R^5$ einen 2- oder 3wertigen $C_2$-$C_{10}$-heteroaromatischen Rest bedeutet, der sich von einem 2-fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^4$ und $R^5$ zusammen mit dem N-Atom, an das sie gebunden sind, einen $C_1$-$C_7$-azacyclischen Ring bilden, der aromatisch ist und gegebenenfalls ein oder mehrere N-, O- oder S-Atome enthalten kann, oder $R^4$ und $R^5$ zusammen mit dem N-Atom, an das sie gebunden sind, einen 2,2,4-Trimethyl-1,2-dihydrochinolylrest bedeuten.

Insbesondere bevorzugt sind Verbindungen der Formel Ia, worin R ein Radikal der Formel

$$R^1-\overset{\displaystyle R^3}{\underset{\displaystyle R^2}{C}}-$$

bedeutet, wobei $R^1$, $R^2$ und $R^3$ zusammen mit dem C-Atom, an das sie gebunden sind, $C_8$-$C_{12}$-Alkyl bilden und keiner dieser Substituenten $R^1$, $R^2$ und $R^3$ Wasserstoff sein darf.

Im weiteren betrifft die vorliegende Erfindung die Verbindung 1-Ethylthio-3-(triazol-3-yl)-amino-propan-2-ol.

Beispiele für Verbindungen der Formel I sind:

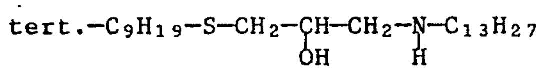

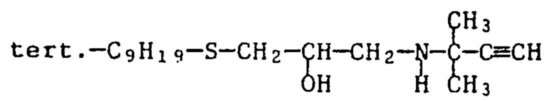

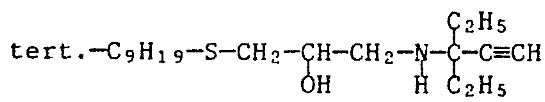

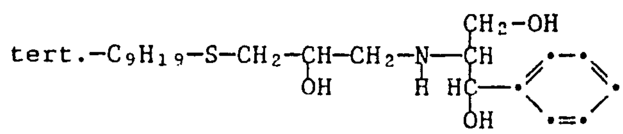

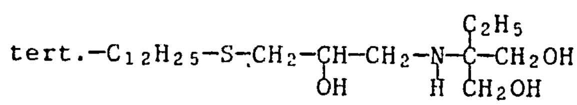

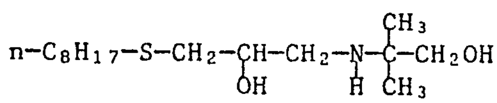

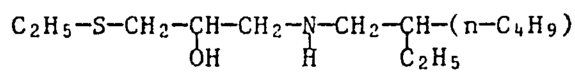

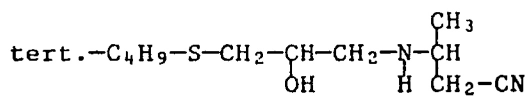

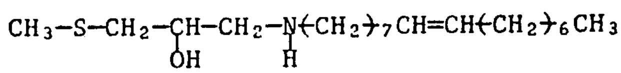

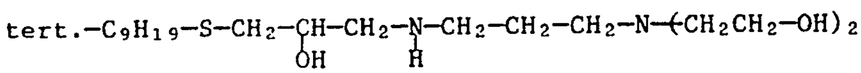

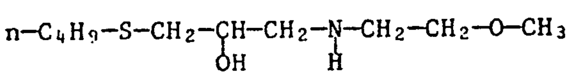

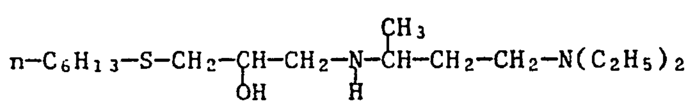

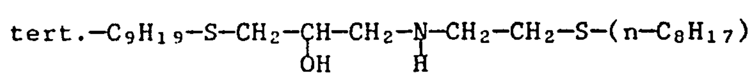

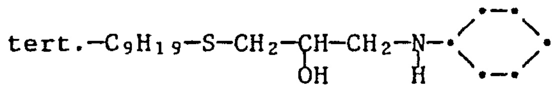

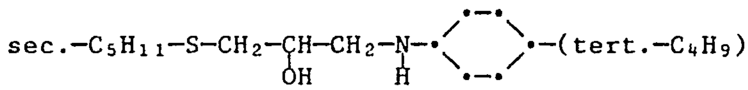

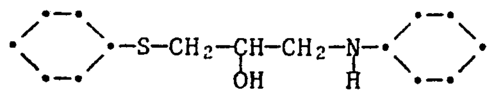

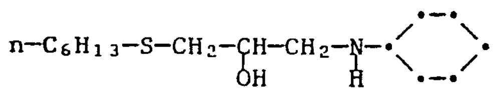

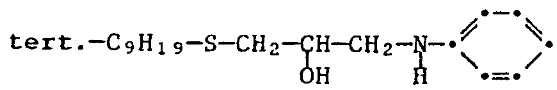

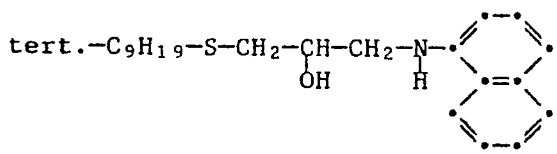

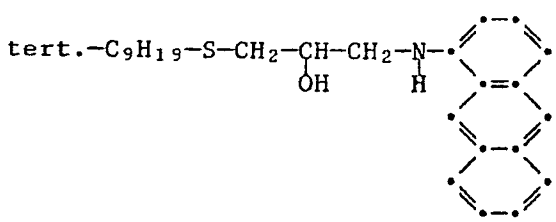

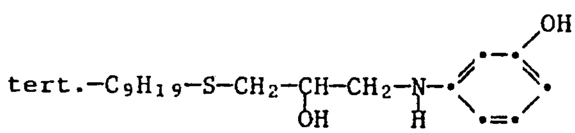

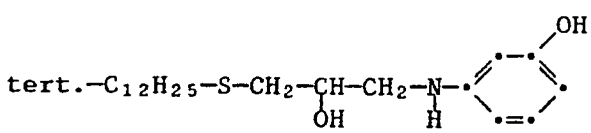

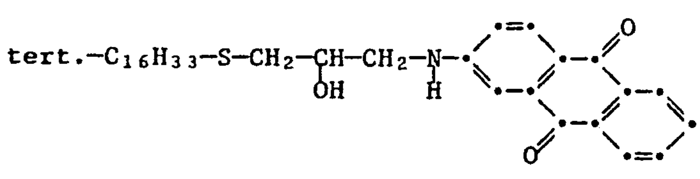

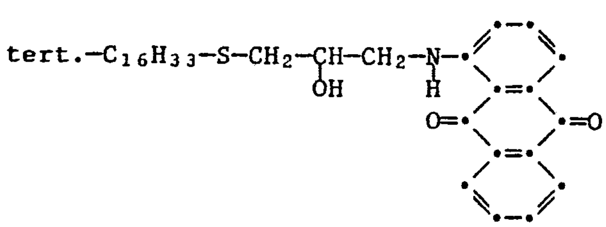

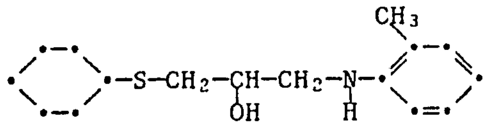

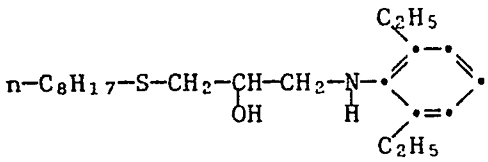

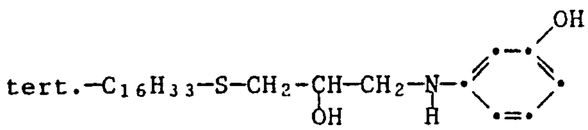

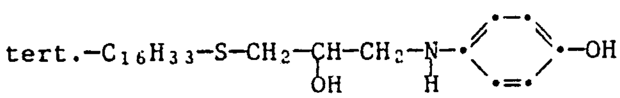

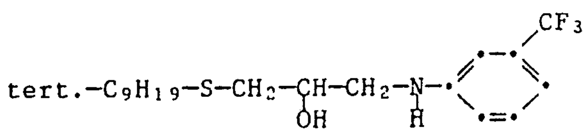

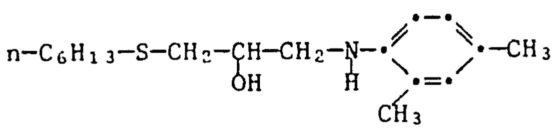

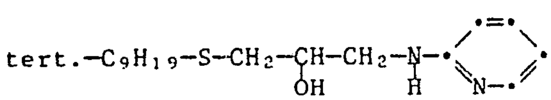

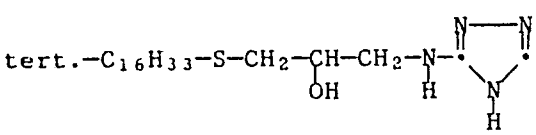

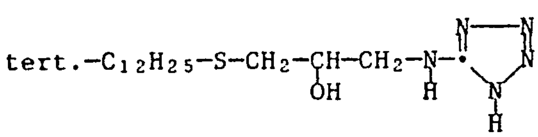

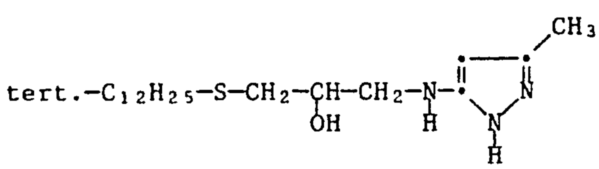

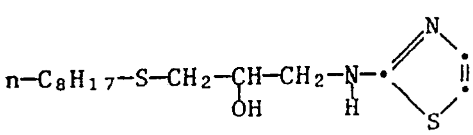

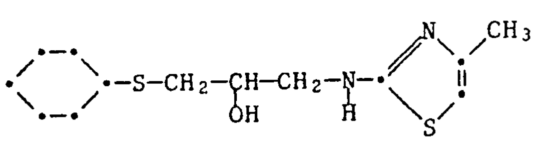

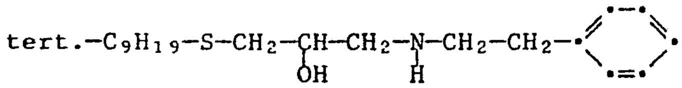

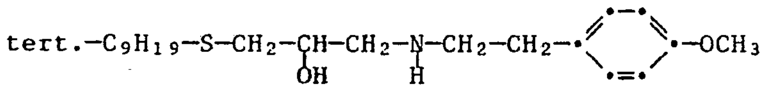

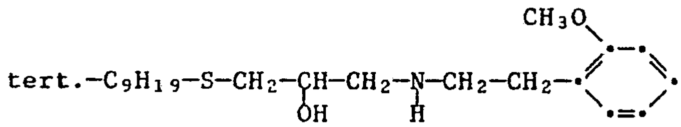

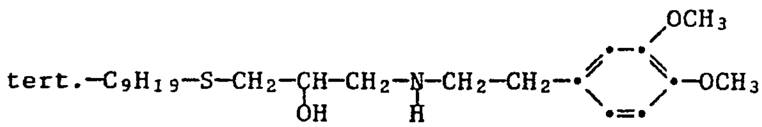

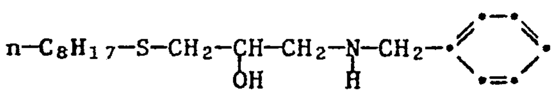

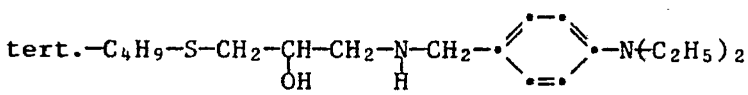

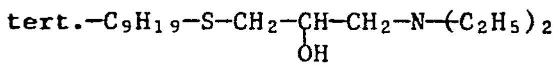

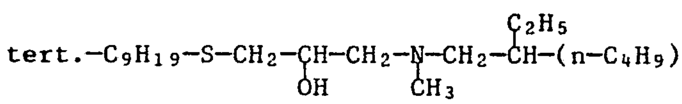

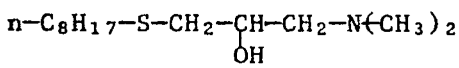

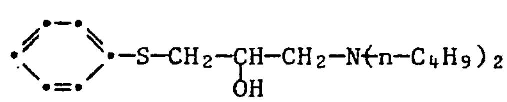

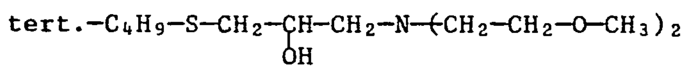

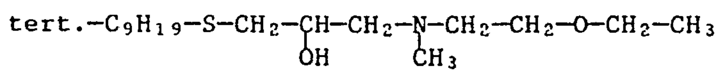

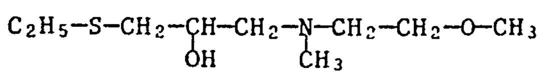

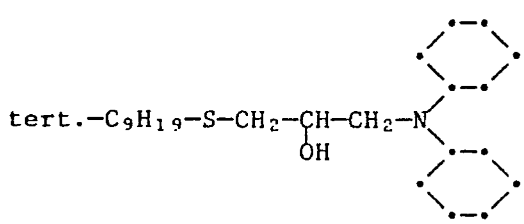

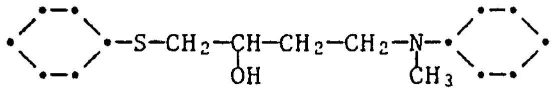

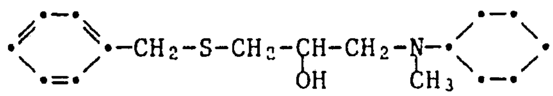

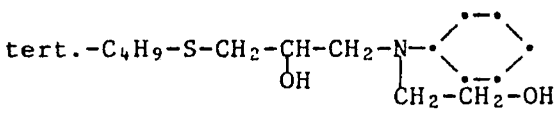

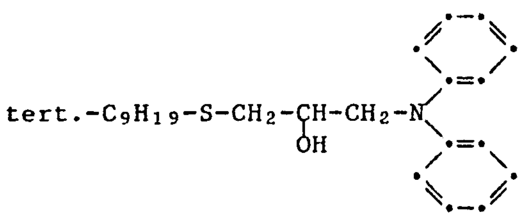

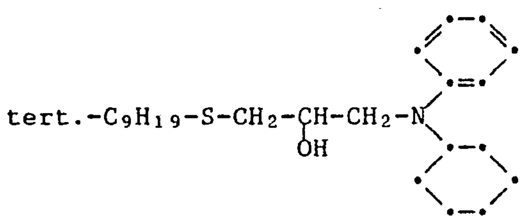

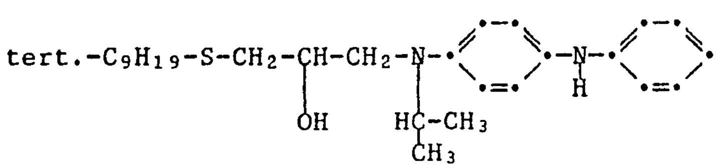

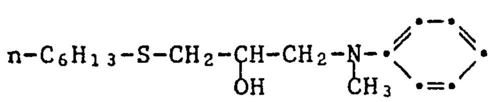

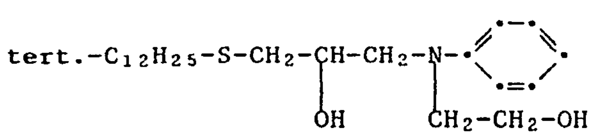

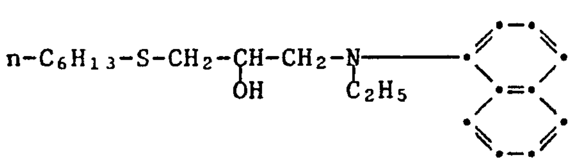

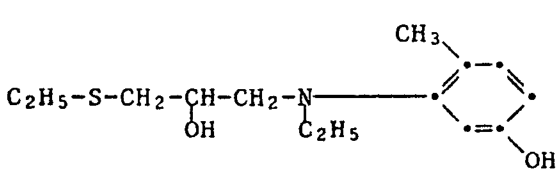

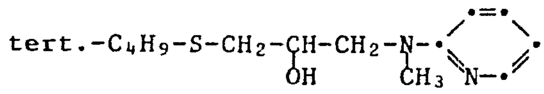

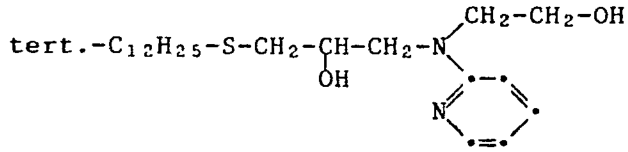

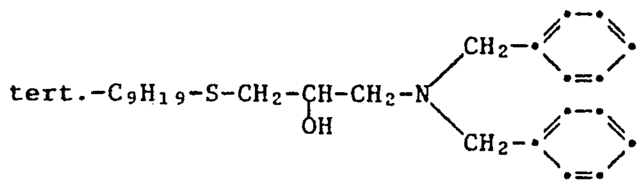

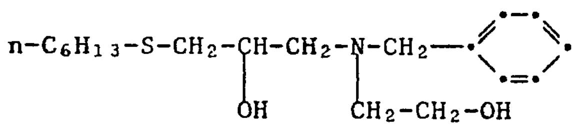

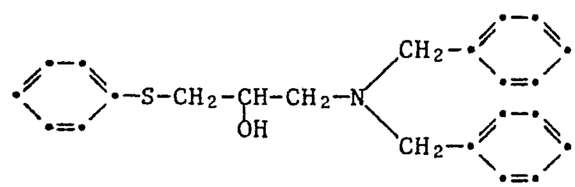

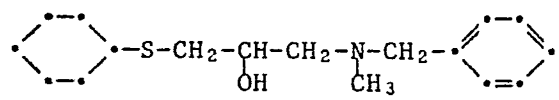

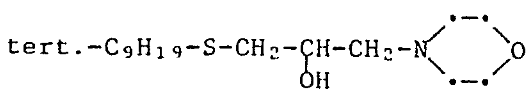

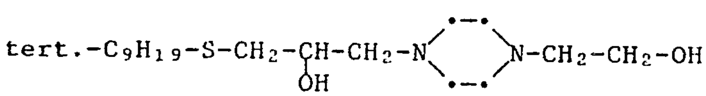

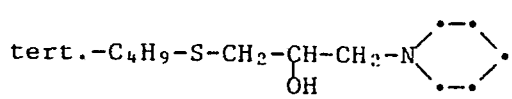

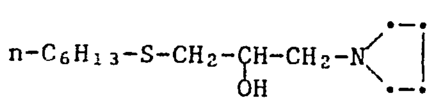

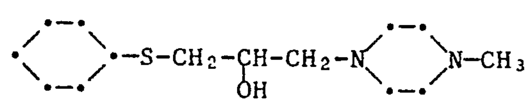

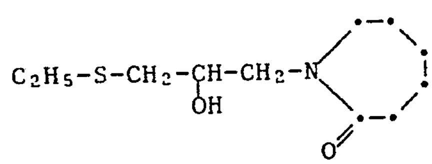

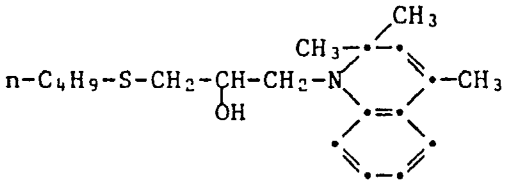

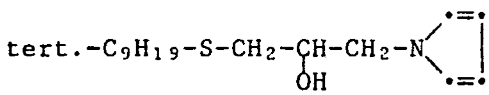

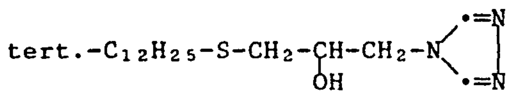

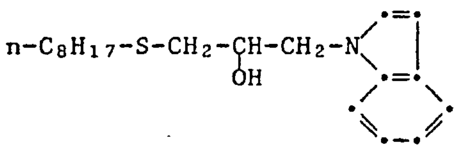

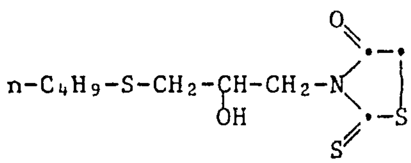

$(tert.-C_9H_{19}-S-CH_2-CH-CH_2)_2NH$
OH

$(n-C_8H_{17}-S-CH_2-CH-CH_2)_2NH$
OH

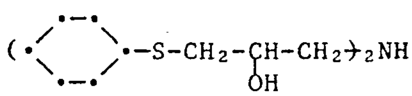

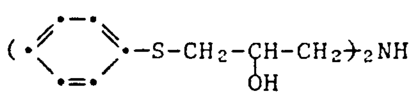

$(tert.-C_4H_9-S-CH_2-CH-CH_2)_2NH$
OH

OH
$sec.-C_4H_9-S-CH_2-CH-CH_2$
NH
$n-C_4H_9-S-CH_2-CH-CH_2$
OH

$(tert.-C_9H_{19}-S-CH_2-CH-CH_2)_2N-CH_2-CH_2-OH$
OH

$(tert.-C_9H_{19}-S-CH_2-CH-CH_2)_2N-(n-C_4H_9)$
OH

$(C_2H_5-S-CH_2-\underset{OH}{CH}-CH_2)_2N-CH_2-CH_2-OH$

$(n-C_4H_9-S-CH_2-\underset{OH}{CH}-CH_2)_2N-CH_2-CH_2-CH_2-N-(CH_2-CH_2-OH)_2$

$(n-C_6H_{13}-S-CH_2-\underset{OH}{CH}-CH_2)_2N-(CH_2)_8CH=CH-(CH_2)_7CH_3$

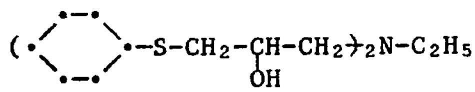

$(C_2H_5-S-CH_2-\underset{OH}{CH}-CH_2)_2N-CH_2-\underset{OH}{CH}-\underset{OH}{CH_2}$

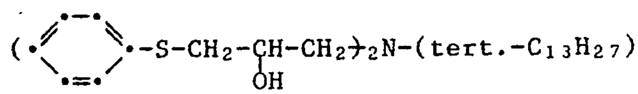

$(n-C_6H_{13}-S-CH_2-\underset{OH}{CH}-CH_2)_2N-CH_2-CH_2-O-CH_3$

$(tert.-C_9H_{19}-S-CH_2-\underset{OH}{CH}-CH_2)_2N-CH-CH_2-S-(n-C_8H_{17})$

$(tert.-C_4H_9-S-CH_2-\underset{OH}{CH}-CH_2)_2N-(tert.-C_{13}H_{27})$

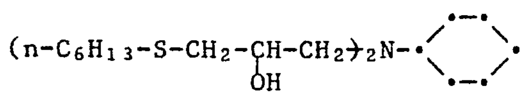

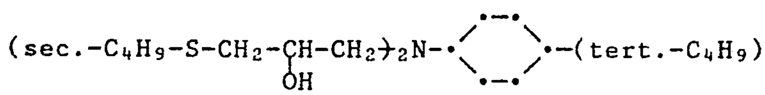

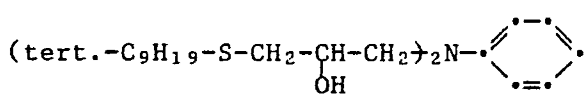

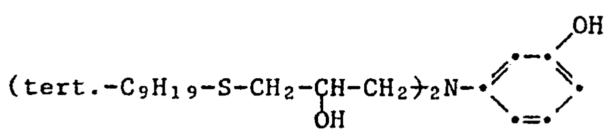

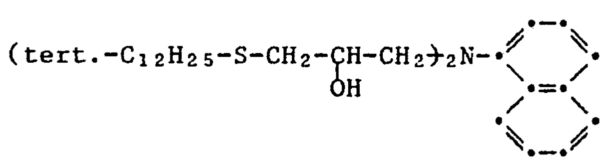

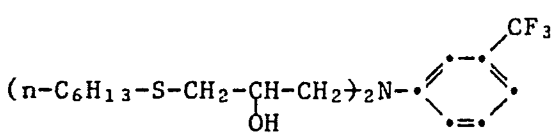

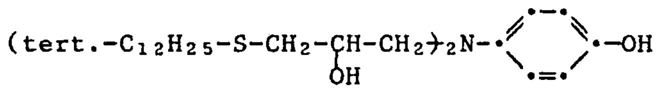

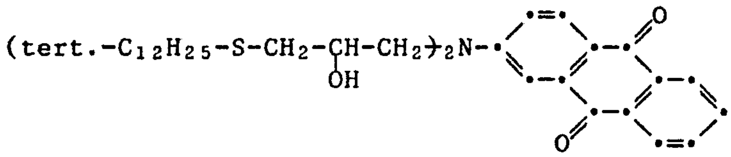

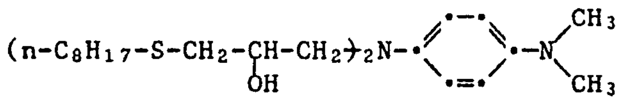

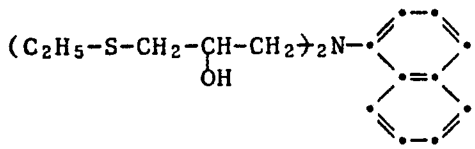

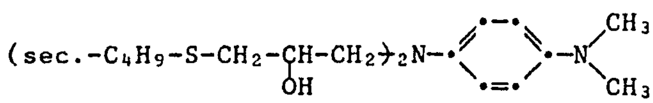

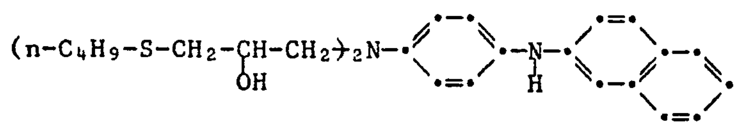

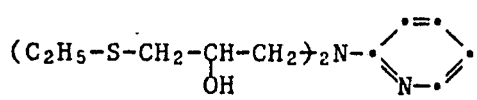

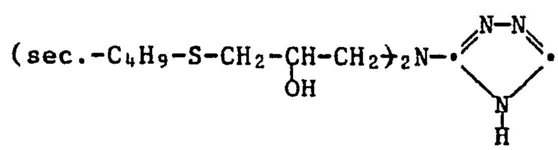

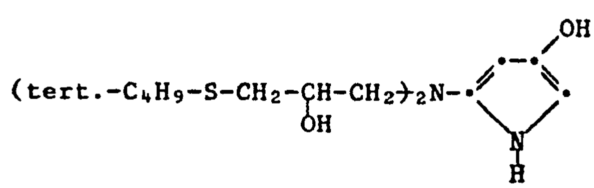

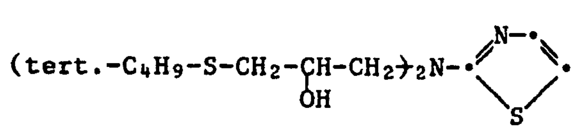

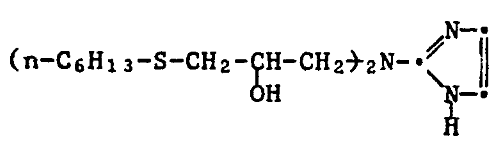

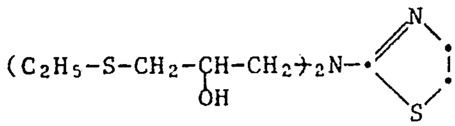

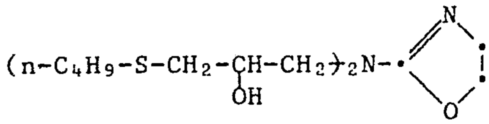

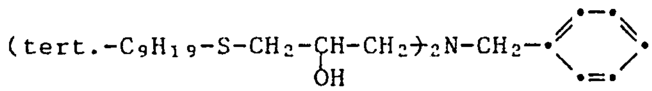

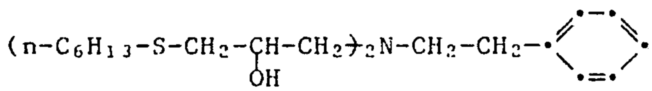

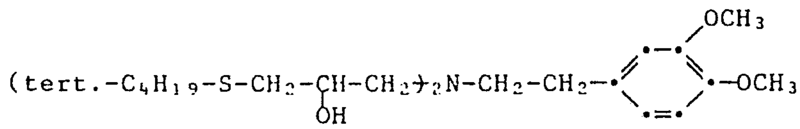

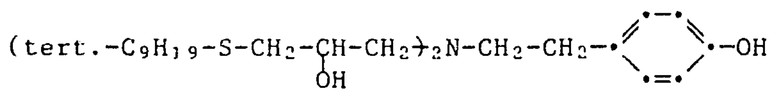

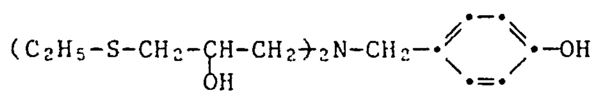

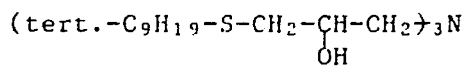

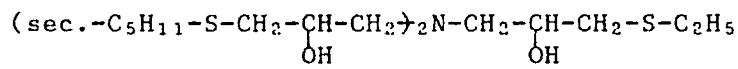

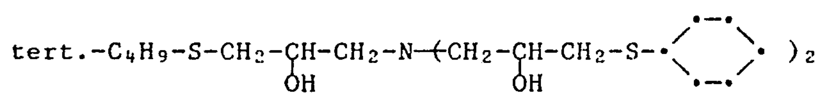

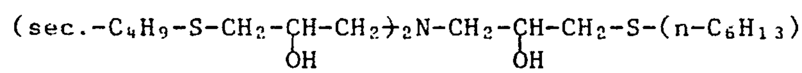

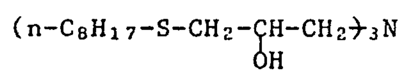

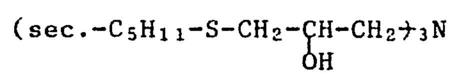

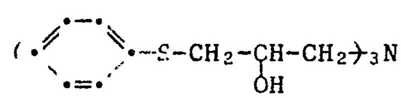

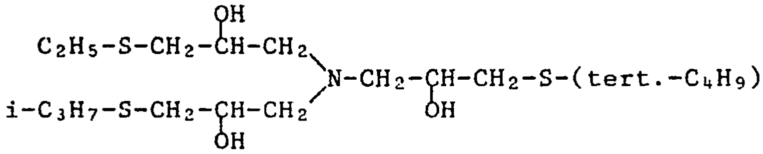

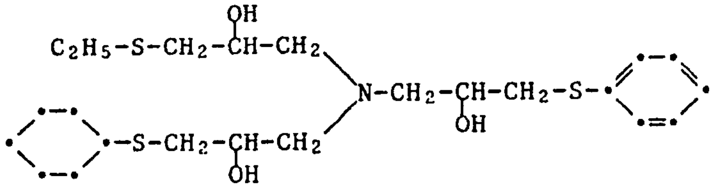

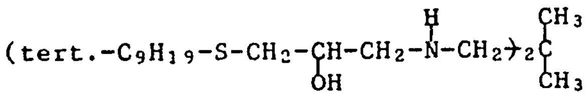

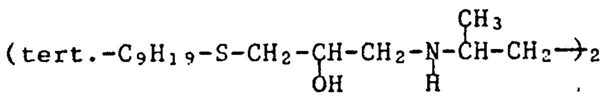

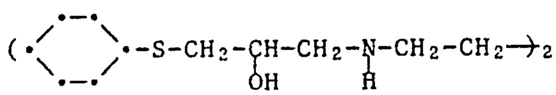

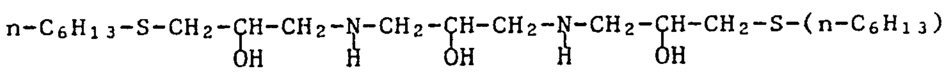

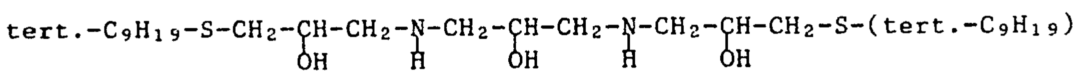

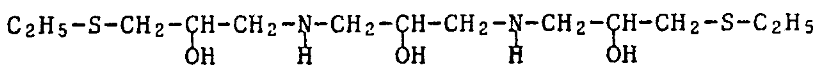

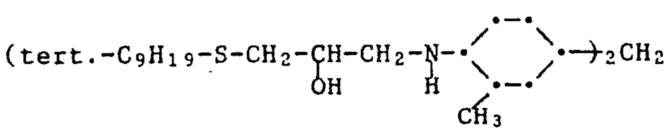

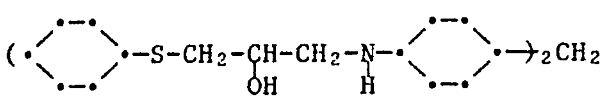

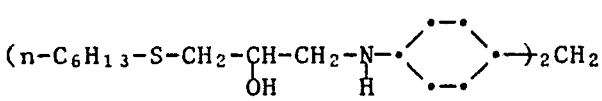

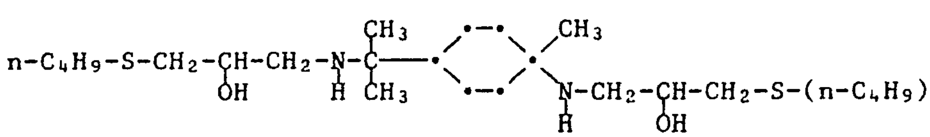

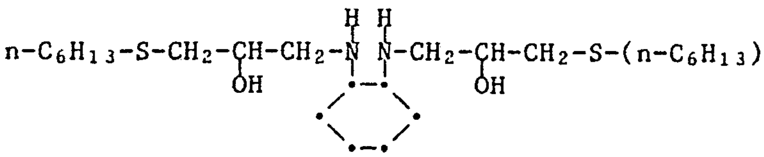

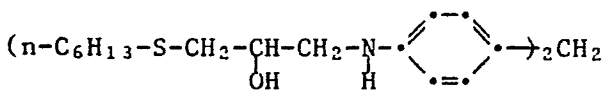

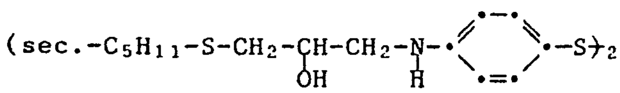

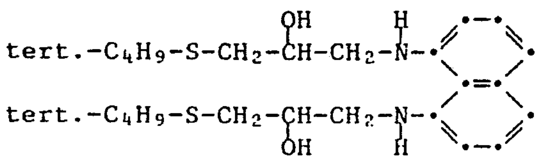

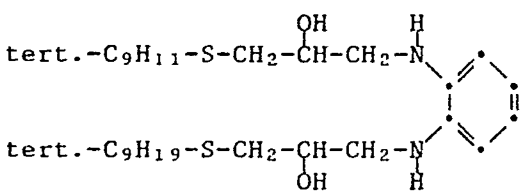

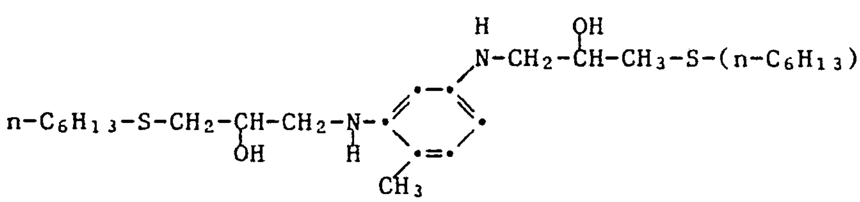

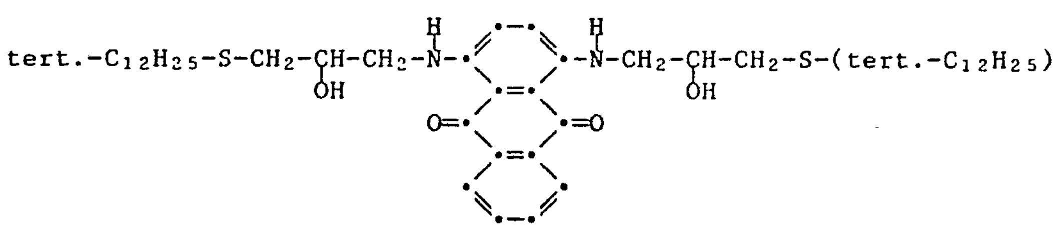

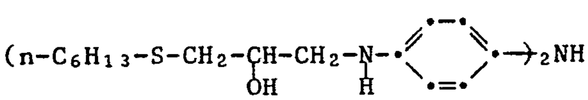

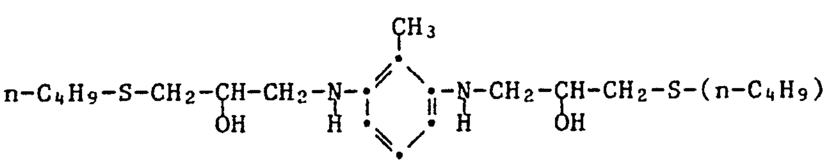

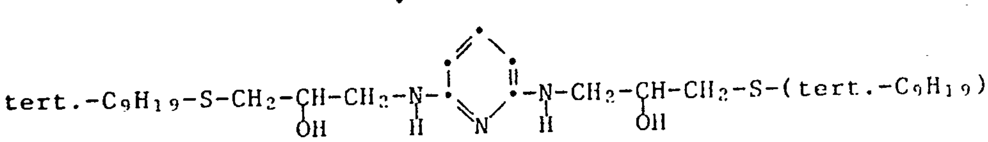

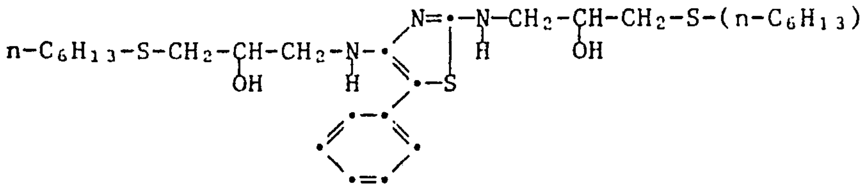

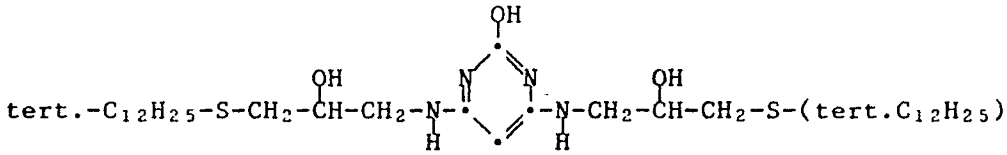

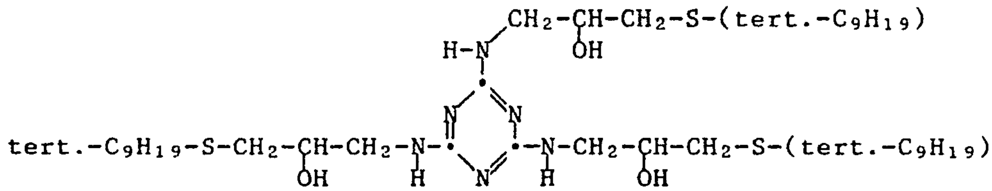

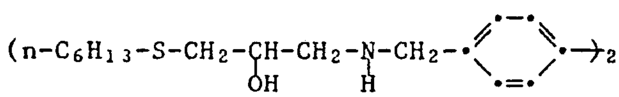

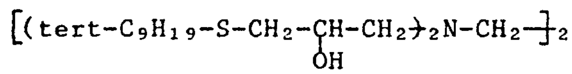

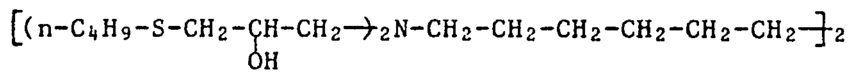

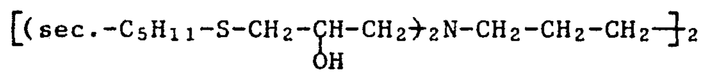

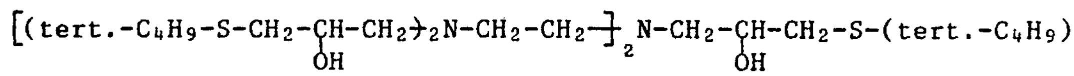

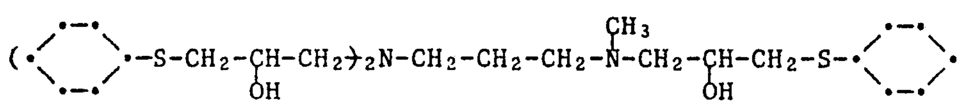

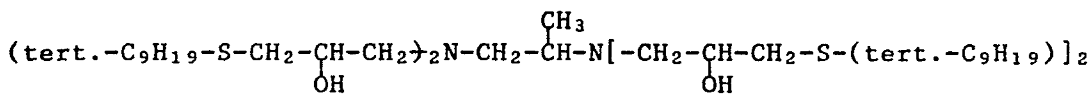

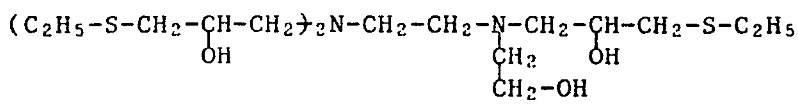

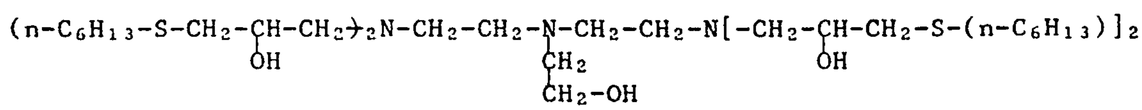

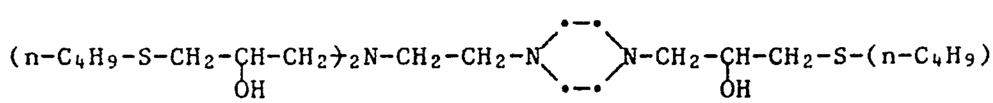

$$(C_2H_5-S-CH_2-\underset{OH}{CH}-CH_2)_2N-(CH_2-CH_2-O)_5CH_2-CH_2-N(-CH_2-\underset{OH}{CH}-CH_2-S-C_2H_5)_2$$

$$[(n-C_6H_{13}-S-CH_2-\underset{OH}{CH}-CH_2)_2N-CH_2-CH_2-]_2S$$

$$(C_2H_5-S-CH_2-\underset{OH}{CH}-CH_2)_2N-(CH_2)_3-O-(CH_2)_4-O-(CH_2)_3-N(CH_2\underset{OH}{CH}-CH_2-S-C_2H_5)_2$$

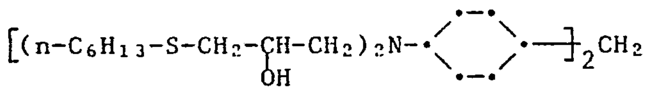

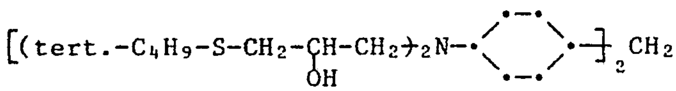

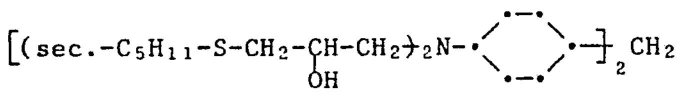

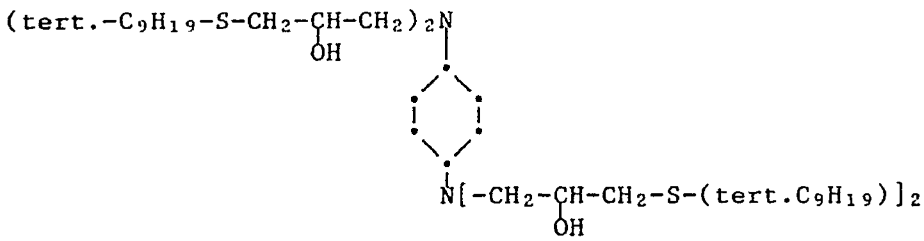

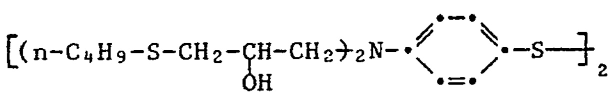

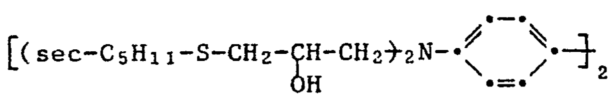

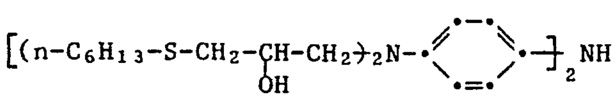

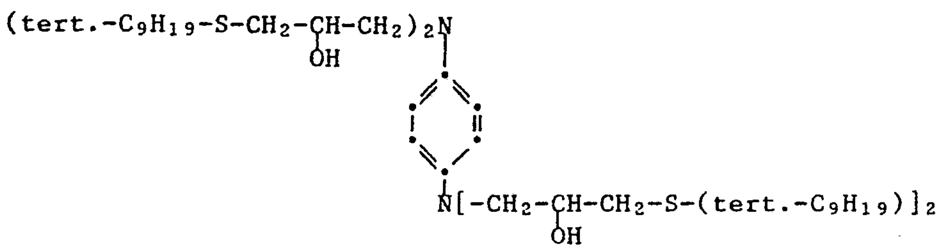

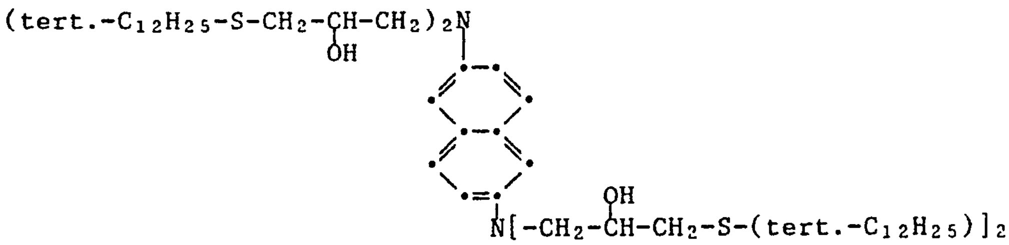

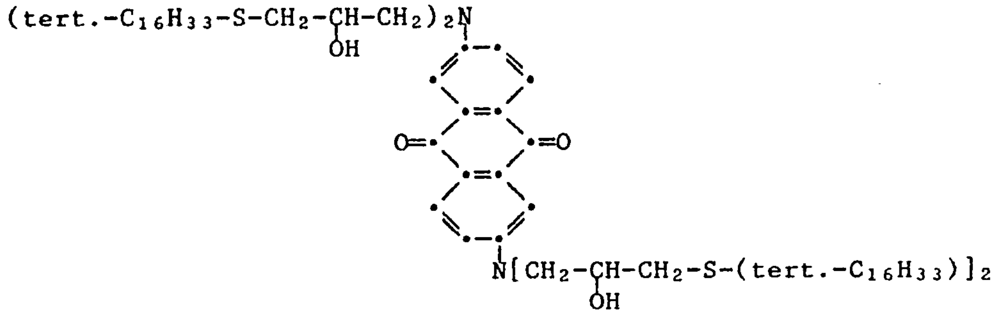

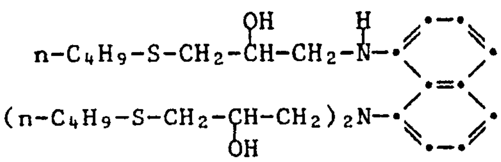

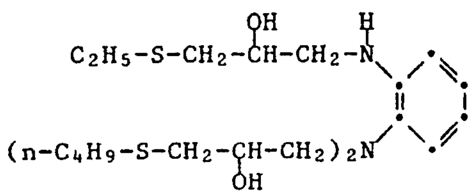

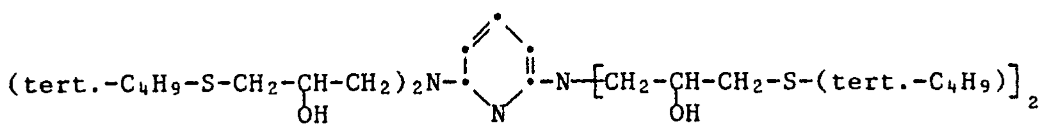

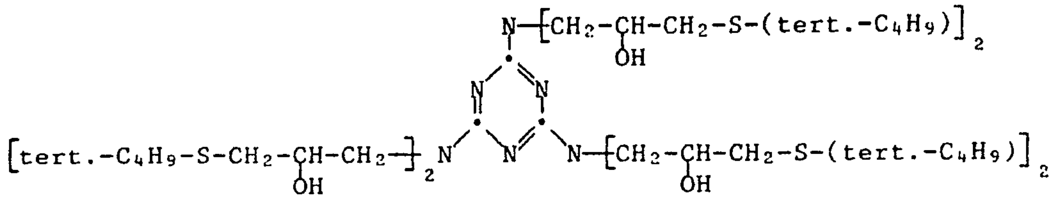

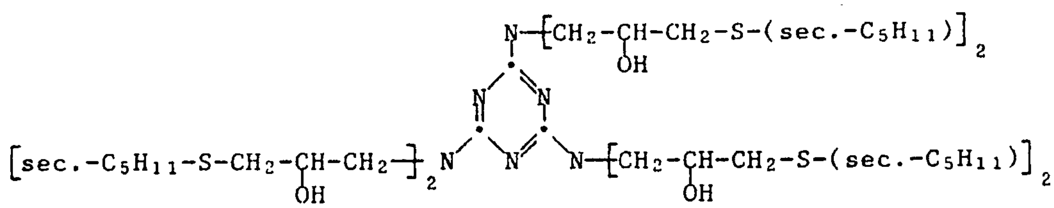

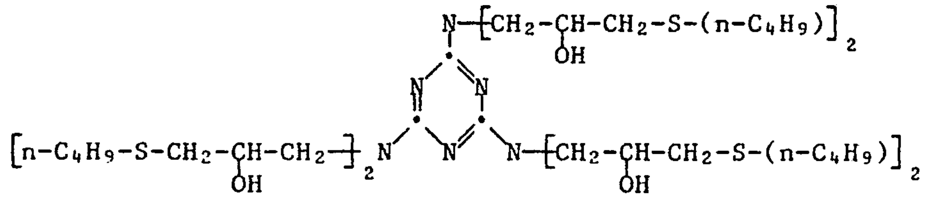

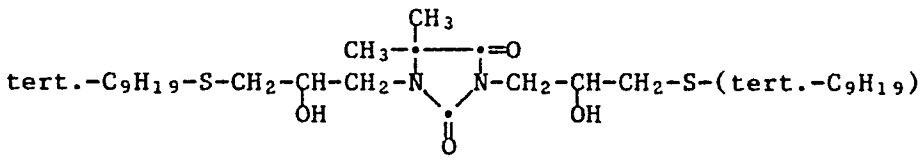

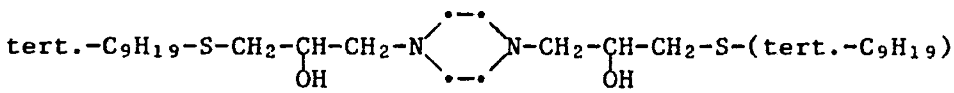

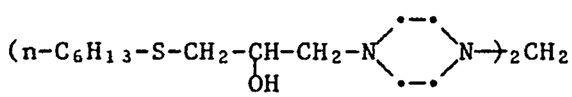

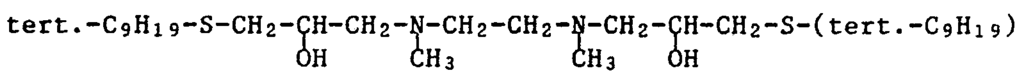

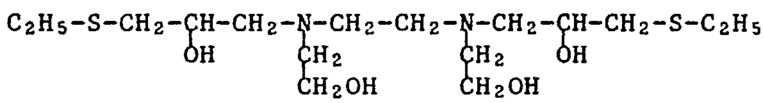

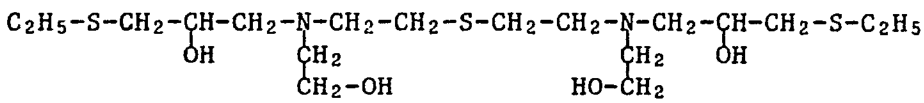

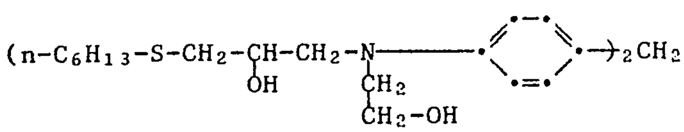

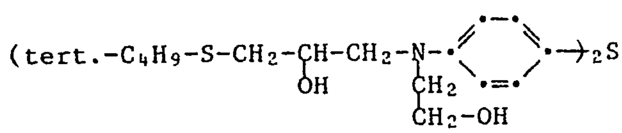

Die Verbindungen der Formel I bzw. Ia können vorteilhaft in an sich bekannter Weise durch Umsetzung eines Glycidylthioethers der Formel II

(II)

mit einem Amin der Formel III

$$NH\langle^{R^4}_{R^5} \qquad (III)$$

erhalten werden, wobei R, $R^4$ und $R^5$ die vorstehend definierten Bedeutungen haben.

Für die Umsetzung kann ein Lösungsmittel verwendet werden. Vorteilhaft ist es jedoch, auf ein Lösungsmittel zu verzichten, denn die fast ausnahmslos flüssigen Glycidylthioether wirken bei festen Aminoverbindungen selbst als Lösungsmittel.

Die Reaktionstemperaturen liegen zweckmässig zwischen Raumtemperatur und maximal 150°C, je nach der Basizität der Aminoverbindungen.

Die Verbindungen der Formel I können aber auch durch Umsetzung eines Mercaptans der Formel IV

R-SH (IV)

mit einer N-Glycidylverbindung der Formel V

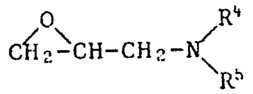

(V)

erhalten werden.

Eine weitere Möglichkeit, die Verbindungen der Formel I herzustellen, besteht in der Umsetzung eines Amins der Formel III oder eines Mercaptans der Formel IV mit Chlorhydrinderivaten der Formeln VI oder VII, wie sie bei der Addition von Epichlorhydrin an HS- oder HN-Verbindungen (auch in situ) erhalten werden (können).

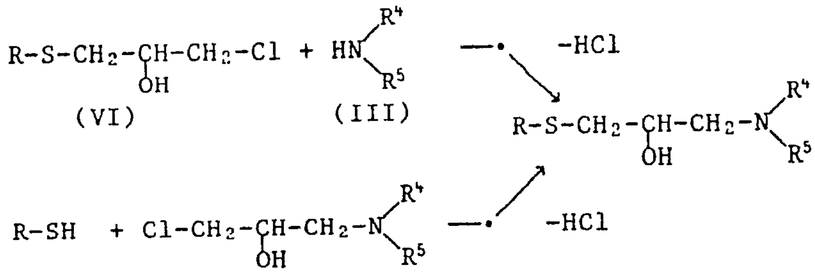


(VI) (III) (IV) (VII)

In diesem Falle ist ein Chlorwasserstoff-Akzeptor in Form einer Base erforderlich.

Die $NH_2$-Gruppe reagiert im allgemeinen bifunktionell, ausgenommen bei sterischer Hinderung, wie z.B. bei einer an ein tertiäres C-Atom gebundenen $NH_2$-Gruppe, die nur monofunktionell reagiert. Für die monofunktionelle Umsetzung einer normalen $NH_2$-Gruppe ist ein gewisser Überschuss an der Amino-Verbindung erforderlich, der später mittels Destillation oder auch aufgrund unterschiedlicher Lösungseigenschaften abgetrennt werden kann.

Bevorzugte Amine der Formel III sind beispielsweise die folgenden:

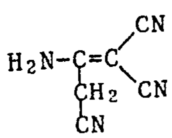

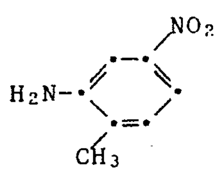

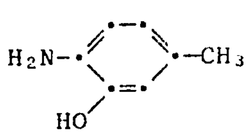

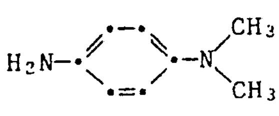

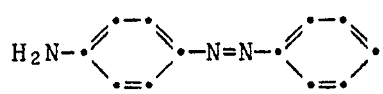

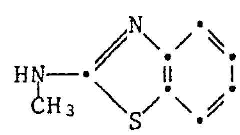

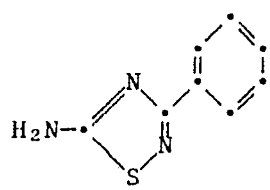

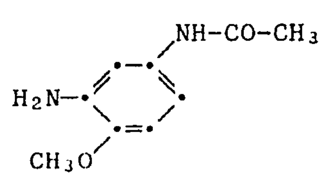

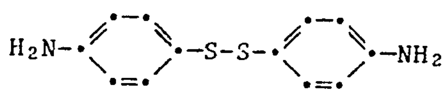

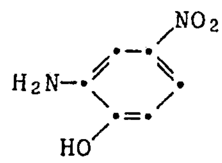

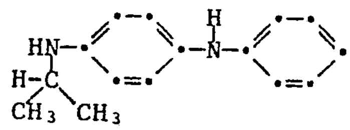

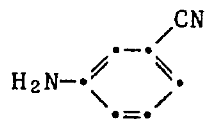

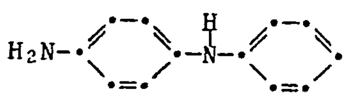

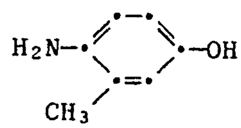

$H_2N-CH_2-CH_2-S-CH_2-CH_2-NH_2$

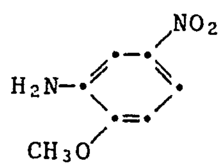

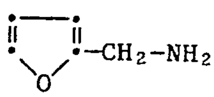

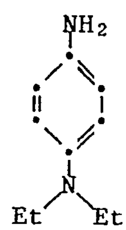

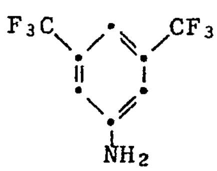

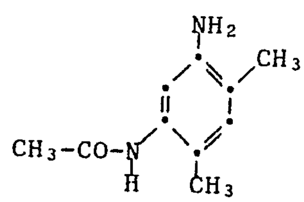

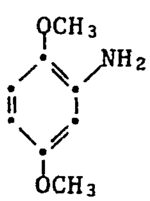

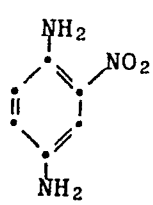

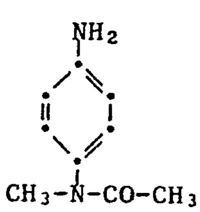

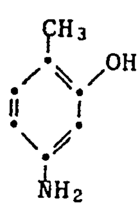

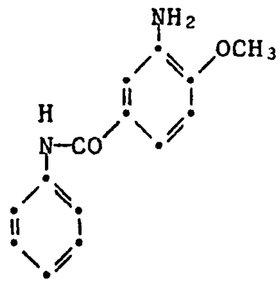

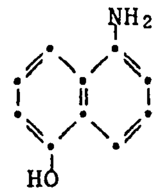

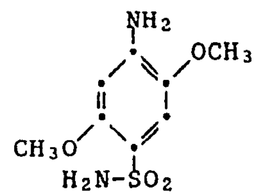

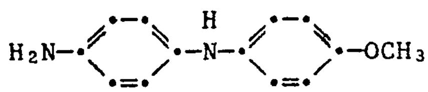

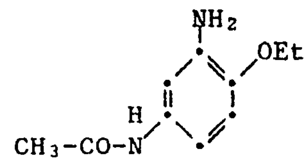

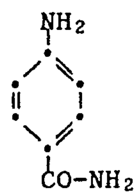

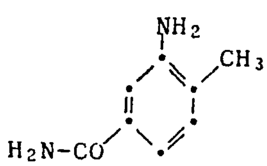

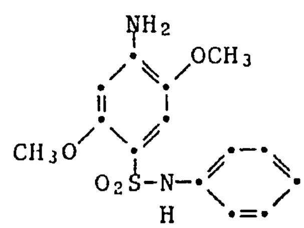

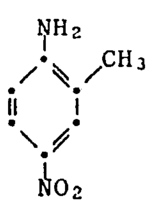

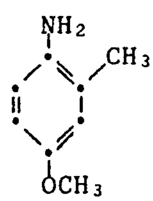

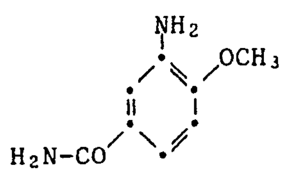

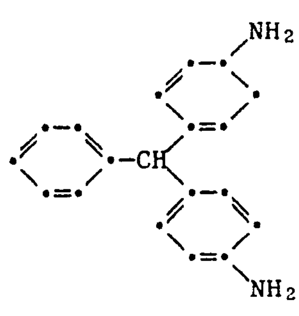

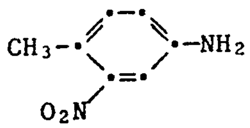

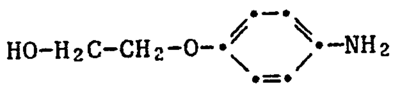

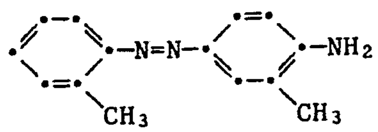

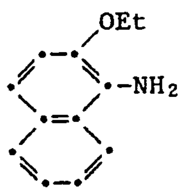

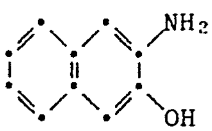

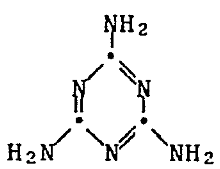

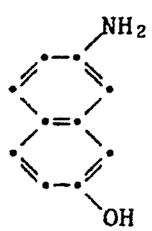

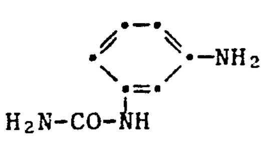

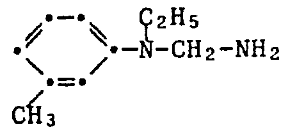

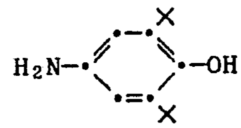

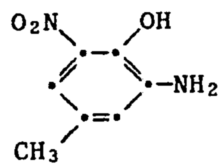

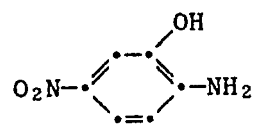

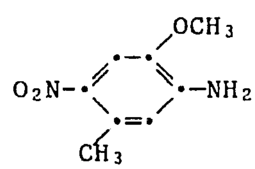

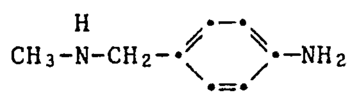

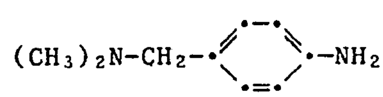

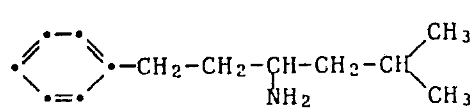

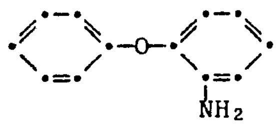

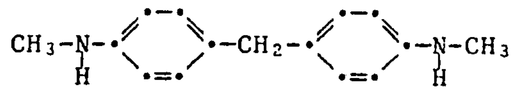

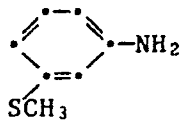

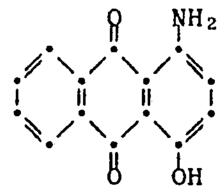

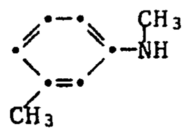

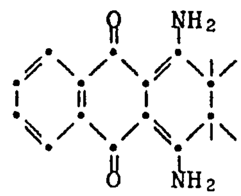

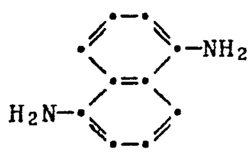

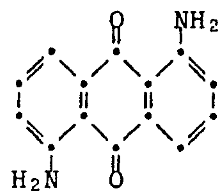

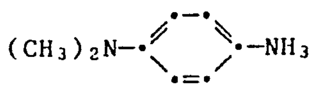

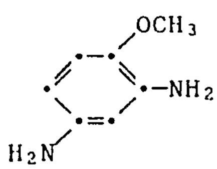

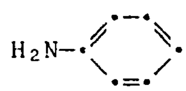

,

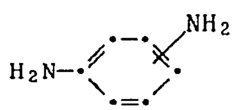

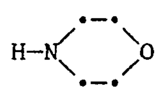

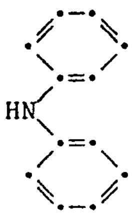

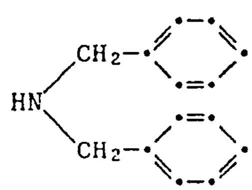

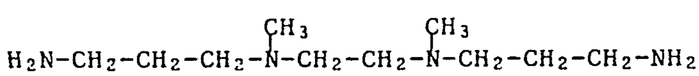

Ein weiterer Gegenstand der Erfindung ist die Verwendung von Verbindungen der Formeln I bzw. Ia als Hochdruck- und verschleissmindernde Additive in Schmiermitteln, Hydraulikflüssigkeiten oder Metallbearbeitungsflüssigkeiten.

Die Verbindungen der Formel I bzw. Ia sind im allgemeinen von flüssiger Beschaffenheit, jedoch von unterschiedlicher Viskosität. Sie sind in Schmiermitteln, Hydraulikflüssigkeiten und Metallbearbeitungsflüssigkeiten in ausreichender Menge löslich. Bei den hochviskosen Vertretern bietet die Verdünnung mit z.B. einem Paraffinöl oder auch einem entsprechenden Grundöl eine günstige Konfektionsform.

Die erfindungsgemässen Verbindungen sind als Zusätze zu Schmiermitteln, Hydraulikflüssigkeiten und Metallbearbeitungsflüssigkeiten, insbesondere zu Schmiermitteln und Hydraulikflüssigkeiten und ganz speziell zu Schmiermitteln sehr gut geeignet und führen zu einer Verbesserung der Gebrauchseigenschaften wie z.B. der Hochdruck- und Antiverschleiss-Eigenschaften.

Die Verbindungen der Formel I werden den Schmiermitteln, Hydraulikflüssigkeiten und Metallbearbeitungsflüssigkeiten vorteilhaft in einer Menge von 0,01 bis 10 Gew.-%, vorzugsweise in einer Menge von 0,05 bis 5 Gew.-%, bezogen auf das Schmiermittel, die Hydraulikflüssigkeit oder die Metallbearbeitungsflüssigkeit, zugesetzt.

Solche Schmier-, Hydraulik- und Metallbearbeitungssysteme können von polarer oder unpolarer Natur sein. Die Auswahlkriterien ergeben sich aus den Löslichkeitseigenschaften der entsprechenden Verbindungen.

Die in Frage kommenden Schmiermittel, Hydraulikflüssigkeiten bzw. Metallbearbeitungsflüssigkeiten sind dem Fachmann geläufig und z.B. im «Schmiermittel Taschenbuch» (Hüthig Verlag, Heidelberg, 1974) resp. in «Ullmanns Encyclopädie der technischen Chemie», Bd. 13, Seiten 85-94 (Verlag Chemie, Weinheim, 1977) bzw. in D. Klamann «Schmierstoffe und verwandte Produkte», Seiten 158-174 (Verlag Chemie, Weinheim, 1982) beschrieben.

Besonders geeignet sind neben Mineralölen z.B. Poly-α-Olefine, Schmiermittel auf Esterbasis, Phosphate, Glykole, Polyglykole und Polyalkylenglykole, sowie deren Mischungen mit Wasser, und Wasser selbst.

Die Schmiermittel, Hydraulikflüssigkeiten oder Metallbearbeitungsflüssigkeiten können zusätzlich andere Additive enthalten, die zugegeben werden, um die Grundeigenschaften von diesen Substanzen noch weiter zu verbessern; dazu gehören: Antioxidantien, Metallpassivatore, Rostinhibitoren, Viskositätsindex-Verbesserer, Stockpunkterniedriger, Dispergiermittel, Detergentien, sowie andere Hochdruck-Zusätze und Antiverschleiss-Additive.

*Beispiele für phenolische Antioxidantien*

*1. Alkylierte Monophenole*

2,6-Di-tert-butyl-4-methylphenol
2,6-Di-tert-butylphenol
2-tert-butyl-4,6-Dimethylphenol
2,6-Di-tert-butyl-4-ethylphenol
2,6-Di-tert-butyl-4-ethylphenol
2,6-Di-tert-butyl-4-n-butylphenol
2,6-Di-tert-butyl-4-iso-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert-butyl-4-methoxymethylphenyl
o-tert-Butylphenol

*2. Alkylierte Hydrochinone*

2,6-Di-tert-butyl-4-methoxyphenol
2,5-Di-tert-butyl-hydrochinon
2,5-Di-tert-amyl-hydrochinon
2,6-Diphenyl-4-octadecyloxyphenol

*3. Hydroxylierte Thiodiphenylether*

2,2'-Thio-bis-(6-tert-butyl-4-methylphenol)
2,2'-Thio-bis-(4-octylphenol)
4,4'-Thio-bis-(6-tert-butyl-3-methylphenol)
4,4'-Thio-bis-(6-tert-butyl-2-methylphenol)

*4. Alkyliden-Bisphenole*

2,2'-Methylen-bis-(6-tert-butyl-4-methylphenol)
2,2'-Methylen-bis-(6-tert-butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert-butylphenol)
2,2'-Ethyliden-bis-(4,6-di-tert-butylphenol)
2,2'-Ethyliden-bis-(6-tert-butyl-4-iso-butylphenol)
2,2'-Methylen-bis-[6-(α-methylbenzyl)-4-nonylphenol)
2,2'-Methylen-bis-[6-(α,α-dimethylbenzyl)-4--nonylphenol]
4,4'-Methylen-bis-(2,6-di-tert-butylphenol)
4,4'-Methylen-bis-(6-tert-butyl-methylphenol)
1,1-Bis(5-tert-butyl-4-hydroxy-2-methylphenyl)--butan
2,6-Di-(3-tert-butyl-5-methyl-2-hydroxybenzyl)--4-methylphenol
1,1,3-Tris-(5-tert-butyl-4-hydroxy-2-methylphenyl)-3-n-dodecyl-mercaptobutan
Ethylenglycol-bis-[3,3-bis-(3'-tert-butyl-4'--hydroxyphenyl)-butyrat]
Di-(3-tert-butyl-4-hydroxy-5-methylphenyl)-dicyclopentadien
Di-[2-(3'-tert-butyl-2'-hydroxy-5'-methyl-benzyl)--6-tert-butyl-4-methyl-phenyl]-terephthalat

*5. Benzylverbindungen*

1,3,5-Tri-(3,5-di-tert-butyl-4-hydroxybenzyl)--2,4,6-trimethylbenzol
Di-(3,5-di-tert-butyl-4-hydroxybenzyl)-sulfid
3,5-Di-tert-butyl-4-hydroxybenzyl-mercaptoessigsäure-isooctylester
Bis-(4-tert-butyl-3-hydroxy-2,6-dimethylbenzyl)--dithiol-terephthalat
1,3,5-Tris-(3,5-di-tert-butyl-4-hydroxybenzyl)--isocyanurat
1,3,5-Tris-(4-tert-butyl-3-hydroxy-2,6-dimethylbenzyl)-isocyanurat

3,5-Di-tert-butyl-4-hydroxybenzyl-phosphonsäure-dioctadecylester
3,5-Di-tert-butyl-4-hydroxybenzyl-phosphonsäure-monoethylester
Calcium-salz

*6. Acylaminophenole*

4-Hydroxy-laurinsäureanilid
4-Hydroxy-stearinsäureanilid
2,4-Bis-octylmercapto-6-(3,5-di-tert-butyl-4-hydroxyanilino)-s-triazin
N-(3,5-di-tert-butyl-4-hydroxyphenyl)-carbaminsäureoctylester

*7. Ester der β-(3,5-Di-tert-butyl-4-hydroxyphenyl)-propionsäure*

mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

*8. Ester der β-(5-tert-butyl-4-hydroxy-3-methylphenyl)-propionsäure*

mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

*9. Amide der β-(3,5-Di-tert-butyl-4-hydroxyphenyl)-propionsäure,*

wie z.B:.

N,N'-Di-(3,5-di-tert-butyl-4-hydroxyphenylpropionyl)-hexamethylendiamin
N,N'-Di-(3,5-di-tert-butyl-4-hydroxyphenylpropionyl)-trimethylendiamin
N,N'-Di-(3,5-di-tert-butyl-4-hydroxyphenylpropionyl)-hydrazin

*Beispiele für aminische Antioxidantien:*

N,N'-Di-isopropyl-p-phenylendiamin
N,N'-Di-sec-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Diphenyl-p-phenylendiamin
N,N'-Di-(naphthyl-2)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert-butyl-4-dimethylamino-methyl-phenol
2,4'-Diamino-diphenylmethan
4,4'-Diamino-diphenylmethan
N,N,N',N'-Tetramethyl-4,4'-diamino-diphenylmethan
1,2-Di-[(2-methyl-phenyl)-amino]-ethan
1,2-Di-(phenylamino)-propan
(o-Tolyl)-biguanid
Di-[4-(1',3'-dimethyl-butyl)-phenyl]amin
tert-octyliertes N-Phenyl-1-naphthylamino
Gemisch aus mono- und dialkylierten tert-Butyl-/tert-Octylphenylaminen.

*Beispiele für Metallpassivatoren sind:*

für Kupfer, z.B.:

Triazol, Benztriazol und deren Derivate, 2-Mercaptobenzthiazol, 2,5-Dimercaptothiadiazol, Salicylidenpropylendiamin, Salze von Salicylaminoguanidin.

*Beispiele für Rost-Inhibitoren sind:*

a) Organische Säuren, ihre Ester, Metallsalze und Anhydride, z.B.:

N-Oleoyl-sarcosin, Sorbitan-mono-oleat, Blei-naphthenat, Dodecenylbernsteinäure-anhydrid, Alkenylbernsteinsäure-Halbester, 4-Nonylphenoxy-essigsäure.

b) Stickstoffhaltige Verbindungen, z.B.:

I. Primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen, z.B.:

Substituierte Imidazole und Oxazoline.

c) Phosphorhaltige Verbindungen, z.B.:

Aminsalze von Phosphorsäurepartialestern.

d) Schwefelhaltige Verbindungen, z.B.:

Barium-dinonylnaphthalin-sulfonate, Calciumpetroleum-sulfonate.

*Beispiele für Viskositätsindex-Verbesserer sind z.B.:*

Polymethacrylate, Vinylpyrrolidon/Methacrylat-Copolymere, Polybutene, Olefin-Copolymere, Styrol/Acrylat-Copolymere.

*Beispiele für Stockpunkterniedriger sind z.B.:*

Polymethacrylat, alkylierte Naphthalinderivate.

*Beispiele für Dispergiermittel/Tenside sind z.B.:*

Polybutenylbernsteinsäure-imide, Polybutenylphosphonsäurederivate, basische Magnesium-, Calcium-, und Bariumsulfonate und -phenolate.

*Beispiele für Verschleissschutz-Additive sind z.B.:*

Schwefel und/oder Phosphor und/oder Halogen enthaltende Verbindungen, wie geschwefelte pflanzliche Öle, Zinkdialkyldithio-phosphate, Tritolyl-phosphat, chlorierte Paraffine, Alkyl- und Aryldisulfide.

*Beispiel 1*

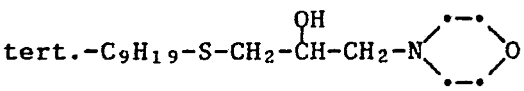

17,4 g Morpholin werden in einem Kolben vorgelegt, auf 90°C erwärmt und unter Rühren 47,6 g tert-Nonylglycidylthioether eingetropft. Nach beendeter Zugabe wird das Gemisch noch 0,5 Stunden bei 90°C nachgerührt. Die Ausbeute beträgt 100% d.Th. einer gelben, schwach viskosen Flüssigkeit mit einem Brechungsindex von $n_D^{20}$ : 1.4914.

*Beispiel 2*

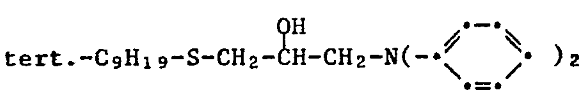

Ein Gemisch aus 15,2 g tert-Nonylmercaptan und katalytischen Mengen Natriumhydrid wird auf 100°C erwärmt und bei gleicher Temperatur werden unter Rühren 22,5 g N-Glycidyldiphenylamin (CA 64, 3385 d) eingetropft. Zur Vervollständigung der Reaktion wird noch 1,5 Stunden bei 100°C nachgerührt.

Ausbeute: 37,7 g einer gelben viskosen Flüssigkeit mit einem Brechungsindex von $n_D^{20}$ : 1.5699.

*Beispiel 3*

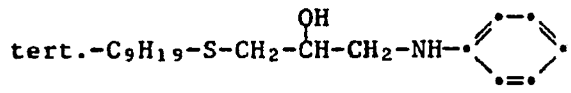

Eine Mischung aus 43,3 g tert-Nonylglycidylthioether und 46,5 g Anilin wird 8 Stunden unter Rühren bei 100°C erwärmt. Das überschüssige Anilin wird anschliessend im Wasserstrahlvakuum abdestilliert und der Rückstand im Ölpumpenvakuum fraktioniert. Man erhält 51,1 g ≙ 82,6% d.Th. einer Flüssigkeit mit einem Siedepunkt von 169°C bei 0,08 mbar und einem Brechungsindex von $n_D^{20}$ : 1.5419.

*Beispiel 4*

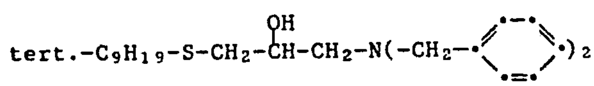

35,7 g tert-Nonylglycidylthioether und 14,8 g Dibenzylamin werden 15 Stunden auf 100°C erwärmt. Nach beendeter Reaktion beträgt die Ausbeute 100% d.Th. einer gelben Flüssigkeit mit einem Brechungsindex von $n_D^{20}$ : 1.5169.

In analoger Weise zu den Beispielen 1-4 werden weitere Verbindungen hergestellt, die in der folgenden Tabelle 1 zusammengestellt sind.

TABELLE 1

| Beispiel Nr. | Formel | Bemerkungen | Physikalische Daten | |
|---|---|---|---|---|
| | | | Siedepunkt (°C) | $n_D^{20}$ |
| 5 | 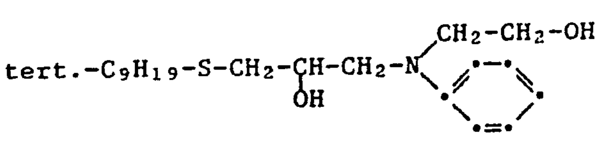 | gelbe viskose Flüssigkeit | | 1,5416 |
| 6 | 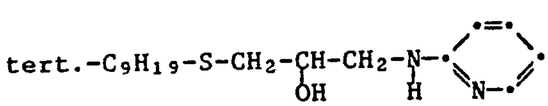 | | | 1,5445 |
| 7 | 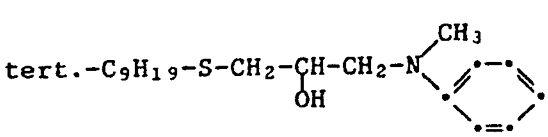 | | | 1,5383 |

TABELLE 1 (Fortsetzung)

| Beispiel Nr. | Formel | Bemerkungen | Physikalische Daten: Siedepunkt (°C) | Physikalische Daten: $n_D^{20}$ |
|---|---|---|---|---|
| 8 | 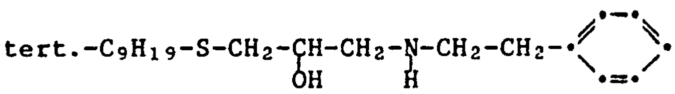 | farblose Flüssigkeit | 186-88/ 0,09 mbar | 1,5245 |
| 9 | 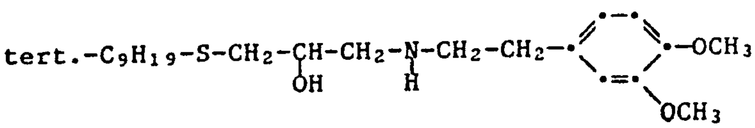 | viskose Flüssigkeit | 200-05/ 0,001 mbar | 1,5308 |
| 10 | 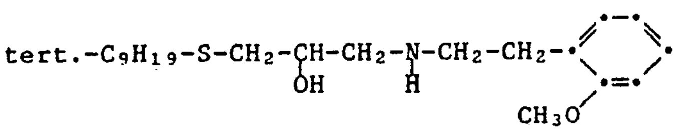 | gelbliche viskose Flüssigkeit | 176-80/ 0,001 mbar | 1,5278 |
| 11 | 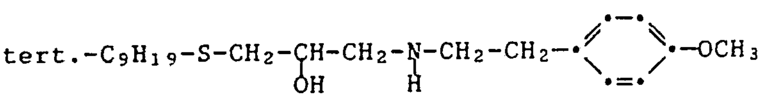 | | 185-90/ 0,001 mbar | 1,5269 |
| 12 | 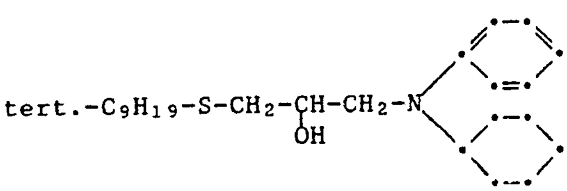 | | | 1,5326 |
| 13 | 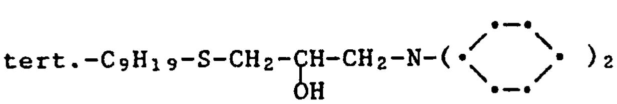 | | | 1,4956 |
| 14 | 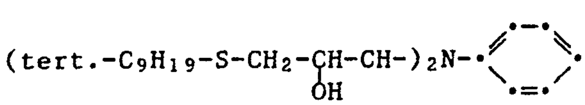 | | | 1,5246 |
| 15 | 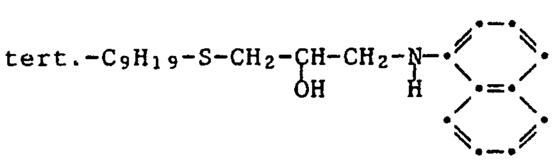 | | 225-30/ 0,001 mbar | 1,5874 |

TABELLE 1 (Fortsetzung)

| Beispiel Nr. | Formel | Bemerkungen | Physikalische Daten: Siedepunkt (°C) | Physikalische Daten: $n_D^{20}$ |
|---|---|---|---|---|
| 16 | 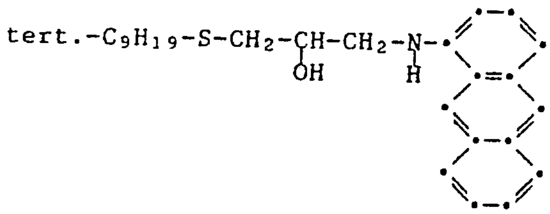 | NMR-Spektrum | | |
| 17 | tert.-$C_9H_{19}$-S-$CH_2$-$\underset{OH}{CH}$-$CH_2$-$\underset{H}{N}$-$\overset{CH_3}{\underset{CH_3}{C}}$-C≡CH | | 125-30/ 0,05 mbar | 1,4900 |
| 18 | (tert.-$C_9H_{19}$-S-$CH_2$-$\underset{OH}{CH}$-$CH_2$-)$_2$NH | | | 1,5074 |
| 19 | (tert.-$C_9H_{19}$-S-$CH_2$-$\underset{OH}{CH}$-$CH_2$-)$_3$N | | | 1,5046 |
| 20 | [(tert.-$C_9H_{19}$-S-$CH_2$-$\underset{OH}{CH}$-$CH_2$-)$_2$N-$CH_2$-]$_2$ | | | 1,5075 |
| 21 | tert.-$C_{12}H_{25}$-S-$CH_2$-$\underset{OH}{CH}$-$CH_2$-$\underset{H}{N}$-(i-$C_{13}H_{27}$) | | | 1,4645 |
| 22 | tert.-$C_9H_{19}$-S-$CH_2$-$\underset{OH}{CH}$-$CH_2$-$\underset{H}{N}$-$\overset{C_2H_5}{\underset{C_2H_5}{C}}$-C≡CH | | | 1,4888 |
| 23 | 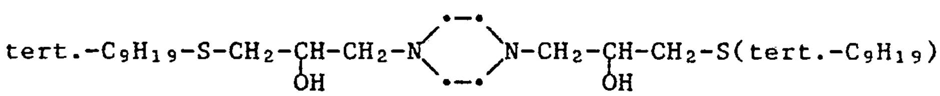 | | | 1,5059 |
| 24 | 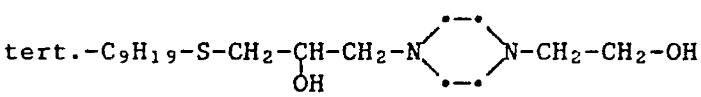 | | | 1,5102 |
| 25 | (tert.-$C_9H_{19}$-S-$CH_2$-$\underset{OH}{CH}$-$CH_2$-)$_2$N-($CH_2$-)$_3$N-($CH_2$-$CH_2$-OH)$_2$ | | | 1,5073 |
| 26 | 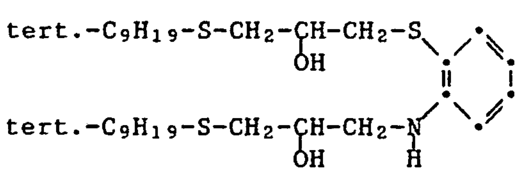 | | | 1,5415 |

TABELLE 1 (Fortsetzung)

| Beispiel Nr. | Formel | Bemerkungen | Physikalische Daten Siedepunkt (°C) | $n_D^{20}$ |
|---|---|---|---|---|
| 27 | 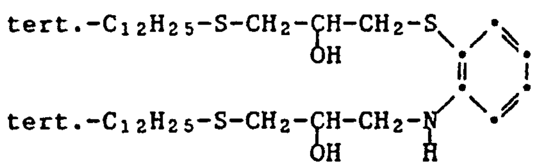 | | | 1,5229 |
| 28 | 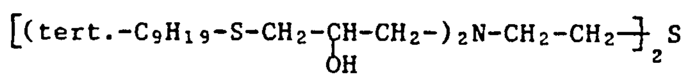 | | | 1,5115 |
| 29 | 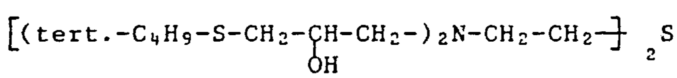 | | | 1,5218 |
| 30 | 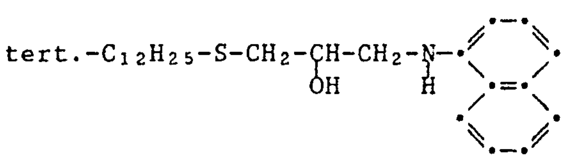 | | | 1,5472 |
| 31 | 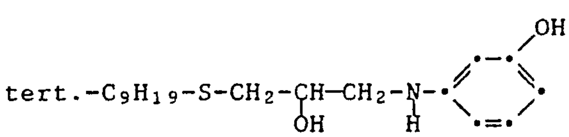 | | | $n_D^{40}$: 1,5407 |
| 32 | 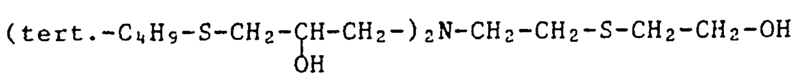 | | | $n_D^{40}$: 1,5257 |
| 33 | 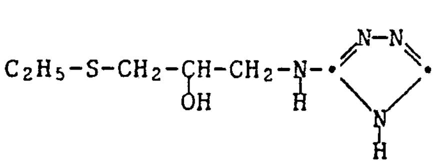 | | 211/0,2 mbar | $n_D^{40}$: 1,5510 |

*Beispiel 31*

Mit dem Shell-Vierkugel-Apparat (IP 239/73 Extreme pressure and wear lubricant test for oils and greases — four ball machine) werden folgende Werte bestimmt:

1. W.L. = Weld load [Schweisslast in Newton (N)]. Das ist die Last, bei der die Kugeln innerhalb von 10 Sekunden zusammenschweissen.
2. W.S.D. = Wear Scar Diameter in mm: Das ist der mittlere Verschleissdurchmesser bei einer Belastung von 400 N während 1 h.

Als Testflüssigkeit für die Wirksamkeit der Additive wird ein Basisöl der Viskosität ISO-VH 100 mit niedrigem Aromatengehalt und 0,035% S verwendet.

| Additiv Beispiel Nr. | W.L. (N) | | W.S.D. 1 h (mm) | |
|---|---|---|---|---|
| | 1% Additiv | 2,5 % Additiv | 0,25% Additiv | 1,0% Additiv |
| 3 | 1850 | 2050 | 0,45 | 0,50 |
| 15 | 1850 | | 0,50 | 0,45 |
| 16 | 1700 | | 0,52 | 0,47 |
| 27 | 2000 | | | 0,50 |

Das Basisöl liefert unter diesen Testbedingungen eine Weld load von 1450 N und einen Wear Scar Diameter von 0,95 mm.

## Patentansprüche

1. Zusammensetzung enthaltend ein Schmiermittel, eine Hydraulikflüssigkeit oder eine Metallbearbeitungsflüssigkeit und wenigstens eine Verbindung der Formel I

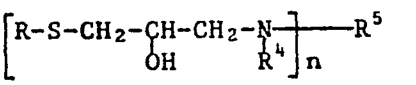 (I)

worin n gleich 1 bis 6 ist und R ein Radikal der Formel

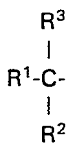

sein kann, wobei $R^1$, $R^2$ und $R^3$ unabhängig voneinander $C_1$-$C_{21}$-Alkyl sind und zusammen nicht mehr als 22 C-Atome besitzen und $R^2$ und $R^3$ ausserdem Wasserstoff sein können, oder worin R $C_5$,$C_{12}$-Cycloalkyl, $C_7$-$C_9$-Aralkyl, unsubstituiertes oder durch $C_1$-$C_4$-Alkyl substituiertes Phenyl oder Naphthyl, Furyl, Furfuryl, Thienyl, Morpholinyl, Imidazolyl, Thiazolyl, Oxazolyl, Imidazolinyl, Thiazolinyl, Oxazolinyl, Benzimidazolinyl, Benzthiazolinyl oder Benzoxazolinyl ist, und worin $R^4$ Wasserstoff, unsubstituiertes oder durch -OH, $-OCH_3$, -CN oder $-N(R^6)_2$ substituiertes $C_1$-$C_{20}$-Alkyl ist, und $R^6$ $C_1$-$C_4$-Alkyl bedeutet, das gegebenenfalls durch -OH substituiert ist, und $R^4$ als $C_1$-$C_{20}$-Alkyl gegebenenfalls durch -O-, -S- oder -N$<$ unterbrochen ist, oder $R^4$ $C_4$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl, unsubstituiertes oder durch ein oder zwei $C_1$-$C_4$-Alkyl oder $-CF_3$, eine oder zwei OH-Gruppen oder durch ein oder zwei $-N(R^7)(R^8)$ substituiertes $C_6$, $C_{10}$- oder $C_{14}$-Aryl ist, und $R^7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_{10}$-Hetaryl, ein nichtaromatischer $C_2$-$C_5$-Heterocyclus, unsubstituiertes oder durch -OH, $C_1$-$C_4$-Alkoxy oder $-N(R^6)_2$ substituiertes $C_7$-$C_{14}$-Aralkyl ist oder $R^4$ $-CH_2$-CH(OH)-$CH_2$-S-R ist, und $R^5$ unsubstituiertes oder durch $-OCH_3$, -CN oder $-N(R^6)_2$ substituiertes $C_4$-$C_{20}$-Alkyl mit $R^6$ in der vorstehenden Bedeutung ist, das gegebenenfalls durch -O-, -S- oder -N$<$ unterbrochen sein kann, oder $R^5$ durch $N(R^6)$(Tolyl) oder $C_1$-$C_{10}$-Hetaryl substituiertes $C_1$-$C_{20}$-Alkyl ist oder $R^5$ unsubstituiertes $C_4$-$C_{20}$-Alkenyl oder durch ein oder mehrere -CN substituiertes $C_3$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl oder unsubstituiertes oder durch eine oder mehrere $C_1$-$C_4$-Alkylgruppen, die gegebenenfalls durch -NH- oder $-N(R^6)$- unterbrochen sind, eine oder mehrere $C_1$-$C_4$-Alkoxy-, $C_1$-$C_4$-Alkylthio- und/oder OH-Gruppen, ein oder mehrere $-NO_2$, $-CF_3$ und/oder -CN, Hydroxyethoxy, Phenoxy, Ureido, Carbamoyl, Sulfamoyl, Benzolazo, Toluolazo, Anilinocarbonyl, Anilinosulfonyl, $-S-CH_2-CH(OH)-CH_2-S-R$ und/oder durch ein oder zwei $-N(R^7)(R^8)$ substituiertes $C_6$-, $C_{10}$- oder $C_{14}$-Aryl ist, mit $R^7$ und $R^8$ in der vorstehenden Bedeutung wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl oder $C_1$-$C_4$-Alkyl substituiertes $C_1$-$C_{10}$-Hetaryl, unsubstituiertes oder durch -OH, eine oder mehrere $C_1$-$C_4$-Alkoxygruppen oder durch $-N(R^6)_2$ substituiertes $C_7$-$C_{14}$-Aralkyl ist, mit $R^6$ in der vorstehenden Bedeutung, oder $R^5$ $-CH_2$-$-CH(OH)CH_2$-S-R ist oder $R_5$ einen 2wertigen $C_2$-$C_{12}$-aliphatischen Rest bedeutet, der sich von einem 2fach durch $-NH_2$ substituierten $C_2$-$C_{12}$-Alkan herleitet und unsubstituiert oder durch -OH, $-OCH_3$ oder $-N(R^6)_2$ substituiert sein kann und gegebenenfalls durch -O-, -S- oder $-N(R^9)$- unterbrochen ist, wobei $R^9$ unsubstituiertes oder durch -OH substituiertes $C_1$-$C_4$-Alkyl oder $-CH_2-CH(OH)-CH_2-S-R$ ist, oder $R^5$ einen 2- bis 4wertigen $C_6$-$C_{12}$-cycloaliphatischen Rest bedeutet, der sich von einem 2fach bis 4fach durch $-NH_2$ substituierten $C_6$-$C_{12}$-Cycloalkan herleitet und unsubstituiert oder durch $C_1$-$C_4$-Alkyl substituiert sein kann, oder $R^5$ einen Rest der Formel

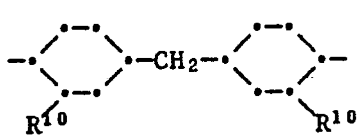

darstellt, worin $R^{10}$ Wasserstoff oder $C_1$-$C_4$-Alkyl ist, oder $R^5$ einen Rest der Formel

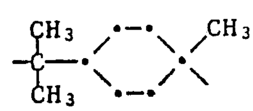

oder einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $-NO_2$ oder $C_1$-$C_4$-Alkyl substituiert sein kann, oder $R^5$ Anthrachinonylen, 2,3-Dihydroanthrachinonylen oder einen Rest der Formel

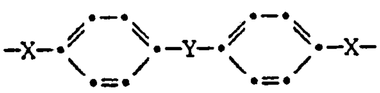

darstellt, worin X $-CH_2$- oder eine direkte Bindung von Y $-CH_2$-, $-C(C_6H_5)H$-, -S-S-, -NH- oder eine direkte Bindung ist, oder $R^5$ einen 2- oder 3wertigen $C_2$-$C_{10}$-heteroaromatischen Rest bedeutet, der sich von einem 2fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^5$ einen 2wertigen $C_7$-$C_{14}$-araliphatischen Rest bedeutet, der sich von einem 2fach durch $-NH_2$ substituierten $C_7$-$C_{14}$-Aralkan herleitet, oder $R^4$ und $R^5$ zusammen mit dem N-Atom, an das sie gebunden sind, einen $C_1$-$C_7$-azacyclischen Ring bilden, der aromatisch oder nicht aromatisch sein kann und gegebenenfalls ein oder mehrere N-, O- oder S-Atome enthalten kann, wobei das N-Atom gegebenenfalls durch $C_1$-$C_4$-Alkyl, welches seinerseits durch -OH substituiert sein kann, substituiert ist und der $C_1$-$C_7$-azacyclische Ring an einem C-Atom gegebenenfalls durch $C_1$-$C_4$-Alkyl, =O oder =S substituiert sein kann, oder $R^4$ und $R^5$ zusammen mit dem N-Atom, an das sie gebunden sind, 2,2,4-Trimethyl-1,2-dihydrochinolyl, einen Teil eines $C_1$-$C_7$-azacyclischen 2- bis 6wertigen Rings oder ein Radikal der Formeln

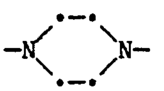 ,

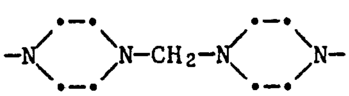

oder

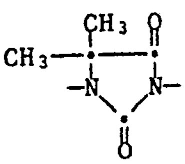

bedeuten.

2. Zusammensetzung gemäss Anspruch 1, worin in Formel I n gleich 1 ist.

3. Zusammensetzung gemäss Anspruch 1, worin in Formel I n gleich 2 bis 6 ist.

4. Zusammensetzung gemäss Anspruch 3, worin in Formel I n gleich 2 ist.

5. Zusammensetzung gemäss Anspruch 1, worin in Formel I R ein Radikal der Formel

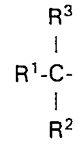

ist, wobei $R^1$, $R^2$ und $R^3$ unabhängig voneinander $C_1$-$C_{18}$-Alkyl sind und zusammen nicht mehr als 22 C-Atome besitzen und $R^2$ und $R^3$ ausserdem Wasserstoff sein können, oder worin R $C_5$-$C_6$-Cycloalkyl, Benzyl, unsubstituiertes oder durch $C_1$-$C_4$-Alkyl substituiertes Phenyl oder unsubstituiertes Naphthyl ist.

6. Zusammensetzung gemäss Anspruch 5, worin in Formel I R ein Radikal der Formel

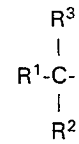

ist, wobei $R^1$, $R^2$ und $R^3$ zusammen mit dem C-Atom, an das sie gebunden sind, $C_4$-$C_{16}$-Alkyl bilden, wobei keiner dieser Substituenten $R^1$, $R^2$ und $R^3$ Wasserstoff sein darf, oder worin R Cyclohexyl, Benzyl, unsubstituiertes oder durch $C_1$-$C_4$-Alkyl substituiertes Phenyl oder unsubstituiertes Naphthyl ist.

7. Zusammensetzung gemäss Anspruch 1, worin in Formel I R ein Radikal der Formel

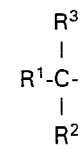

ist, wobei $R^1$ $C_1$-$C_{12}$-Alkyl ist und $R^2$ und $R^3$ Wasserstoff sind.

8. Zusammensetzung gemäss Anspruch 1, worin in Formel I R ein Radikal der Formel

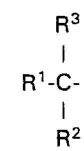

ist, wobei $R^1$ und $R^2$ unabhängig voneinander $C_1$-$C_{12}$-Alkyl sind und zusammen mit dem C-Atom, an das sie gebunden sind, $C_3$-$C_{14}$-Alkyl bilden und $R^3$ Wasserstoff ist.

9. Zusammensetzung gemäss Anspruch 1, worin in Formel I $R^4$ Wasserstoff, unsubstituiertes oder durch ein oder zwei $C_1$-$C_4$-Alkyl oder $-CF_3$, eine oder zwei OH-Gruppen oder durch ein oder zwei $-N(R^7)-(R^8)$ substituiertes $C_6$-, $C_{10}$- oder $C_{14}$-Aryl ist und $R^7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_{10}$-Hetaryl bedeutet, und $R^5$ unsubstituiertes oder durch eine oder mehrere $C_1$-$C_4$-Alkylgruppen, die gegebenenfalls durch -NH- oder $-N(R^6)$- unterbrochen sind, eine oder meh-

rere $C_1$-$C_4$-Alkoxy-$C_1$-$C_4$-Alkylthio- und/oder OH-Gruppen, ein oder mehrere -$NO_2$, -$CF_3$ und/oder -CN, Hydroxyethoxy, Phenoxy, Ureido, Carbamoyl, Sulfamoyl, Benzolazo, Toluolazo, Anilinocarbonyl, Anilinosulfonyl, -S-$CH_2CH(OH)$-$CH_2$-S-R und/oder durch ein oder zwei -$N(R^7)(R^8)$ substituiertes $C_6$-, $C_{10}$- oder $C_{14}$-Aryl list, mit $R^7$ und $R^8$ in der vorstehenden Bedeutung, wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl und/oder $C_1$-$C_4$-Alkyl substituiertes $C_1$-$C_{10}$-Hetaryl ist, oder $R^5$ einen 2- oder 3wertigen $C_6$-, $C_{10}$- oder $C_{14}$-aromatischen Rest bedeutet, der sich von einem 2fach oder 3fach durch -$NH_2$ substituierten $C_6$-, $C_{10}$- oder $C_{14}$-Aromaten herleitet und unsubstituiert oder durch -OH, $NO_2$ oder $C_1$-$C_4$-Alkyl substituiert sein kann, oder $R^5$ Anthrachinonylen, 2,3-Dihydroanthrachinonylen oder einen Rest der Formel

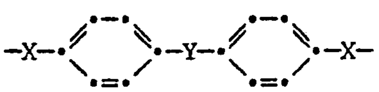

darstellt, worin X eine direkte Bindung und Y -$CH_2$-, -$C(C_6H_5)H$-S-S-, -NH- oder eine direkte Bindung ist, oder $R^5$ einen 2- oder 3wertigen $C_2$-$C_{10}$-heteroaromatischen Rest bedeutet, der sich von einem 2fach oder 3fach durch -$NH_2$ substituierten $C_2$-$C_{10}$-Heteroaromaten herleitet und unsubstituiert oder durch -OH oder $C_6$- oder $C_{10}$-Aryl substituiert ist, oder $R^4$ und $R^5$ zusammen mit dem N-Atom, an das sie gebunden sind, einen $C_1$-$C_7$-azacyclischen Ring bilden, der aromatisch ist und gegebenenfalls ein oder mehrere N-, O- oder S-Atome enthalten kann, oder $R^4$ und $R^5$ zusammen mit dem N-Atom, an das sie gebunden sind, einen 2,2,4-Trimethyl-1,2-dihydrochinolylrest bedeuten.

10. Verbindungen der Formel Ia

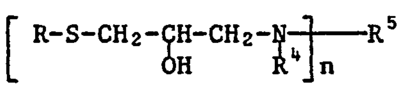 (Ia)

worin n gleich 1 bis 6 ist und R ein Radikal der Formel

$$\begin{array}{c} R^3 \\ | \\ R^1\text{-}C\text{-} \\ | \\ R^2 \end{array}$$

bedeutet, wobei $R^1$, $R^2$ und $R^3$ zusammen mit dem C-Atom, an das sie gebunden sind, $C_5$-$C_{20}$-Alkyl bilden und keiner dieser Substituenten $R^1$, $R^2$ und $R^3$ Wasserstoff sein darf und worin $R^4$ Wasserstoff, unsubstituiertes oder durch -OH, -$OCH_3$-, -CN oder -$N(R^6)_2$ substituiertes $C_1$-$C_{20}$-Alkyl ist, und $R^6$ $C_1$-$C_4$-Alkyl bedeutet, das gegebenenfalls durch -OH substituiert ist, und $R^4$ als $C_1$-$C_{20}$-Alkyl gegebenenfalls durch -O-, -S- oder -N< unterbrochen ist, oder $R^4$ $C_4$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl, unsubstituiertes oder durch ein oder zwei $C_1$-$C_4$-Alkyl oder -$CF_3$, eine oder zwei OH-Gruppen oder durch ein oder zwei -$N(R^7)(R^8)$ substituiertes $C_6$-, $C_{10}$- oder $C_{14}$-Aryl ist, und $R_7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_{10}$-Hetaryl, ein nichtaromatischer $C_2$-$C_5$-Heterocyclus, unsubstituiertes oder durch -OH, $C_1$-$C_4$-Alkoxy oder -$N(R^6)_2$ substituiertes $C_7$-$C_{14}$-Aralkyl ist oder $R^4$ -$CH(OH)$-$CH_2$-S-R ist, und $R^5$ unsubstituiertes oder durch -$OCH_3$, -CN oder -$N(R^6)_2$ substituiertes $C_5$-$C_{20}$- Alkyl mit $R^6$ in der vorstehenden Bedeutung ist, das gegebenenfalls durch -O-, -S- oder -N< unterbrochen sein kann, oder $R^5$ durch $N(R^6)$(Tolyl) oder $C_1$-$C_{10}$-Hetaryl substituiertes $C_1$-$C_{20}$-Alkyl ist, oder $R^5$ unsubstituiertes $C_4$-$C_{20}$-Alkenyl, oder durch ein oder mehrere -CN substituiertes $C_3$-$C_{20}$-Alkenyl, $C_4$-$C_{20}$-Alkinyl, unsubstituiertes oder durch $C_1$-$C_4$-Alkyl substituiertes $C_6$-$C_{12}$-Cycloalkyl oder unsubstituiertes oder durch eine oder mehrere $C_1$-$C_4$-Alkylgruppen, die gegebenenfalls durch -NH- oder -$N(R^6)$- unterbrochen sind, eine oder mehrere $C_1$-$C_4$-Alkoxy-$C_1$-$C_4$-Alkylthio- und/oder OH-Gruppen, ein oder mehrere -$NO_2$, -$CF_3$ und/oder -CN, Hydroxyethoxy, Phenoxy, Ureido, Carbamoyl, Sulfamoyl, Benzolazo, Toluolazo, Anilinocarbonyl, Anilinosulfonyl, -S-$CH_2$-$CH(OH)$-$CH_2$--S-R und/oder durch ein oder zwei $N(R^7)(R^8)$ substituiertes $C_6$-, $C_{10}$- oder $C_{14}$-Aryl ist, mit $R^7$ und $R^8$ in der vorstehenden Bedeutung, wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl oder $C_1$-$C_4$-Alkyl substituiertes $C_1$-$C_{10}$-Hetaryl, unsubstituiertes oder durch -OH, eine oder mehrere $C_1$-$C_4$-Alkoxygruppen oder durch -$N(R^6)_2$ substituiertes $C_7$-$C_{14}$-Aralkyl ist, mit $R^6$ in der vorstehenden Bedeutung, oder $R^5$ -$CH_2$-$CH(OH)$-$CH_2$-S-R ist oder $R^5$ einen 2wertigen $C_2$-$C_{12}$-aliphatischen Rest bedeutet, der sich von einem 2fach durch -$NH_2$ substituierten $C_2$-$C_{12}$-Alkan herleitet und unsubstituiert oder durch -OH, -$OCH_3$ oder -$N(R^6)_2$ mit $R^6$ in der vorstehenden Bedeutung substituiert sein kann und gegebenenfalls durch -O-, -S- oder -$N(R^9)$- unterbrochen ist, wobei $R^9$ unsubstituiertes oder durch -OH substituiertes $C_1$-$C_4$-Alkyl oder -$CH_2$-$CH(OH)$-$CH_2$-S-R ist, oder $R^5$ einen 2- bis 4wertigen $C_6$-$C_{12}$-cycloaliphatischen Rest bedeutet, der sich von einem 2fach bis 4fach durch -$NH_2$ substituierten $C_6$-$C_{12}$-Cycloalkan herleitet und unsubstituiert oder durch $C_1$-$C_4$-Alkyl substituiert sein kann, oder $R^5$ einen Rest der Formel

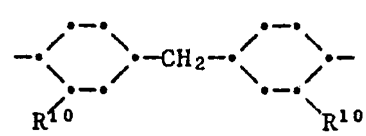

darstellt, worin $R^{10}$ Wasserstoff oder $C_1$-$C_4$-Alkyl ist, oder $R^5$ einen Rest der Formel

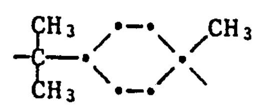

oder einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $-NO_2$ oder $C_1$-$C_4$-Alkyl substituiert sein kann, oder $R^5$ Anthrachinonylen, 2,3-Dihydroanthrachinonylen oder einen Rest der Formel

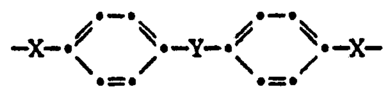

darstellt, worin X $-CH_2-$ oder eine direkte Bindung und Y $-CH_2-$, $-C(C_6H_5)H-$, -S-S-, -NH- oder eine direkte Bindung ist, oder $R^5$ einen 2- oder 3wertigen $C_2$-$C_{10}$-heteroaromatischen Rest bedeutet, der sich von einem 2fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^5$ einen 2wertigen $C_7$-$C_{14}$-araliphatischen Rest bedeutet, der sich von einem 2fach durch $-NH_2$ substituierten $C_7$-$C_{14}$-Aralkan herleitet, oder $R^4$ und $R^5$ zusammen mit dem N-Atom, an das sie gebunden sind, einen $C_1$-$C_7$-azacyclischen Ring bilden, der aromatisch oder nicht aromatisch sein kann und gegebenenfalls ein oder mehrere N-, O- oder S-Atome enthalten kann, wobei das N-Atom gegebenenfalls durch $C_1$-$C_4$-Alkyl, welches seinerseits durch -OH substituiert sein kann, substituiert ist und der $C_1$-$C_7$-azacyclische Ring an einem C-Atom gegebenenfalls durch $C_1$-$C_4$-Alkyl, =O oder =S substituiert sein kann, oder $R^4$ und $R^5$ zusammen mit dem N-Atom, an das sie gebunden sind, 2,2,4-Trimethyl-1,2-dihydrochinolyl, einen Teil eines $C_1$-$C_7$-azacyclischen 2- bis 6wertigen Rings oder ein Radikal der Formeln

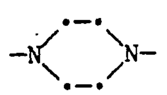

,

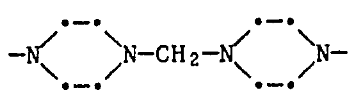

oder

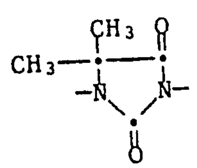

bedeuten.

11. Verbindungen gemäss Anspruch 10 der Formel Ia, worin n gleich 1 ist.

12. Verbindungen gemäss Anspruch 10 der Formel Ia, worin n gleich 2 bis 6 ist.

13. Verbindungen gemäss Anspruch 12 der Formel Ia, worin n gleich 2 ist.

14. Verbindungen gemäss Anspruch 10, worin in Formel Ia $R^4$ Wasserstoff, unsubstituiertes oder durch ein oder zwei $C_1$-$C_4$-Alkyl oder $-CF_3$, eine oder zwei OH-Gruppen oder durch ein oder zwei $-N(R^7)(R^8)$ substituiertes $C_6$-, $C_{10}$- oder $C_{14}$-Aryl ist und $R^7$ Wasserstoff oder $C_1$-$C_4$-Alkyl und $R^8$ $C_1$-$C_4$-Alkyl oder $C_6$- oder $C_{10}$-Aryl bedeuten, oder worin $R^4$ Anthrachinonyl, unsubstituiertes oder durch -OH oder $C_1$-$C_4$-Alkyl substituiertes $C_1$-$C_6$-Hetaryl bedeutet, und $R^5$ unsubstituiertes oder durch eine oder mehrere $C_1$-$C_4$-Alkylgruppen, die gegebenenfalls durch -NH- oder $-N(R^6)-$ unterbrochen sind, eine oder mehrere $C_1$-$C_4$-Alkoxy-$C_1$-$C_4$-Alkylthio- und/oder OH-Gruppen, ein oder mehrere $-NO_2$, $-CF_3$ und/oder -CN, Hydroxyethoxy, Phenoxy, Ureido, Carbamoyl, Sulfamoyl, Benzolazo, Toluolazo, Anilinocarbonyl, Anilinosulfonyl, $-S-CH_2-CH(OH)-CH_2-S-R$ und/oder durch ein oder zwei $-N(R^7)(R^8)$ substituiertes $C_6$-, $C_{10}$- oder $C_{14}$-Aryl ist, mit $R^7$ und $R^8$ in der vorstehenden Bedeutung, wobei $R^8$ zusätzlich Acetyl oder Methoxyphenyl darstellt, oder worin $R^5$ Anthrachinonyl, Hydroxyanthrachinonyl, unsubstituiertes oder durch -OH, Phenyl und/oder $C_1$-$C_4$-Alkyl substituiertes $C_1$-$C_{10}$-Hetaryl ist, oder $R^5$ einen 2- oder 3wertigen $C_6$-, $C_{10}$- oder $C_{14}$-aromatischen Rest bedeutet, der sich von einem 2fach oder 3fach durch $-NH_2$ substituierten $C_6$-, $C_{10}$- oder $C_{14}$-Aromaten herleitet und unsubstituiert oder durch -OH, $-NO_2$ oder $C_1$-$C_4$-Alkyl substituiert sein kann, oder $R^5$ Anthrachinonylen, 2,3-Dihydroanthrachinonylen oder einen Rest der Formel

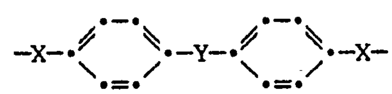

darstellt, worin X eine direkte Bindung und Y $-CH_2-$, $-C(C_6H_5)H-$, -S-S-, -NH- oder eine direkte Bindung ist, oder $R^5$ einen 2- oder 3wertigen $C_2$-$C_{10}$-heteroaromatischen Rest bedeutet, der sich von einem 2fach oder 3fach durch $-NH_2$ substituierten $C_2$-$C_{10}$-Heteroaromaten herleitet und unsubstituiert oder durch -OH oder $C_6$- oder $C_{10}$-Aryl substituiert ist, oder $R^4$ und $R^5$ zusammen mit dem N-Atom, an das sie gebunden sind, einen $C_1$-$C_7$-azacyclischen Ring bilden, der aromatisch ist und gegebenenfalls ein oder mehrere N-, O- oder S-Atome enthalten kann, oder $R^4$ und $R^5$ zusammen mit dem N-Atom, an das sie gebunden sind, einen 2,2,4-Trimethyl-1,2-dihydrochinolylrest bedeuten.

15. Verbindungen gemäss Anspruch 10 der Formel Ia, worin R ein Radikal der Formel

$R^3$
|
$R^1$-C-
|
$R^2$

bedeutet, wobei $R^1$, $R^2$ und $R^3$ zusammen mit dem C-Atom, an das sie gebunden sind, $C_8$-$C_{12}$-Alkyl bilden und keiner dieser Substituenten $R^1$, $R^2$ und $R^3$ Wasserstoff sein darf.

16. 1-Ethylthio-3-(triazol-3-yl)-amino-propan-2-ol.

17. Verwendung von Verbindungen der Formeln I nach Anspruch 1 und Ia nach Anspruch 10 als Hochdruck- und verschleissmindernde Additive in Schmiermitteln, Hydraulikflüssigkeiten oder Metallbearbeitungsflüssigkeiten.

**Claims**

1. A composition containing a lubricant, a hydraulic fluid or a metal-working fluid and at least one compound of the formula I

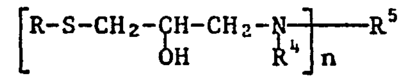
(I)

wherein n is 1 to 6 and R is a radical of the formula

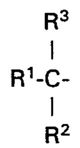

in which $R^1$, $R^2$ and $R^3$ independently of one another are $C_1$-$C_{21}$alkyl and together have not more than 22 carbon atoms, and $R^2$ and $R^3$ can also be hydrogen, or in which R is $C_5$-$C_{12}$cycloalkyl, $C_7$-$C_9$aralkyl, unsubstituted or $C_1$-$C_4$alkyl-substituted phenyl or naphthyl, furyl, furfuryl, thienyl, morpholinyl, imidazolyl, thiazolyl, oxazolyl, imidazolinyl, thiazolinyl, oxazolinyl, benzimidazolinyl, benzothiazolinyl or benzoxazolinyl, and in which $R^4$ is hydrogen or $C_1$-$C_{20}$alkyl which is unsubstituted or substituted by -OH, -$OCH_3$, -CN or -$N(R^6)_2$, and $R^6$ is unsubstituted or OH-substituted $C_1$-$C_4$alkyl, and $C_1$-$C_{20}$alkyl $R^4$ may be interrupted by -O-, -S- or -N<, or $R^4$ is $C_4$-$C_{20}$alkenyl, $C_4$-$C_{20}$alkynyl, unsubstituted or $C_1$-$C_4$alkyl-substituted $C_6$-$C_{12}$cycloalkyl, $C_6$-, $C_{10}$- or $C_{14}$aryl which is unsubstituted or substituted by one or two $C_1$-$C_4$alkyl or -$CF_3$, one or two OH groups or one or two -$N(R^7)(R^8)$, and $R^7$ is hydrogen or $C_1$-$C_4$alkyl and $R^8$ is $C_1$-$C_4$alkyl or $C_6$- or $C_{10}$aryl, or in which $R^4$ is anthraquinonyl, $C_1$-$C_{10}$heteroaryl which is unsubstituted or substituted by -OH or $C_1$-$C_4$alkyl, a non-aromatic $C_2$-$C_5$heterocyclic rings or $C_7$-$C_{14}$-aralkyl which is unsubstituted or substituted by -OH, $C_1$-$C_4$alkoxy or -$N(R^6)_2$ or $R^4$ is -$CH_2$-CH(OH)-$CH_2$-S-R, and $R^5$ is $C_4$-$C_{20}$alkyl which is unsubstituted or substituted by -$OCH_3$, -CN or -$N(R^6)_2$, $R^6$ being as defined above, and which may be interrupted by -O-, -S- or -N<, or $R^5$ is $C_1$-$C_{20}$alkyl which is substituted by $N(R^9)$(tolyl) or $C_1$-$C_{10}$heteroaryl, or $R^5$ is unsubstituted $C_4$-$C_{20}$alkenyl or $C_3$-$C_{20}$alkenyl substituted by one or more -CN, $C_4$-$C_{20}$alkynyl, unsubstituted or $C_1$-$C_4$alkyl-substituted $C_6$-$C_{12}$cycloalkyl or $C_6$-, $C_{10}$- or $C_{14}$aryl which is unsubstituted or substituted by one or more $C_1$-$C_4$alkyl groups which may be interrupted by -NH- or -$N(R^6)$-, one or more $C_1$-$C_4$alkoxy, $C_1$-$C_4$alkylthio and/or OH groups, one or more -$NO_2$, -$CF_3$ and/or -CN, hydroxyethoxy, phenoxy, ureido, carbamoyl, sulfamoyl, benzeneazo, tolueneazo, anilinocarbonyl, anilinosulfonyl, S-$CH_2$-CH(OH)-$CH_2$-S-R and/or one or two -$N(R^7)(R^8)$, $R^7$ and $R^8$ being as defined above and $R^8$ additionally also being acetyl or methoxyphenyl, or in which $R^5$ is anthraquinonyl, hydroxyanthraquinonyl, $C_1$-$C_{10}$heteroaryl which is unsubstituted or substituted by -OH, phenyl or $C_1$-$C_4$alkyl, or $C_7$-$C_{14}$aralkyl which is unsubstituted or substituted by -OH, one or more $C_1$-$C_4$alkoxy groups or by -$N(R^6)_2$, $R^6$ being as defined above, or $R^5$ is -$CH_2$-CH(OH)$CH_2$-S-R or $R^5$ is a divalent $C_2$-$C_{12}$-aliphatic radical which is derived from a $C_2$-$C_{12}$alkane disubstituted by -$NH_2$ and can be unsubstituted or substituted by -OH, -$OCH_3$ or -$N((R^6)_2$ and may be interrupted by -O-, -S- or -$N(R^9)$-, $R^9$ being unsubstituted or OH-substituted $C_1$-$C_4$alkyl or -$CH_2$-CH(OH)-$CH_2$-S-R, or $R^5$ is a divalent to tetravalent $C_6$-$C_{12}$cycloaliphatic radical which is derived from a $C_6$-$C_{12}$cycloalkane disubstituted or tetrasubstituted by -$NH_2$ and can be unsubstituted or substituted by $C_1$-$C_4$alkyl, or $R^5$ is a radical of the formula

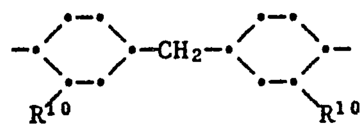

in which $R^{10}$ is hydrogen or $C_1$-$C_4$alkyl, or $R^5$ is a radical of the formula

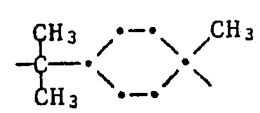

or a divalent or trivalent $C_6$-, $C_{10}$- or $C_{14}$aromatic radical which is derived from a $C_6$-, $C_{10}$- or $C_{14}$aromatic compound disubstituted or trisubstituted by -$NH_2$ and can be unsubstituted or substituted by -OH, -$NO_2$ or $C_1$-$C_4$alkyl, or $R^5$ is anthraquinonylene, 2,3-dihydroanthraquinonylene or a radical of the formula

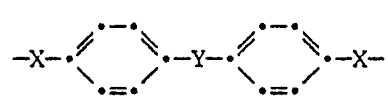

in which X is -$CH_2$- or a direct bond and Y is -$CH_2$-, -$C(C_6H_5)$-, -S-S-, -NH- or a direct bond, or $R^5$ is a divalent or trivalent $C_2$-$C_{10}$heteroaromatic radical which is derived from a $C_2$-$C_{10}$heteroaromatic ring disubstituted or trisubstituted by -$NH_2$ and is unsubstituted or substituted by -OH or $C_6$- or $C_{10}$aryl, or $R^5$ is a divalent $C_7$-$C_{14}$araliphatic radical which is derived from a $C_7$-$C_{14}$aralkane disubstituted by -$NH_2$, or $R^4$ and $R^5$ together with the N atom to which they are linked form a $C_1$-$C_7$azacyclic ring which can be aromatoc or non-aromatic and may contain one or more N, O or S atoms, the N atom being unsubstituted or substituted by $C_1$-$C_4$alkyl which in turn can be substituted by -OH, and the $C_1$-$C_7$azacyclic ring can be unsubstituted or substituted on one C atom by $C_1$-$C_4$alkyl, =O or =S, or $R^4$ and $R^5$ together with the N atom to which they are linked are 2,2,4-trimethyl-1,2-dihydroquinonyl, a part of a $C_1$-$C_7$azacyclic divalent to hexavalent ring or a radical of the formulae

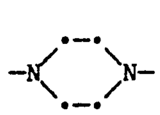

,

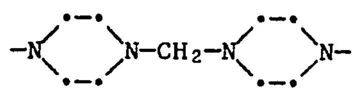

or

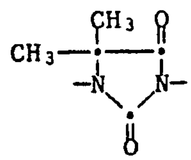

2. A composition according to claim 1, wherein n in the formula I is 1.

3. A composition according to claim 1, wherein n in the formula I is 2 to 6.

4. A composition according to claim 3, wherein n in the formula I is 2.

5. A composition according to claim 1, wherein R in the formula I is a radical of the formula

$R^3$
I
$R^1$-C-
I
$R^2$

in which $R^1$, $R^2$ and $R^3$ independently of one another are $C_1$-$C_{18}$alkyl and do not have more than 22 carbon atoms in total, and $R^2$ and $R^3$ can also be hydrogen, or wherein R is $C_5$-$C_6$cycloalkyl, benzyl, unsubstituted or $C_1$-$C_4$alkyl-substituted phenyl or unsubstituted naphthyl.

6. A composition according to claim 5, wherein R in the formula I is a radical of the formula

$R^3$
I
$R^1$-C-
I
$R^2$

in which $R^1$, $R^2$ and $R^3$ together with the carbon atom to which they are linked form $C_4$-$C_{16}$alkyl, in which case none of these substituents $R^1$, $R^2$ and $R^3$ may be hydrogen, or wherein R is cyclohexyl, benzyl, unsubstituted or $C_1$-$C_4$alkyl-substituted phenyl or unsubstituted naphthyl.

7. A composition according to claim 1, wherein R in the formula I is a radical of the formula

$R^3$
I
$R^1$-C-
I
$R^2$

in which $R^1$ is $C_1$-$C_{12}$alkyl and $R^2$ and $R^3$ are hydrogen.

8. A composition according to claim 1, wherein R in the formula I is a radical of the formula

$R^3$
I
$R^1$-C-
I
$R^2$

in which $R^1$ and $R^2$ independently of one another are $C_1$-$C_{12}$alkyl and together with the carbon atom to which they are linked form $C_3$-$C_{14}$alkyl and $R^3$ is hydrogen.

9. A composition according to claim 1, wherein $R^4$ in the formula I is hydrogen, $C_6$-, $C_{10}$- or $C_{14}$aryl which is unsubstituted or substituted by one or two $C_1$-$C_4$alkyl or -$CF_3$, one or two OH groups or one or two -N($R^7$)($R^8$) and $R^7$ is hydrogen or $C_1$-$C_4$alkyl and $R^8$ is $C_1$-$C_4$alkyl or $C_6$- or $C_{10}$aryl or wherein $R^4$ is anthraquinonyl or is $C_1$-$C_{10}$heteroaryl which is unsubstituted or substituted by -OH or $C_1$-$C_4$alkyl, and $R^5$ is $C_6$-, $C_{10}$- or $C_{14}$ aryl which is unsubstituted or substituted by one or more $C_1$-$C_4$alkyl groups which may be interrupted by -NH- or -N($R^6$)-, one or more $C_1$-$C_4$alkoxy, $C_1$-$C_4$alkylthio and/or OH groups, one or more -$NO_2$, -$CF_3$ and/or -CN, hydroxyethoxy, phenoxy, ureido, carbamoyl, sulfamoyl, benzeneazo, tolueneazo, anilinocarbonyl, anilinosulfonyl, -S-$CH_2$-CH(OH)-$CH_2$-S-R and/or one or two -N($R^7$)-($R^8$), $R^7$ and $R^8$ being as defined above and $R^8$ also being acetyl or methoxyphenyl, or wherein $R^5$ is anthraquinonyl, hydroxyanthraquinonyl or is $C_1$-$C_{10}$-heteroaryl which is unsubstituted or substituted by -OH, phenyl and/or $C_1$-$C_4$alkyl or $R^5$ is a divalent or trivalent $C_6$-, $C_{10}$- or $C_{14}$aromatic radical which is derived from a $C_6$-, $C_{10}$- or $C_{14}$aromatic compound disubstituted or trisubstituted by -$NH_2$ and can be unsubstituted or substituted by -OH, $NO_2$ or $C_1$-$C_4$-alkyl, or $R^5$ is anthraquinonylene, 2,3-dihydroanthraquinonylene or a radical of the formula

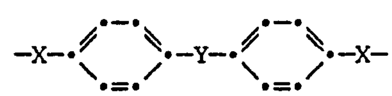

in which X is a direct bond and Y is -$CH_2$-, -C($C_6H_5$)-H-, -S-S-, -NH- or a direct bond, or $R^5$ is a divalent or trivalent $C_2$-$C_{10}$heteroaromatic radical which is derived from a $C_2$-$C_{10}$heteroaromatic compound disubstituted or trisubstituted by -$NH_2$ and is unsubstituted or substituted by -OH or $C_6$- or $C_{10}$aryl, or $R^4$ and $R^5$ together with the N atom to which they are linked form a $C_1$-$C_7$azacyclic ring which is aromatic and may contain one or more N, O or S atoms, or $R^4$ and $R^5$ together with the N atom to which they are linked are a 2,2,4-trimethyl-1,2-dihydroquinolyl radical.

10. A compound of the formula Ia

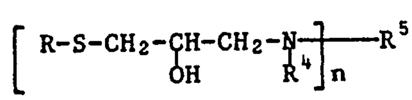

(Ia)

wherein n is 1 to 6 and R is a radical of the formula

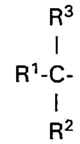

in which $R^1$, $R^2$ and $R^3$ together with the carbon atom to which they are linked form $C_5$-$C_{20}$alkyl and none of these substituents $R^1$, $R^2$ and $R^3$ may be hydrogen, and wherein $R^4$ is hydrogen or $C_1$-$C_{20}$alkyl which is unsubstituted or substituted by -OH, $-OCH_3$, -CN or $-N(R^6)_2$, and $R^6$ is unsubstituted or OH-substituted $C_1$-$C_4$alkyl, and $C_1$-$C_{20}$alkyl $R^4$ may be interrupted by -O-, -S- or -N<, or $R^4$ is $C_4$-$C_{20}$alkenyl, $C_4$-$C_{20}$alkynyl, unsubstituted or $C_1$-$C_4$alkyl-substituted $C_6$-$C_{12}$cycloalkyl, $C_6$-, $C_{10}$- or $C_{14}$aryl which is unsubstituted or substituted by one or two $C_1$-$C_4$alkyl or $-CF_3$, one or two OH groups or one or two $-N(R^7)(R^8)$, and $R^7$ is hydrogen or $C_1$-$C_4$alkyl and $R^8$ is $C_1$-$C_4$alkyl or $C_6$- or $C_{10}$aryl, or in which $R^4$ is anthraquinonyl, $C_1$-$C_{10}$heteroaryl which is unsubstituted or substituted by -OH or $C_1$-$C_4$alkyl, a non-aromatic $C_2$-$C_5$heterocyclic ring or $C_7$-$C_{14}$aralkyl which is unsubstituted or substituted by -OH, $C_1$-$C_4$alkoxy or $-N(R^6)_2$ or $R^4$ is $-CH(OH)-CH_2-S-R$, and $R^5$ is $C_5$-$C_{20}$alkyl which is unsubstituted or substituted by $-OCH_3$, -CN or $-N(R^6)_2$, $R^6$ being as defined above, and which may be interrupted by -O-, -S- or -N<, or $R^5$ is $C_1$-$C_{20}$alkyl which is substituted by $N(R^6)$(tolyl) or $C_1$-$C_{10}$heteroaryl, or $R^5$ is unsubstituted $C_4$-$C_{20}$alkenyl or $C_3$-$C_{20}$alkenyl substituted by one or more -CN, $C_4$-$C_{20}$alkynyl, unsubstituted or $C_1$-$C_4$alkyl-substituted $C_6$-$C_{12}$cycloalkyl or $C_6$-, $C_{10}$- or $C_{14}$aryl which is unsubstituted or substituted by one or more $C_1$-$C_4$alkyl groups which may be interrupted by -NH- or $-N(R^6)-$, one or more $C_1$-$C_4$alkoxy, $C_1$-$C_4$alkylthio and/or OH groups, one or more $-NO_2$, $-CF_3$ and/or -CN, hydroxyethoxy, phenoxy, ureido, carbamoyl, sulfamoyl, benzeneazo, tolueneazo, anilinocarbonyl, anilinosulfonyl, $S-CH_2-CH(OH)-CH_2-$ -S-R and/or one or two $-N(R^7)(R^8)$, $R^7$ and $R^8$ being as defined above and $R^8$ additionally also being acetyl or methoxyphenyl, or in which $R^5$ is anthraquinonyl, hydroxyanthraquinonyl, $C_1$-$C_{10}$heteroaryl which is unsubstituted or substituted by -OH, phenyl or $C_1$-$C_4$alkyl, or $C_7$-$C_{14}$aralkyl which is unsubstituted or substituted by -OH, one or more $C_1$-$C_4$alkoxy groups or by $-N(R^6)_2$, $R^6$ being as defined above, or $R^5$ is $-CH_2-CH(OH)CH_2-S-R$ or $R^5$ is a divalent $C_2$-$C_{12}$aliphatic radical which is derived from a $C_2$-$C_{12}$alkane disubstituted by $-NH_2$ and can be unsubstituted or substituted by -OH, $-OCH_3$ or $-N(R^6)_2$, $R^6$ being as defined above, and may be interrupted by -O-, -S- or $N(R^9)-$, $R^9$ being unsubstituted or OH-substituted $C_1$-$C_4$alkyl or $-CH_2-CH(OH)-CH_2-S-R$, or $R^5$ is a divalent to tetravalent $C_6$-$C_{12}$cycloaliphatic radical which is derived from a $C_6$-$C_{12}$cycloalkane disubstituted or tetrasubstituted by $-NH_2$ and can be unsubstituted or substituted by $C_1$-$C_4$alkyl, or $R^5$ is a radical of the formula

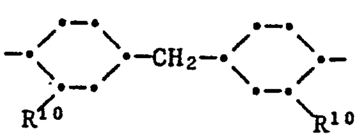

in which $R^{10}$ is hydrogen or $C_1$-$C_4$alkyl, or $R^5$ is a radical of the formula

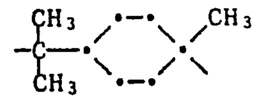

or a divalent or trivalent $C_6$-, $C_{10}$- or $C_{14}$aromatic radical which is derived from a $C_6$-, $C_{10}$- or $C_{14}$aromatic compound disubstituted or trisubstituted by $-NH_2$ and can be unsubstituted or substituted by -OH, $-NO_2$ or $C_1$-$C_4$alkyl, or $R^5$ is anthraquinonylene, 2,3-dihydroanthraquinonylene or a radical of the formula

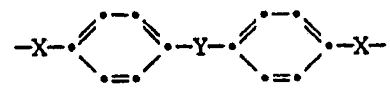

in which X is $-CH_2-$ or a direct bond and Y is $-CH_2-$, $-C(C_6H_5)-$, -S-S-, -NH- or a direct bond, or $R^5$ is a divalent or trivalent $C_2$-$C_{10}$heteroaromatic radical which is derived from a $C_2$-$C_{10}$heteroaromatic ring disubstituted or trisubstituted by $-NH_2$ and is unsubstituted or substituted by -OH or $C_6$- or $C_{10}$aryl, or $R^5$ is a divalent $C_7$-$C_{14}$araliphatic radical which is derived from a $C_7$-$C_{14}$aralkane disubstituted by $-NH_2$, or $R^4$ and $R^5$ together with the N atom to which they are linked form a $C_1$-$C_7$azacyclic ring which can be aromatic or non-aromatic and may contain one or more N, O or S atoms, the N atom being unsubstituted or substituted by $C_1$-$C_4$alkyl which in turn can be substituted by -OH, and the $C_1$-$C_7$azacyclic ring can be unsubstituted or substituted on one C atom by $C_1$-$C_4$alkyl, =O or =S, or $R^4$ and $R^5$ together with the N atom to which they are linked are 2,2,4-trimethyl-1,2-dihydroquinolyl, a part of a $C_1$-$C_7$azacyclic divalent to hexavalent ring or a radical of the formulae

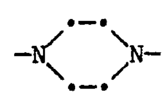,
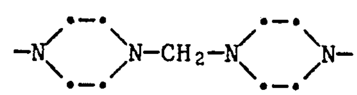

or

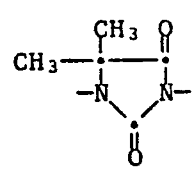

11. A compound according to claim 10 of the formula Ia, wherein n is 1.

12. A compound according to claim 10 of the formula Ia, wherein n is 2 to 6.

13. A compound according to claim 12 of the formula Ia, wherein n is 2.

14. A compound according to claim 10, wherein $R^4$ in the formula I is hydrogen, $C_6$-, $C_{10}$ or $C_{14}$aryl which is unsubstituted or substituted by one or two

$C_1$-$C_4$alkyl or -$CF_3$, one or two OH groups or one or two -N($R^7$)($R^8$) and $R^7$ is hydrogen or $C_1$-$C_4$alkyl and $R^8$ is $C_1$-$C_4$alkyl or $C_6$- or $C_{10}$aryl or wherein $R^4$ is anthraquinonyl or is $C_1$-$C_6$heteroaryl which is unsubstituted or substituted by -OH or $C_1$-$C_4$alkyl, and $R^5$ is $C_6$-, $C_{10}$- or $C_{14}$aryl which is unsubstituted or substituted by one or more $C_1$-$C_4$alkyl groups which may be interrupted by -NH- or -N($R^6$)-, one or more $C_1$-$C_4$alkoxy, $C_1$-$C_4$alkylthio and/or OH groups, one or more -$NO_2$, -$CF_3$ and/or -CN, hydroxyethoxy, phenoxy, ureido, carbamoyl, sulfamoyl, benzeneazo, tolueneazo, anilinocarbonyl, anilinosulfonyl, -S-$CH_2$-CH(OH)-$CH_2$-S-R and/or one or two -N($R^7$)($R^8$), $R^7$ and $R^8$ being as defined above and $R^8$ also being acetyl or methoxyphenyl, or wherein $R^5$ is anthraquinonyl, hydroxyanthraquinonyl or is $C_1$-$C_{10}$heteroaryl which is unsubstituted or substituted by -OH, phenyl and/or $C_1$-$C_4$alkyl or $R^5$ is a divalent or trivalent $C_6$-, $C_{10}$- or $C_{14}$aromatic radical which is derived from a $C_6$-, $C_{10}$- or $C_{14}$aromatic compound disubstituted or trisubstituted by -$NH_2$ and can be unsubstituted or substituted by -OH, $NO_2$ or $C_1$-$C_4$alkyl, or $R^5$ is anthraquinonylene, 2,3-dihydroanthraquinonylene or a radical of the formula

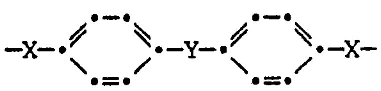

in which X is a direct bond and Y is -$CH_2$-, -C($C_6H_5$)H-, -S-S-, -NH- or a direct bond, or $R^5$ is a divalent or trivalent $C_2$-$C_{10}$heteroaromatic radical which is derived from a $C_2$-$C_{10}$heteroaromatic and is unsubstituted or substituted by -OH or $C_6$- or $C_{10}$aryl, or $R^4$ and $R^5$ together with the N atom to which they are linked form a $C_1$-$C_7$azacyclic ring which is aromatic and may contain one or more N, O or S atoms, or $R^4$ and $R^5$ together with the N atom to which they are linked are a 2,2,4-trimethyl-1,2-dihydroquinolyl radical.

15. A compound according to claim 10 of the formula Ia, wherein R is a radical of the formula

$R^3$
|
$R^1$-C-
|
$R^2$

in which $R^1$, $R^2$ and $R^3$ together with the carbon atom to which they are linked form $C_8$-$C_{12}$alkyl and none of these substituents $R^1$, $R^2$ and $R^3$ must be hydrogen.

16. 1-Ethylthio-3-(triazol-3-yl)-amino-propan-2-ol.

17. The use of a compound of the formulae I according to claim 1 and Ia according to claim 10 as an extreme-pressure and anti-wear additive in lubricants, hydraulic fluids or metal-working fluids.

**Revendications**

1. Composition contenant un lubrifiant, un liquide hydraulique et un liquide pour l'usinage de métaux et au moins un composé répondant à la formule I:

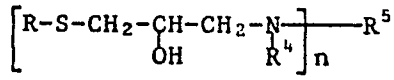 (I)

dans laquelle:

n désigne un nombre de 1 à 6,

R représente un radical de formule:

$R^3$
|
$R^1$-C-
|
$R^2$

dans lequel $R^1$, $R^2$ et $R^3$ représentent chacun, indépendamment les uns des autres, un alkyle en $C_1$-$C_{21}$ et, ensemble, ne contiennent pas plus de 22 atomes de carbone, et $R^2$ et $R^3$ peuvent en outre représenter l'hydrogène, ou

R représente cycloalkyle en $C_5$-$C_{12}$, un aralkyle en $C_7$-$C_9$, un radical phényle ou naphtyle non substitué ou porteur d'un alkyle en $C_1$-$C_4$, un furyle, un furfuryle, un thiényle, un morpholinyle, un imidazolyle, un thiazolyle, un oxazolyle, un imidazolinyle, un thiazolinyle, un oxazolinyle, un benzimidazolinyle, un benzothiazolinyle ou un benzoxazolinyle,

$R^4$ représente l'hydrogène, ou un alkyle en $C_1$-$C_{20}$ non substitué ou porteur d'un -OH, d'un -$OCH_3$, d'un -CN ou d'un -N($R^6$)$_2$, le symbole $R^6$ désignant un alkyle en $C_1$-$C_4$ éventuellement porteur d'un -OH, et l'alkyle en $C_1$-$C_{20}$ que représente $R^4$ étant éventuellement interrompu par -O-, -S- ou -N⟨, ou

$R^4$ représente un alcényle en $C_4$-$C_{20}$, un alcynyle en $C_4$-$C_{20}$, un cycloalkyle en $C_6$-$C_{12}$ non substitué ou porteur d'un alkyle en $C_1$-$C_4$, ou un aryle en $C_6$, en $C_{10}$ ou en $C_{14}$ non substitué ou porteur d'un ou de deux radicaux alkyles en $C_1$-$C_4$ ou -$CF_3$, d'un ou de deux radicaux -OH ou d'un ou de deux radicaux -N($R^7$)($R^8$), le symbole $R^7$ désignant l'hydrogène ou un alkyle en $C_1$-$C_4$ et $R^8$ un alkyle en $C_1$-$C_4$ ou un aryle en $C_6$ ou $C_{10}$, ou

$R^4$ représente un anthraquinonyle, un hétéroaryle en $C_1$-$C_{10}$ non substitué ou porteur d'un -OH ou d'un alkyle en $C_1$-$C_4$, un hétérocycle non-aromatique en $C_2$-$C_5$ ou un aralkyle en $C_7$-$C_{14}$ non substitué ou porteur d'un -OH, d'un alcoxy en $C_1$-$C_4$ ou d'un radical -N($R^6$)$_2$, ou

$R^4$ représente un radical -$CH_2$-CH(OH)-$CH_2$-S-R, et

$R^5$ représente un alkyle en $C_4$-$C_{20}$ non substitué ou porteur d'un -$OCH_3$, d'un -CN ou d'un radical -N($R^6$)$_2$ dont le symbole $R^6$ a la signification précédemment donnée, ce radical alkyle en $C_4$-$C_{20}$ pouvant être interrompu par -O-, -S- ou -N⟨, ou

$R^5$ représente un alkyle en $C_1$-$C_{20}$ porteur d'un radical -N($R^6$)(tolyl) ou d'un hétéro-aryle en $C_1$-$C_{10}$, ou

$R^5$ représente un alcényle en $C_4$-$C_{20}$ non substitué, un alcényle en $C_3$-$C_{20}$ porteur d'un ou de plusieurs -CN, un alcynyle en $C_4$-$C_{20}$, un cyclo-alkyle en $C_6$-$C_{12}$ non substitué ou porteur d'un alkyle en $C_1$-$C_4$, ou un radical aryle en $C_6$, en $C_{10}$ ou en $C_{14}$

non substitué ou porteur d'un ou plusieurs alkyles en $C_1$-$C_4$, éventuellement interrompus par -NH- ou -N($R^6$)-, d'un ou plusieurs radicaux alcoxy en $C_1$-$C_4$, alkylthio en $C_1$-$C_4$ et/ou -OH, d'un ou plusieurs radicaux -$NO_2$, -$CF_3$ et/ou -CN, d'un hydroxy-éthoxy, d'un phénoxy, d'un uréido, d'un carbamoyle, d'un sulfamoyle, d'un benzèneazo, d'un toluène-azo, d'un anilinocarbonyle, d'un anilinosulfonyle, d'un radical -S-$CH_2$-CH(OH)-$CH_2$-S-R et/ou d'un ou de deux radicaux -N($R^7$)($R^8$), les symboles $R^7$ et $R^8$ ayant les significations indiquées ci-dessus et $R^8$ pouvant en outre représenter un acétyle ou un méthoxyphényle, ou

$R^5$ représente un anthraquinonyle, un hydroxy-anthraquinonyle, un hétéro-aryle en $C_1$-$C_{10}$ non substitué ou porteur d'un -OH, d'un phényle ou d'un alkyle en $C_1$-$C_4$, ou un aralkyle en $C_7$-$C_{14}$ non substitué ou porteur d'un -OH, d'un ou plusieurs alcoxy en $C_1$-$C_4$ ou d'un radical -N($R^6$)$_2$ dont le symbole $R^6$ a la signification indiquée plus haut, ou

$R^5$ représente un radical -$CH_2$-CH(OH)$CH_2$-S-R, ou

$R^5$ représente un radical aliphatique bivalent en $C_2$-$C_{12}$ qui dérive d'un alcane en $C_2$-$C_{12}$ porteur de deux radicaux -$NH_2$, ce radical aliphatique ne portant pas de substituant ou pouvant porteur un -OH, un -$OCH_3$ ou un radical -N($R^6$)$_2$ et étant éventuellement interrompu par -O-, par -S- ou par un radical -N($R^9$)- dont le symbole $R^9$ représente un alkyle en $C_1$-$C_4$ non substitué ou porteur d'un -OH ou un radical -$CH_2$-CH(OH)-$CH_2$-S-R, ou

$R^5$ représente un radical cycloaliphatique en $C_6$-$C_{12}$ bivalent, trivalent ou quadrivalent qui dérive d'un cycloalcane en $C_2$-$C_{12}$ porteur de deux à quatre radicaux -$NH_2$ et qui peut être dépourvu de substituant ou porter un alkyle en $C_1$-$C_4$, ou

$R^5$ représente un radical de formule:

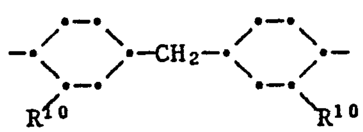

dans lequel $R^{10}$ représente l'hydrogène ou un alkyle en $C_1$-$C_4$, ou

$R^5$ représente un radical de formule:

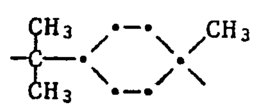

ou un radical aromatique bivalent ou trivalent en $C_6$, en $C_{10}$ ou en $C_{14}$ qui dérive d'un hydrocarbure aromatique en $C_6$, en $C_{10}$ ou en $C_{14}$ porteur de 2 ou 3 radicaux -$NH_2$ et qui peut être non substitué ou porter un -OH, un-$NO_2$ ou un alkyle en $C_1$-$C_4$, ou

$R^5$ représente un anthraquinonylène, un dihydro-2,3 anthraquinonylène ou un radical de formule:

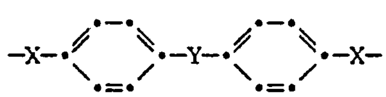

dans lequel X représente -$CH_2$- ou une liaison directe et Y représente -$CH_2$-, -C($C_6H_5$)H-, -S-S-, -NH- ou une liaison directe, ou

$R^5$ représente un radical hétéro-aromatique bivalent ou trivalent en $C_2$-$C_{10}$ qui dérive d'un composé hétéro-aromatique en $C_2$-$C_{10}$ porteur de 2 ou de 3 radicaux -$NH_2$ et qui est dépourvu de substituant ou porte un -OH ou un aryle en $C_6$ ou $C_{10}$, ou

$R^5$ représente un radical araliphatique bivalent en $C_7$-$C_{14}$ qui dérive d'un aralcane en $C_7$-$C_{14}$ porteur de 2 radicaux -$NH_2$, ou

$R^4$ et $R^5$ forment ensemble, et avec l'atome d'azote auquel ils sont liés, un radical azacyclique en $C_1$-$C_7$ qui peut être aromatique ou non-aromatique et qui peut éventuellement contenir un ou plusieurs atomes N, O ou S, l'atome N pouvant porter un alkyle en $C_1$-$C_4$, lui-même éventuellement porteur d'un -OH, et le radical azacyclique en $C_1$-$C_7$ portant éventuellement, sur un atome de carbone, un alkyle en $C_1$-$C_4$, =O ou =S, ou

$R^4$ et $R^5$ représentent ensemble, et avec l'atome d'azote auquel ils sont liés, un radical triméthyl-2,2,4 dihydro-1,2 quinonyle, une partie d'un radical azacyclique bi- à hexavalent en $C_1$-$C_7$ ou un radical répondant à l'une des formules:

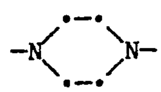

,

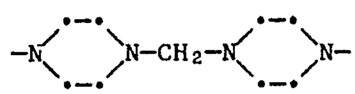

et

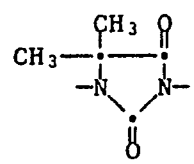

2. Composition selon la revendication 1 dans laquelle l'indice n contenu dans la formule I est égal à 1.

3. Composition selon la revendication 1 dans laquelle l'indice n contenu dans la formule I désigne un nombre de 2 à 6.

4. Composition selon la revendication 3 dans laquelle l'indice n contenu dans la formule I représente le nombre 2.

5. Composition selon la revendication 1 caractérisée en ce que, dans la formule I, R représente un radical de formule:

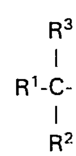

dans lequel $R^1$, $R^2$ et $R^3$ représentent chacun, indépendamment les uns des autres, un alkyle en $C_1$-$C_{18}$

et, ensemble, ne contiennent pas plus de 22 atomes de carbone, $R^2$ et $R^3$ pouvant en outre représenter chacun l'hydrogène, ou R représente un cycloalkyle en $C_5$ ou $C_6$, un benzyle, un phényle non substitué ou porteur d'un alkyle en $C_1$-$C_4$, ou un naphtyle non substitué.

6. Composition selon la revendication 5 caractérisée en ce que, dans la formule I, R représente un radical de formule:

$R^3$
I
$R^1$-C-
I
$R^2$

dans lequel $R^1$, $R^2$ et $R^3$ forment ensemble et avec l'atome de carbone auquel ils sont liés un radical alkyle en $C_4$-$C_{16}$, aucun de ces symboles $R^1$, $R^2$ et $R^3$ ne représentent l'hydrogène, ou R représente un cyclohexyle, un benzyle, un phényle non substitué ou porteur d'un alkyle en $C_1$-$C_4$, ou un naphtyle non substitué.

7. Composition selon la revendication 1 dans laquelle le symbole R contenu dans la formule I représente un radical de formule:

$R^3$
I
$R^1$-C-
I
$R^2$

dans lequel $R^1$ représente un alkyle en $C_1$-$C_{12}$, et $R^2$ et $R^3$ représentent chacun l'hydrogène.

8. Composition selon la revendication 1 dans laquelle le symbole R contenu dans la formule I représente un radical de formule:

$R^3$
I
$R^1$-C-
I
$R^2$

dans lequel $R^1$ et $R^2$ représentent chacun, indépendamment l'un de l'autre, un alkyle en $C_1$-$C_{12}$ et forment ensemble et avec l'atome de carbone auquel ils sont liés un radical alkyle en $C_3$-$C_{14}$, et $R^3$ représente l'hydrogène.

9. Composition selon la revendication 1 caractérisée en ce que, dans la formule I, $R^4$ représente l'hydrogène ou un radical aryle en $C_6$, en $C_{10}$ ou en $C_{14}$ non substitué ou porteur d'un ou de deux alkyles en $C_1$-$C_4$ ou $-CF_3$, d'un ou de deux groupes -OH ou d'un ou de deux radicaux $-N(R^7)(R^8)$, $R^7$ désignant l'hydrogène ou un alkyle en $C_1$-$C_4$ et $R^8$ un alkyle en $C_1$-$C_4$ ou un aryle en $C_6$ ou $C_{10}$, ou $R^4$ représente un anthraquinonyle ou un radical hétéro-aryle en $C_1$-$C_{10}$ non substitué ou porteur d'un-OH ou d'un alkyle en $C_1$-$C_4$, et $R^5$ représente un radical aryle en $C_6$, en $C_{10}$ ou en $C_{14}$ non substitué ou porteur d'un ou plusieurs alkyles en $C_1$-$C_4$, éventuellement interrompus par -NH ou $-N(R^6)$-, d'un ou plusieurs radicaux alcoxy en $C_1$-$C_4$, alkylthio en $C_1$-$C_4$ et/ou -OH, d'un ou plusieurs radicaux $-NO_2$, $-CF_3$ et/ou -CN, d'un hydroxyéthoxy, d'un phénoxy, d'un uréido, d'un carbamoyle, d'un sulfamoyle, d'un benzène-azo, d'un toluène-azo, d'un anilinocarbonyle, d'un anilinosulfonyle, d'un radical $-S-CH_2-CH(OH)-CH_2-S-R$ et/ou d'un ou de deux radicaux $-N(R^7)(R^8)$, les symboles $R^7$ et $R^8$ ayant les significations indiquées ci-dessus et $R^8$ pouvant en outre représenter un acétyle ou un méthoxyphényle, ou $R^5$ représente un anthraquinonyle, un hydroxyanthraquinonyle, ou un radical hétéro-aryle en $C_1$-$C_{10}$ non substitué ou porteur d'un -OH, d'un phényle et/ou d'un alkyle en $C_1$-$C_4$, ou $R^5$ représente un radical aromatique en $C_6$, en $C_{10}$ ou en $C_{14}$, bivalent ou trivalent, qui provient d'un hydrocarbure aromatique en $C_6$, en $C_{10}$ ou en $C_{14}$ porteur de deux ou de trois radicaux $-NH_2$ et qui peut être dépourvu de substituant ou porter un -OH, un $-NO_2$ ou un alkyle en $C_1$-$C_4$, ou $R^5$ représente un anthraquinonylène, dihydro-2,3-anthraquinonylène ou un radical de formule:

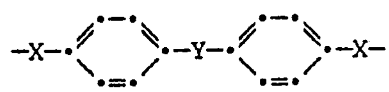

dans lequel X représente une liaison directe et Y représente $-CH_2$-, $-C(C_6H_5)H$-, -S-S-, -NH- ou une liaison directe, ou $R^5$ représente un radical hétéroaromatique en $C_2$-$C_{10}$ bivalent ou trivalent qui provient d'un hydrocarbure hétéro-aromatique en $C_2$-$C_{10}$ porteur de 2 ou de 3 radicaux $-NH_2$ et qui est dépourvu de substituant ou porte un -OH ou un aryle en $C_6$ ou $C_{10}$, ou $R^4$ et $R^4$ forment ensemble, et avec l'atome N auquel ils sont liés, un radical azacyclique en $C_1$-$C_1$ qui est aromatique et qui peut éventuellement contenir un ou plusieurs atomes N, O ou S, ou $R^4$ et $R^5$ forment ensemble, et avec l'atome N auquel ils sont liés, un radical triméthyl-2,2,4 dihydro-1,2 quinolyle.

10. Composés qui répondent à la formule Ia:

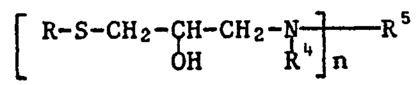

(Ia)

dans laquelle:

n désigne un nombre de 1 à 6,

R représente un radical de formule:

$R^3$
I
$R^1$-C-
I
$R^2$

dans lequel $R^1$, $R^2$ et $R^3$ forment ensemble et avec l'atome de carbone auquel ils sont liés un radical alkyle en $C_5$-$C_{20}$ et aucun des symboles $R^1$, $R^2$ et $R^3$ ne représente l'hydrogène,

$R^4$ représente l'hydrogène ou un alkyle en $C_1$-$C_{20}$ non substitué ou porteur d'un radical -OH, $-OCH_3$, -CN ou $-N(R^6)_2$, le symbole $R^6$ désignant un alkyle en $C_1$-$C_4$ éventuellement porteur d'un -OH, et l'alkyle

en $C_1$-$C_{20}$ que peut représenter $R^4$ étant éventuellement interrompu par -O-, -S- ou -N<, ou

$R^4$ représente un alcényle en $C_4$-$C_{20}$, un alcynyle en $C_4$-$C_{20}$, un cycloalkyle en $C_6$-$C_{12}$ non substitué ou porteur d'un alkyle en $C_1$-$C_4$, ou un aryle en $C_6$, en $C_{10}$ ou en $C_{14}$ non substitué ou porteur d'un ou de deux radicaux alkyles en $C_1$-$C_4$ ou -$CF_3$, d'un ou de deux radicaux -OH ou d'un ou de deux radicaux -N($R^7$)($R^8$), le symbole $R^7$ désignant l'hydrogène ou un alkyle en $C_1$-$C_4$ et $R^8$ un alkyle en $C_1$-$C_4$ ou un aryle en $C_6$ ou $C_{10}$, ou

$R^4$ représente un anthraquinonyle, un hétéroaryle en $C_1$-$C_{10}$ non substitué ou porteur d'un -OH ou d'un alkyle en $C_1$-$C_4$, un hétérocycle non-aromatique en $C_2$-$C_5$ ou un aralkyle en $C_7$-$C_{14}$ non substitué ou porteur d'un -OH, d'un alcoxy en $C_1$-$C_4$ ou d'un radical -N($R^6$)$_2$, ou

$R^4$ représente un radical -$CH_2$-CH(OH)-$CH_2$-S-R, et

$R^5$ représente un alkyle en $C_5$-$C_{20}$ non substitué ou porteur d'un -$OCH_3$, d'un -CN ou d'un radical -N($R^6$)$_2$ dont le symbole $R^6$ a la signification précédemment donnée, ce radical alkyle pouvant éventuellement être interrompu par -O-, -S- ou -N<, ou

$R^5$ représente un alkyle en $C_1$-$C_{20}$ porteur d'un radical -N($R^6$)(tolyl) ou d'un hétéro-aryle en $C_1$-$C_{10}$, ou

$R^5$ représente un alcényle en $C_4$-$C_{20}$ non substitué, un alcényle en $C_3$-$C_{20}$ porteur d'un ou de plusieurs -CN, un alcynyle en $C_4$-$C_{20}$, un cyclo-alkyle en $C_6$-$C_{12}$ non substitué ou porteur d'un alkyle en $C_1$-$C_4$, ou un radical aryle en $C_6$, en $C_{10}$ ou en $C_{14}$ non substitué ou porteur d'un ou plusieurs alkyles en $C_1$-$C_4$, éventuellement interrompus par -NH- ou -N($R^6$)-, d'un ou plusieurs radicaux alcoxy en $C_1$-$C_4$, alkylthio en $C_1$-$C_4$ et/ou -OH, d'un ou plusieurs radicaux -$NO_2$, -$CF_3$ et/ou -CN, d'un hydroxy-éthoxy, d'un phénoxy, d'un uréido, d'un carbamoyle, d'un sulfamoyle, d'un benzène-azo, d'un toluène-azo, d'un anilinocarbonyle, d'un anilinosulfonyle, d'un radical -S-$CH_2$-CH(OH)-$CH_2$-S-R et/ou d'un ou de deux radicaux -N($R^7$)($R^8$), les symboles $R^7$ et $R^8$ ayant les significations indiquées ci-dessus et $R^8$ pouvant en outre représenter un acétyle ou un méthoxyphényle, ou

$R^5$ représente un anthraquinonyle, un hydroxy-anthraquinonyle, un hétéro-aryle en $C_1$-$C_{10}$ non substitué ou porteur d'un -OH, d'un phényle ou d'un alkyle en $C_1$-$C_4$, ou un aralkyle en $C_7$-$C_{14}$ non substitué ou porteur d'un -OH, d'un ou plusieurs alcoxy en $C_1$-$C_4$ ou d'un radical -N($R^6$)$_2$ dont le symbole $R^6$ a la signification indiquée plus haut, ou

$R^5$ représente un radical -$CH_2$-CH(OH)$CH_2$-S-R, ou

$R^5$ représente un radical aliphatique bivalent en $C_2$-$C_{12}$ qui dérive d'un alcane en $C_2$-$C_{12}$ porteur de deux radicaux -$NH_2$, ce radical aliphatique ne portant pas de substituant ou pouvant porteur un -OH, un -$OCH_3$ ou un radical -N($R^6$)$_2$ dont le symbole $R^6$ a la signification précédemment donnée, et étant éventuellement interrompu par -O-, par -S- ou par un radical -N($R^9$) dont le symbole $R^9$ représente un alkyle en $C_1$-$C_4$ non substitué ou porteur d'un -OH ou un radical -$CH_2$-CH(OH)-$CH_2$-S-R, ou

$R^5$ représente un radical cycloaliphatique en $C_6$-$C_{12}$ bivalent, trivalent ou quadrivalent qui dérive d'un cycloalcane en $C_6$-$C_{12}$ porteur de deux à quatre radicaux -$NH_2$ et qui peut être dépourvu de substituant ou porter un alkyle en $C_1$-$C_4$, ou

$R^5$ représente un radical de formule:

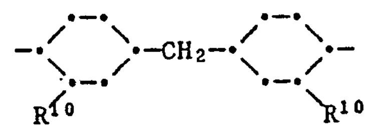

dans lequel $R^{10}$ représente l'hydrogène ou un alkyle en $C_1$-$C_4$, ou

$R^5$ représente un radical de formule:

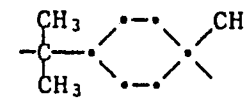

ou un radical aromatique bivalent ou trivalent en $C_6$, en $C_{10}$ ou en $C_{14}$ qui dérivé d'un hydrocarbure aromatique en $C_6$, $C_{10}$ ou en $C_{14}$ porteur de 2 ou de 3 radicaux -$NH_2$ et qui peut être non substitué ou porter un -OH, un -$NO_2$ ou un alkyle en $C_1$-$C_4$, ou

$R^5$ représente un anthraquinonylène, un dihydro-2,3 anthraquinonylène ou un radical de formule:

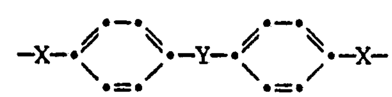

dans lequel X représente -$CH_2$- ou une liaison directe et Y représente -$CH_2$-, -C($C_6H_5$)H-, -S-S-, -NH- ou une liaison directe, ou

$R^5$ représente un radical hétéro-aromatique bivalent ou trivalent en $C_2$-$C_{10}$ qui dérive d'un composé hétéro-aromatique en $C_2$-$C_{10}$ porteur de 2 ou de 3 radicaux -$NH_2$ et qui est dépourvu de substituant ou porte un -OH ou un aryle en $C_6$ ou $C_{10}$, ou

$R^5$ représente un radical araliphatique bivalent en $C_7$-$C_{14}$ qui dérive d'un aralcane en $C_7$-$C_{14}$ porteur de 2 radicaux -$NH_2$, ou

$R^4$ et $R^5$ forment ensemble, et avec l'atome d'azote auquel ils sont liés, un radical azacyclique en $C_1$-$C_7$ qui peut être aromatique ou non-aromatique et qui peut éventuellement contenir un ou plusieurs atomes N, O ou S, l'atome N pouvant porter un alkyle en $C_1$-$C_4$, lui-même éventuellement porteur d'un -OH, et le radical azacyclique en $C_1$-$C_7$ portant éventuellement, sur un atome de carbone, un alkyle en $C_1$-$C_4$, =O ou =S, ou

$R^4$ et $R^5$ représentent ensemble, et avec l'atome d'azote auquel ils sont liés, un radical triméthyl-2,2,4 dihydro-1,2 quinolyle, une partie d'un radical azacyclique bi- à hexavalent en $C_1$-$C_7$ ou un radical répondant à l'une des formules:

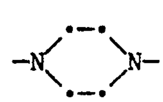,

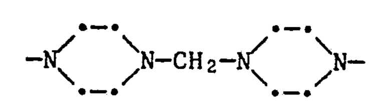

et

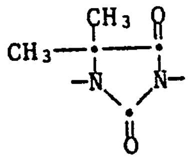

11. Composés de formule Ia selon la revendication 10 dans lesquels n est égal à 1.

12. Composés de formule Ia selon la revendication 10 dans lesquels n désigne un nombre de 2 à 6.

13. Composés de formule Ia selon la revendication 12 dans lesquels n est égal à 2.

14. Composés selon la revendication 10 caractérisés en ce que, dans la formule Ia, $R^4$ représente l'hydrogène ou un radical aryle en $C_6$, en $C_{10}$ ou en $C_{14}$ non substitué ou porteur d'un ou de deux radicaux alkyles en $C_1$-$C_4$ ou $-CF_3$, d'un ou de deux radicaux -OH ou d'un ou de deux radicaux $-R(R^7)(R^8)$, le symbole $R^7$ représentant l'hydrogène ou un alkyle en $C_1$-$C_4$ et le symbole $R^8$ un alkyle en $C_1$-$C_4$ ou un aryle en $C_6$ ou $C_{10}$, ou $R^4$ représente un anthraquinonyle ou un radical hétéro-aryle en $C_1$-$C_6$ non substitué ou porteur d'un -OH ou d'un alkyle en $C_1$-$C_4$, et $R^5$ représente un aryle en $C_6$, en $C_{10}$ ou en $C_{14}$ non substitué ou porteur d'un ou plusieurs alkyles en $C_1$-$C_4$, éventuellement interrompus par -NH- ou par $-N(R^6)-$, d'un ou plusieurs radicaux alcoxy en $C_1$-$C_4$, alkylthio en $C_1$-$C_4$ et/ou -OH, d'un ou plusieurs radicaux $-NO_2$, $-CF_3$ ou -CN, d'un radical hydroxyéthoxy, phénoxy, uréido, carbamoyle, sulfamoyle, benzène-azo, toluène-azo, anilinocarbonyle, anilinosulfonyle ou $-S-CH_2-CH(OH)-CH_2-S-R$ et/ou d'un ou deux radicaux $-N(R^7)(R^8)$, les symboles $R^7$ et $R^8$ ayant les significations qui leur ont été données ci-dessus et $R^8$ pouvant en outre représenter un radical acétyle ou méthoxyphényle, ou $R^5$ représente un anthraquinonyle, un hydroxy-anthraquinonyle ou un radical hétéro-aryle en $C_1$-$C_{10}$ non substitué ou porteur d'un -OH, d'un phényle et/ou d'un alkyle en $C_1$-$C_4$, ou $R^5$ représente un radical aromatique en $C_6$, en $C_{10}$ ou en $C_{14}$ bivalent ou trivalent qui provient d'un hydrocarbure aromatique en $C_6$, en $C_{10}$ ou en $C_{14}$ porteur de deux ou de trois radicaux $-NH_2$ et qui peut être dépourvu de substituant ou porter un -OH, un $-NO_2$ ou un alkyle en $C_1$-$C_4$, ou $R^5$ représente un anthraquinonylène, un dihydro-2,3 anthraquinonylène ou un radical de formule:

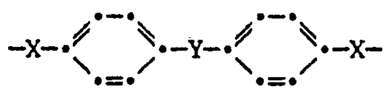

dans lequel X représente une liaison directe et Y représente $-CH_2-$, $-C(C_6H_5)H-$, -S-S-, -NH- ou une liaison directe, ou $R^5$ représente un radical hétéro-aromatique en $C_2$-$C_{10}$ bivalent ou trivalent qui provient d'un hydrocarbure hétéro-aromatique en $C_2$-$C_{10}$ porteur de deux ou de trois radicaux $-NH_2$ et qui peut être dépourvu de substituant ou être porteur d'un -OH ou d'un aryle en $C_6$ ou $C_{10}$, ou $R^4$ et $R^5$ forment ensemble et avec l'atome d'azote auquel ils sont liés un radical azacyclique en $C_1$-$C_7$ qui est aromatique et qui peut éventuellement contenir un ou plusieurs atomes N, O ou S, ou $R^4$ et $R^5$ forment ensemble et avec l'atome d'azote auquel ils sont liés un radical triméthyl-2,2,4 dihydro-1,2 quinolyle.

15. Composés de formula Ia selon la revendication 10 dans lesquels R représente un radical de formule:

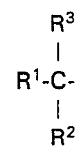

dans lequel $R^1$, $R^2$ et $R^3$ forment ensemble et avec l'atome de carbone auquel ils sont liés un radical alkyle en $C_8$-$C_{12}$ et aucun de ces symboles $R^1$, $R^2$ et $R^3$ ne représente l'hydrogène.

16. Ethylthio-1 (triazolyl-3 amino)-3 propanol-2.

17. Application de composés de formule I selon la revendication 1 et de formule Ia selon la revendication 10 comme additifs hautes pressions et anti-usures dans des lubrifiants, des liquides hydrauliques ou des liquides pour l'usinage de métaux.